# EUROPEAN PATENT APPLICATION

(11) **EP 1 832 653 A1**
(43) Date of publication of application: **12.09.2007**
(21) Application number: 06076937.9
(22) Date of filing: 22.10.1999
(51) Int. Cl.: C12N 9/88, C07K 16/40

(54) **MN gene and protein**

(30) Priority: 23.10.1998 US 177776; 23.10.1998 US 178115
(62) Divisional of application: 99955151.8
(71) Applicant: INSTITUTE OF VIROLOGY, 842 46 Bratislava (SK)
(72) Inventor: Zavada, Jan, 160 00 Prague 6 (CZ); Pastorek, Jaromir, 900 31 Stupava (SK); Pastorekova, SIlvia, 900 31 Stupava (SK)
(74) Representative: Froud, Clive

(57) **Abstract**

Identified herein is the location of the MN protein binding site, and MN proteins/polypeptides that compete for attachment to vertebrate cells with immobilized MN protein. Such MN proteins/polypeptides prevent cell-cell adhesion and the formation of intercellular contacts. The MN protein binding site is a therapeutic target that can be blocked by organic or inorganic molecules, preferably organic molecules, more preferably proteins/polypeptides that specifically bind to that site. Therapeutic methods for inhibiting the growth of preneoplastic/neoplastic vertebrate cells that abnormally express MN protein are disclosed. Vectors are provided that encode the variable domains of MN-specific antibodies and a flexible linker polypeptide separating those domains. Further vectors are disclosed that encode a cytotoxic protein/polypeptide operatively linked to the MN gene promoter, and which vectors preferably further encode a cytokine. The MN gene promoter is characterized, and the binding site for a repressor of MN transcription is disclosed.

## Description

### FIELD OF THE INVENTION

The present invention is in the general area of medical genetics and in the fields of biochemical engineering, immunochemistry and oncology. More specifically, it relates to the MN gene - a cellular gene considered to be an oncogene, which encodes the oncoprotein now known alternatively as the MN protein, the MN/CA IX isoenzyme or the MN/G250 protein.

### BACKGROUND OF THE INVENTION

Zavada et al., International Publication Number WO 93/18152 (published 16 September 1993) and U.S. Patent No. 5,387,676 (issued February 7, 1996), describe the elucidation of the biological and molecular nature of MaTu which resulted in the discovery of the MN gene and protein. The MN gene was found to be present in the chromosomal DNA of all vertebrates tested, and its expression to be strongly correlated with tumorigenicity.

The MN protein was first identified in HeLa cells, derived from a human carcinoma of cervix uteri. It is found in many types of human carcinomas (notably uterine cervical, ovarian, endometrial, renal, bladder, breast, colorectal, lung, esophageal, and prostate, among others). Very few normal tissues have been found to express MN protein to any significant degree. Those MN-expressing normal tissues include the human gastric mucosa and gallbladder epithelium, and some other normal tissues of the alimentary tract. Paradoxically, MN gene expression has been found to be lost or reduced in carcinomas and other preneoplastic/neoplastic diseases in some tissues that normally express MN, e.g., gastric mucosa.

in general, oncogenesis may be signified by the abnormal expression of MN protein. For example, oncogenesis may be signified: (1) when MN protein is present in a tissue which normally does not express MN protein to any significant degree; (2) when MN protein is absent from a tissue that normally expresses it; (3) when MN gene expression is at a significantly increased level, or at a significantly reduced level from that normally expressed in a tissue; or (4) when MN protein is expressed in an abnormal location within a cell.

Zavada et al., WO 93/18152 and Zavada et al., WO 95/34650 (published 21 December 1995) disclose how the discovery of the MN gene and protein and the strong association of MN gene expression and tumorigenicity led to the creation of methods that are both diagnostic/prognostic and therapeutic for cancer and precancerous conditions. Methods and compositions were provided therein for identifying the onset and presence of neoplastic disease by detecting or detecting and quantitating abnormal MN gene expression in vertebrates. Abnormal MN gene expression can be detected or detected and quantitated by a variety of conventional assays in vertebrate samples, for example, by immunoassays using MN-specific antibodies to detect or detect and quantitate MN antigen, by hybridization assays or by PCR assays, such as RT-PCR, using MN nucleic acids, such as, MN cDNA, to detect or detect and quantitate MN nucleic acids, such as, MN mRNA.

Zavada et al, WO 93/18152 and WO 95/34650 describe the production of MN-specific antibodies. A representative and preferred MN-specific antibody, the monoclonal antibody M75 (Mab M75), was deposited at the American Type Culture Collection (ATCC) in Manassus, VA (USA) under ATCC Number HB 11128. The M75 antibody was used to discover and identify the MN protein and can be used to identify readily MN antigen in Western blots, in radicimmunoassays and immunohistochemically, for example, in tissue samples that are fresh, frozen, or formalin-, alcohol-, acetone- or otherwise fixed and/or paraffin-embedded and deparaffinized. Another representative and preferred MN-specific antibody, Mab MN12, is secreted by the hybridoma MN 12.2.2, which was deposited at the ATCC under the designation HB 11647. Example 1 of Zavada et al., WO 95/34650 provides representative results from immunohistochemical staining of tissues using MAb M75, which results support the designation of the MN gene as an oncogene.

Many studies have confirmed the diagnostic/prognostic utility of MN. The following articles discuss the use of the MN-specific MAb M75 in diagnosing/prognosing precancerous and cancerous cervical lesions: Leff, D. N., "Half a Century of HeLa Cells: Transatlantic Antigen Enhances Reliability of Cervical Cancer Pap Test, Clinical Trials Pending," BioWorld® Today: The Daily Biotechnology Newspaper, 9(55) (March 24, 1998); Stanbridge, E. J., "Cervical marker can help resolve ambigous Pap smears," Diagnostics Intelligence. 10(5): 11 (1998); Liao and Stanbridge, "Expression of the MN Antigen in Cervical Papanicolaou Smears Is an Early Diagnostic Biomarker of Cervical Dysplasia," Cancer Epidemiology, Biomarkers & Prevention. 5: 549-557 (1996); Brewer et al., "A Study of Biomarkers in Cervical Carcinoma and Clinical Correlation of the Novel Biomarker MN," Gynecologic Oncology, 63: 337-344 (1996); and Liao et al., "Identification of the MN Antigen as a Diagnostic Biomarker of Cervical Intraepithelial Squamous and Glandular Neoplasia and Cervical Carcinomas," American Journal of Pathology, 145(3): 598-609 (1994).

Premalignant and Malignant Colorectal Lesions. MN has been detected in normal gastric, intestinal, and biliary mucosa. [Pastorekova et al., Gastroenterology, 112: 398-408 (1997).] Immunohistochemical analysis of the normal large intestine revealed moderate staining in the proximal colon, with the reaction becoming weaker distally. The staining was confined to the basolateral surfaces of the cryptal epithelial cells, the area of greatest proliferative capacity. As MN is much more abundant in the proliferating cryptal epithelium than in the upper part of the mucosa, it may play a role in control of the proliferation and differentiation of intestinal epithelial cells. Cell proliferation increases abnormally in premalignant and malignant lesions of the colorectal epithelium, and therefore, is considered an indicator of colorectal tumor progression. [Risio, M., J. Cell Biochem. 16G: 79-87 (1992); and Moss et al., Gastroenterology, 111: 1425-1432 (1996).]

The MN protein is now considered to be the first tumor-associated carbonic anhydrase (CA) isoenzyme that has been described. Carbonic anhydrases (CAs) form a large family of genes encoding zinc metalloenzymes of great physiological importance. As catalysts of reversible hydration of carbon dioxide, these enzymes participate in a variety of biological processes, including respiration, calcification, acid-base balance, bone resorption, formation of aqueous humor, cerebrospinal fluid, saliva and gastric acid [reviewed in Dodgson et al., The Carbonic Anhydrases. Plenum Press, New York-London, pp. 398 (1991)]. CAs are widely distributed in different living organisms.

In mammals, at least seven isoenzymes (CA I-Vil) and a few CA-related proteins (CARP/CA VIII, RPTP-β, RPTP-T) had been identified [ Hewett-Emmett and Tashian, Mol. Phyl. Evol., 5: 50-77 (1996)], when analysis of the MN deduced amino acid sequence revealed a striking homology between the central part of the MN protein and carbonic anhydrases, with the conserved zinc-binding site as well as the enzyme's active center. Then MN protein was found to bind zinc and to have CA activity. Based on that data, the MN protein is now considered to be the ninth carbonic anhydrase isoenzyme - MN/CA IX. [Opavsky et al., Genomics. 33: 480-487 (May 1996)]. [See also. Hewett-Emmett, supra, wherein CA IX is suggested as a nomenclatural designation.]

CAs and CA-related proteins show extensive diversity in both their tissue distribution and in their putative or established biological functions [Tashian, R. E., Adv. in Genetics. 30: 321-356 (1992)]. Some of the CAs are expressed in almost all tissues (CA II), while the expression of others appears to be more restricted (CA VI and CA VII in salivary glands). In cells, they may reside in the cytoplasm (CA I, CA II, CA III, and CA VII), in mitochondria (CA V), in secretory granules (CA VI), or they may associate with membrane (CA IV). Occasionally, nuclear localization of some isoenzymes has been noted [Parkkila et al., Gut. 35: 646-650 (1994); Parkkilla et al., Histochem. J., 27: 133-138 (1995); Mori et al., Gastroenterol., 105: 820-826 (1993)].

The CAs and CA-related proteins also differ in kinetic properties and susceptibility to inhibitors [Sly and Hu, Annu. Rev. Biochem., 64: 375-401 (1995)]. In the alimentary tract, carbonic anhydrase activity is involved in many important functions, such as saliva secretion, production of gastric acid, pancreatic juice and bile, intestinal water and ion transport, fatty acid uptake and biogenesis in the liver. At least seven CA isoenzymes have been demonstrated in different regions of the alimentary tract. However, biochemical, histochemical and immunocytochemical studies have revealed a considerable heterogeneity in their levels and distribution [Swensen, E. R., "Distribution and functions of carbonic anhydrase in the gastrointestinal tract," In: The Carbonic Anhydrases. Cellular Physiology and Molecular Genetics, (Dodgson et al. eds.) Plenum Press, New York, pages 265-287 (1991); and Parkkila and Parkkila, Scan J. Gastroenterol., 31: 305-317 (1996)]. While CA II is found along the entire alimentary canal, CA IV is linked to the lower gastrointestinal tract, CA I, III and V are present in only a few tissues, and the expression of CA VI and VII is restricted to salivary glands [Parkkila et al., Gut. 35: 646-650 (1994); Fleming et al., J. Clin. Invest., 96: 2907-2913 (1995); Parkkila et al., Hepatology, 24: 104 (1996)].

MN/CA IX has a number of properties that distinguish it from other known CA isoenzymes and evince its relevance to oncogenesis. Those properties include its density dependent expression in cell culture (e.g., HeLa cells), its correlation with the tumorigenic phenotype of somatic cell hybrids between HeLa and normal human fibroblasts, its close association with several human carcinomas and its absence from corresponding normal tissues [e.g., Zavada et al., Int. J. Cancer, 54: 268-274 (1993); Pastorekova et al., Virology, 187: 620-626 (1992); Liao et al., Am. J. Pathol., 145: 598-609 (1994); Pastorek et al., Oncogene, 9: 2788-2888 (1994); Cote, Women's Health Weekly: News Section, p. 7 (March 30, 1998); Liao et al., Cancer Res., 57: 2827 (1997); Vermylen et al., "Expression of the MN antigen as a biomarker of lung carcinoma and associated precancerous conditions," Proceedings AACR, 39: 334 (1998); McKieman et al., Cancer Res.. 57: 2362 (1997); and Turner et al., Hum. Pathol., 28(6): 740 (1997)]. In addition, the *in vitro* transformation potential of MN/CA IX cDNA has been demonstrated in NIH 3T3 fibroblasts [Pastorek et al., id.].

The MN protein has also been identified with the G250 antigen. Uemura et al., "Expression of Tumor-Associated Antigen MN/G250 in Urologic Carcinoma: Potential Therapeutic Target," J. Urol., 154 (4 Suppl.): 377 (Abstract 1475; 1997) states: "Sequence analysis and database searching revealed that G250 antigen is identical to MN, a human tumor-associated antigen identified in cervical carcinoma (Pastorek et al., 1994)."

### SUMMARY OF THE INVENTION

Identified herein is the location of the MN protein binding site. Of particular importance is the region within the proteoglycan-like domain, aa 61-96 (SEQ ID NO: 97) which contains a 6-fold tandem repeat of 6 amino acids, and within which the epitope for the M75 MAb resides in at least two copies, and within which the MN binding site is considered to be located. An alternative MN binding site may be located in the CA domain.

Also identified are MN proteins and MN polypeptides that compete for attachment to cells with immobilized MN protein. Such MN proteins/polypeptides prevent cell-cell adhesion and the formation of intercellular contacts.

Disclosed herein are cell adhesion assay methods that are used to identify binding site(s) on the MN protein to which vertebrate cells, preferably mammalian cells, more preferably human cells, bind. Such a MN binding site is then identified as a therapeutic target which can be blocked with MN-specific antibodies, or inorganic or organic molecules, preferably organic molecules, more perferably proteins/polypeptides that specifically bind to said site.

Further disclosed are therapeutic methods to treat patients with preneoplastic/neoplastic disease associated with or characterized by abnormal MN expression, which methods are based on blocking said MN binding site with molecules, inorganic or organic, but preferably organic molecules, more preferably proteins/polypeptides, that bind specifically to said binding site. The growth of a vertebrate preneoplastic/neoplastic cell that abnormally expresses MN protein can be inhibited by administering such organic or inorganic molecules, preferably organic molecules, more preferably proteins/polypeptides in a therapeutically effective amount in a physiologically acceptable formulation. Such a preferred therapeutic protein/polypeptide is herein considered to comprise an amino acid sequence selected from the group consisting of SEQ ID NOS: 107-109. Such heptapeptides are considered to be comprised by MN protein partner(s). Blocking the interaction between MN protein and its binding partner(s), is expected to lead to a decrease of tumor growth.

Further provided are other therapeutic methods wherein the growth of a vertebrate, preferably mammalian, more preferably human, preneoplastic or neoplastic cell that abnormally expresses MN protein is inhibited. Said methods comprise transfecting said cell with a vector comprising an expression control sequence operatively linked to a nucleic acid encoding the variable domains of an MN-specific antibody, wherein said domains are separated by a flexible linker peptide, preferably SEQ ID NO: 116. Preferably said expression control sequence comprises the MN gene promoter.

Still further therapeutic methods comprise transfecting said cell with a vector comprising a nucleic acid that encodes a cytotoxic protein/polypeptide, such as HSVtk, operatively linked to the MN gene promoter. Such a therapeutic vector may also comprise a nucleic acid encoding a cytokine, such as, IL-2 or IFN.

Aspects of the instant invention disclosed herein are described in more detail as follows. The therapeutic use of organic or inorganic molecules, preferably organic molecules, is disclosed. Preferred such molecules bind specifically to a site on MN protein to which vertebrate cells adhere in a cell adhesion assay, wherein said molecule when tested in vitro inhibits the adhesion of cells to MN protein. Further preferred are such molecules, which when in contact with a vertebrate preneoplastic or neoplastic cell that abnormally expresses MN protein, inhibit the growth of said cell. Said vertebrate cells are preferably mammalian and more preferably human.

Preferably such a molecule is organic, and more preferably such a organic molecule is a protein or a polypeptide. Still further preferably, said protein or polypeptide comprises an amino acid sequence selected from the group consisting of SEQ ID NOS: 107, 108, 109, 137 and 138. Even more preferably, said polypeptide is selected from the group consisting of SEQ ID NOS: 107, 108, 109, 137 and 138.

The site on MN proteins to which vertebrate cells adhere in said cell adhesion assay is preferably within the proteoglycan-like domain [SEQ ID NO: 50] or within the carbonic anhydrase domain [SEQ ID NO: 51] of the MN protein. Preferably that site comprises an amino acid sequence selected from the group consisting of SEQ ID NOS: 10 and 97-106. Still further preferably, that site has an amino acid sequence selected from the group consisting of SEQ ID NOS: 10 and 97-106.

Another aspect of this invention concerns MN proteins and MN polypeptides which mediate attachment of vertebrate cells in a cell adhesion assay, wherein said MN protein or MN polypeptide when introduced into the extracellular fluid environment of vertebrate cells prevents the formation of intercellular contacts and the adhesion of said vertebrate cells to each other. Such MN proteins and MN polypeptides may be useful to inhibit the growth of vertebrate preneoplastic or neoplastic cells that abnormally express MN protein, when such MN proteins or MN polypeptides are introduced into the extracellular fluid environment of such vertebrate cells. Said vertebrate cells are preferably mammalian, and more preferably human.

Said MN proteins or MN polypeptides which mediate attachment of vertebrate cells in a cell adhesion assay, preferably have amino acid sequences from SEQ ID NO: 97, from SEQ ID NO: 50, or from SEQ ID NO: 51, more preferably from SEQ ID NO: 50. Still more preferably such MN proteins or MN polypeptides comprise amino acid sequences selected from the group consisting of SEQ ID NOS: 10 and 97-106. Alternatively, said MN polypeptides are selected from the group consisting of SEQ ID NOS: 10 and 97-106.

Representative MN proteins and MN polypeptides which mediate attachment of vertebrate cells in a cell adhesion assay, are specifically bound by either the M75 monoclonal antibody that is secreted from the hybridoma VU-M75, which was deposited at the American Type Culture Collection under ATCC No. HB 11128, or by the MN12 monoclonal antibody that is secreted from the hybridoma MN 12.2.2, which was deposited at the American Type Culture Collection under ATCC No. HB 11647, or by both said monoclonal antibodies.

Another aspect of the instant invention is a method of identifying a site on an MN protein to which vertebrate cells adhere by testing a series of overlapping polypeptides from said MN protein in a cell adhesion assay with vertebrate cells, and determining that if cells adhere to a polypeptide from said series, that said polypeptide comprises a site on said MN protein to which vertebrate cells adhere.

Still another aspect of the instant invention is a vector comprising an expression control sequence operatively linked to a nucleic acid encoding the variable domains of a MN-specific antibody, wherein said domains are separated by a flexible linker polypeptide, and wherein said vector, when transfected into a vertebrate preneoplastic or neoplastic cell that abnormally expresses MN protein, inhibits the growth of said cell. Preferably said expression control sequence comprises the MN gene promoter operatively linked to said nucleic acid. Further preferably, said flexible linker polypeptide has the amino acid sequence of SEQ ID NO: 116, and even further preferably, said MN gene promoter has the nucleotide sequence of SEQ ID NO: 27.

Another further aspect of the instant invention concerns a vector comprising a nucleic acid that encodes a cytotoxic protein or cytotoxic polypeptide operatively linked to the MN gene promoter, wherein said vector, when transfected into a vertebrate preneoplastic or neoplastic cell that abnormally expresses MN protein, inhibits the growth of said cell. In one preferred embodiment said cytotoxic protein is HSV thymidine kinase. Preferably, said vector further comprises a nucleic acid encoding a cytokine operatively linked to said MN gene promoter. In alternative and preferred embodiments, said cytokine is interferon or interleukin-2.

The MN gene promoter is characterized herein. The identification of the binding site for a repressor of MN transcription is disclosed. Mutational analysis indicated that the direct repeat AGGGCacAGGGC [SEQ ID NO: 143] is required for efficient repressor binding.

Identification of the protein that binds to the repressor and modification of its binding properties is another route to modulate MN expression leading to cancer therapies. Suppression of MN expression in tumor cells by over expression of a negative regulator is expected to lead to a decrease of tumor growth. A repressor complex comprising at least two subunits was found to bind to SEQ ID NO: 115 of the MN gene promoter. A repressor complex, found to be in direct contact with SEQ ID NO: 115 by UV crosslinking, comprised two proteins having molecular weights of 35 and 42 kilodaltons, respectively.

### Abbreviations

The following abbreviations are used herein:
- aa: - amino acid
- ATCC: - American Type Culture Collection
- bp: - base pairs
- BLV: - bovine leukemia virus
- BSA: - bovine serum albumin
- BRL: - Bethesda Research Laboratories
- CA: - carbonic anhydrase
- CAM: - cell adhesion molecule
- CARP: - carbonic anhydrase related protein
- CAT: - chloramphenicol acetyltransferase
- Ci: - curie
- cm: - centimeter
- CMV: - cytomegalovirus
- cpm: - counts per minute
- C-terminus: - carboxyl-terminus
- CTL: - cytotoxic T lymphocytes
- °C: - degrees centigrade
- DEAE: - diethylaminoethyl
- DMEM: - Dulbecco modified Eagle medium
- ds: - double-stranded
- EDTA: - ethylenediaminetetraacetate
- EGF: - epidermal growth factor
- EIA: - enzyme immunoassay
- ELISA: - enzyme-linked immunosorbent assay
- EMSA: - electrophoretic mobility shift assay
- F: - fibroblasts
- FACS: - cytofluorometric study
- FCS: - fetal calf serum
- FITC: - fluorescein isothiocyanate
- FTP: - DNase 1 footprinting analysis
- GST-MN: - fusion protein MN glutathione S-transferase
- GVC: - ganciclovir
- H: - HeLa cells
- H-E: - haematoxylin-eosin
- HEF: - human embryo fibroblasts
- HeLa K: - standard type of HeLa cells
- HeLa S: - Stanbridge's mutant HeLa D98/AH.2
- H/F-T: - hybrid HeLa fibroblast cells that are tumorigenic; derived from HeLa D98/AH.2
- H/F-N: - hybrid HeLa fibroblast cells that are nontumorigenic; derived from HeLa D98/AH.2
- HPV: - Human papilloma virus
- HRP: - horseradish peroxidase
- HSV: - Herpes simplex virus
- IC: - intracellular
- IFN: - interferon
- IL-2: - interleukin-2
- Inr: - initiator
- IPTG: - isopropyl-Beta-D-thiogalacto-pyranoside
- kb: - kilobase
- kbp: - kilobase pairs
- kd or kDa: - kilodaltons
- KS: - keratan sulphate
- LCMV: - lymphocytic choriomeningitis virus
- LTR: - long terminal repeat
- M: - molar
- mA: - milliampere
- MAb: - monoclonal antibody
- MCSF: - macrophage colony stimulating factor
- ME: - mercaptoethanol
- MEM: - minimal essential medium
- min.: - minute(s)
- mg: - milligram
- ml: - milliliter
- mM: - millimolar
- MMC: - mitomycin C
- mmol: - millimole
- MLV: - murine leukemia virus
- N: - normal concentration
- NEG: - negative
- ng: - nanogram
- nm: - nanometer
- nt: - nucleotide
- N-terminus: - amino-terminus
- ODN: - oligodeoxynucleotide
- ORF: - open reading frame
- PA: - Protein A
- PBS: - phosphate buffered saline
- PCR: - polymerase chain reaction
- PEST: - combination of one-letter abbreviations for proline, glutamic acid, serine, threonine
- PG: - proteoglycan
- pl: - isoelectric point
- PMA: - phorbol 12-myristate 13-acetate
- POS: - positive
- Py: - pyrimidine
- RACE: - rapid amplification of cDNA ends
- RCC: - renal cell carcinoma
- RIA: - radioimmunoassay
- RIP: - radioimmunoprecipitation
- RIPA: - radioimmunoprecipitation assay
- RNP: - RNase protection assay
- RT-PCT: - reverse transcription polymerase chain reaction
- SAC: - Staphylococcus aureus cells
- S. aureus: - Staphylococcus aureus
- sc: - subcutaneous
- SDRE: - serum dose response element
- SDS: - sodium dodecyl sulfate
- SDS-PAGE: - sodium dodecyl sulfate-polyacrylamide gel electrophoresis
- SINE: - short interspersed repeated sequence
- SP: - signal peptide
- SP-RIA: - solid-phase radioimmunoassay
- SSDS: - synthetic splice donor site
- SSH: - subtractive suppressive PCR
- SSPE: - NaCl (0.18 M), sodium phosphate (0.01 M), EDTA (0.001 M)
- TBE: - Tris-borate/EDTA electrophoresis buffer
- TC: - tissue culture
- TCA: - trichloroacetic acid
- TC media: - tissue culture media
- TC: - tissue culture
- tk: - thymidine kinase
- TM: - transmembrane
- TMB: - tetramethylbenzidine
- Tris: - tris (hydroxymethyl) aminomethane
- µCi: - microcurie
- µg: - microgram
- µl: - microliter
- µM: - micromolar
- VSV: - vesicular stomatitis virus
- VV: - vaccinia virus
- X-MLV: - xenotropic murine leukemia virus

### Cell Lines

- AGS: - cell line derived from a primary adenogastric carcinoma [Barranco and Townsend, Cancer Res.. 43: 1703 (1983) and Invest. New Drugs, 1: 117 (1983)]; available from the ATCC under CRL-1739;
- BL-3: - bovine B lymphocytes [ATCC CRL-8037; leukemia cell suspension; J. Natl. Cancer Inst. (Bethesda) 40: 737 (1968)];
- C33: - a cell line derived from a human cervical carcinoma biopsy [Auersperg, N., J. Nat'l. Cancer Inst. (Bethesda), 32: 135-148 (1964)]; available from the ATCC under HTB-31;
- C33A: - human cervical carcinoma cells [ATCC HTB-31; J. Natl. Cancer Inst. (Bethesda) 32: 135 (1964)];
- COS: - simian cell line [Gluzman, Y., Cell, 23: 175 (1981)];
- HeLa K: - standard type of HeLa cells; aneuploid, epithelial-like cell line isolated from a human cervical adenocarcinoma [Gey et al., Cancer Res.. 12: 264 (1952); Jones et al., Obstet. Gynecol.. 38: 945-949 (1971)] obtained from Professor B. Korych, [Institute of Medical Microbiology and Immunology, Charles University; Prague, Czech Republic];
- HeLa D98/AH.2 (also HeLa s): - Mutant HeLa clone that is hypoxanthine guanine phosphoribosyl transferase-deficient (HGPRT) kindly provided by Eric J. Stanbridge [Department of Microbiology, College of Medicine, University of California, Irvine, CA (USA)] and reported in Stanbridge et al., Science, 215: 252-259 (15 Jan. 1982); parent of hybrid cells H/F-N and H/F-T, also obtained from E.J. Stanbridge;
- KATO III: - cell line prepared from a metastatic form of a gastric carcinoma [Sekiguichi et al., Japan J. Exp. Med., 48: 61 (1978)]; available from the ATCC under HTB-103;
- NIH-3T3: - murine fibroblast cell line reported in Aaronson, Science, 237: 178 (1987);
- QT35: - quail fibrosarcoma cells [ECACC: 93120832; Cell, 11: 95 (1977)];
- Raj: - human Burkitt's lymphoma cell line [ATCC CCL-86; Lancet, 1: 238 (1964)];
- Rat2TK: - cell line (rat embryo, thymidine kinase mutant) was derived from a subclone of a 5'-bromo-deoxyuridine resistant strain of the Fischer rat fibroblast 3T3-like cell line Rat1; the cells lack appreciable levels of nuclear thymidine kinase [Ahrens, B., Virology, 113: 408 (1981)];
- SiHa: - human cervical squamous carcinoma cell line [ATCC HTB-35; Friedl et al., Proc. Soc. Exp. Biol. Med., 135: 543 (1990)];
- XC: - cells derived from a rat rhabdomyosarcoma induced with Rous sarcoma virus-induced rat sarcoma [Svoboda, J., Natl. Cancer Center Institute Monograph No. 17, IN: "International Conference on Avian Tumor Viruses" (J.W. Beard ed.), pp. 277-298 (1964)], kindly provided by Jan Svoboda [Institute of Molecular Genetics, Czechoslovak Academy of Sciences; Prague, Czech Republic]; and
- CGL1: - H/F-N hybrid cells (HeLa D98/AH.2 derivative);
- CGL2: - H/F-N hybrid cells (HeLa D98/AH.2 derivative);
- CGL3: - H/F-T hybrid cells (HeLa D98/AH.2 derivative);
- CGL4: - H/F-T hybrid cells (HeLa D98/Ah.2 derivative).

### Nucleotide and Amino Acid Sequence Symbols

The following symbols are used to represent nucleotides herein:
Base

| Symbol | Meaning |
|---|---|
| A | adenine |
| C | cytosine |
| G | guanine |
| T | thymine |
| U | uracil |
| I | inosine |
| M | A or C |
| R | AorG |
| W | A or T/U |
| S | CorG |
| Y | C or T/U |
| K | G or T/U |
| V | AorCorG |
| H | AorCorT/U |
| D | A or G or T/U |
| B | C or G or T/U |
| N/X | A or C or G or T/U |

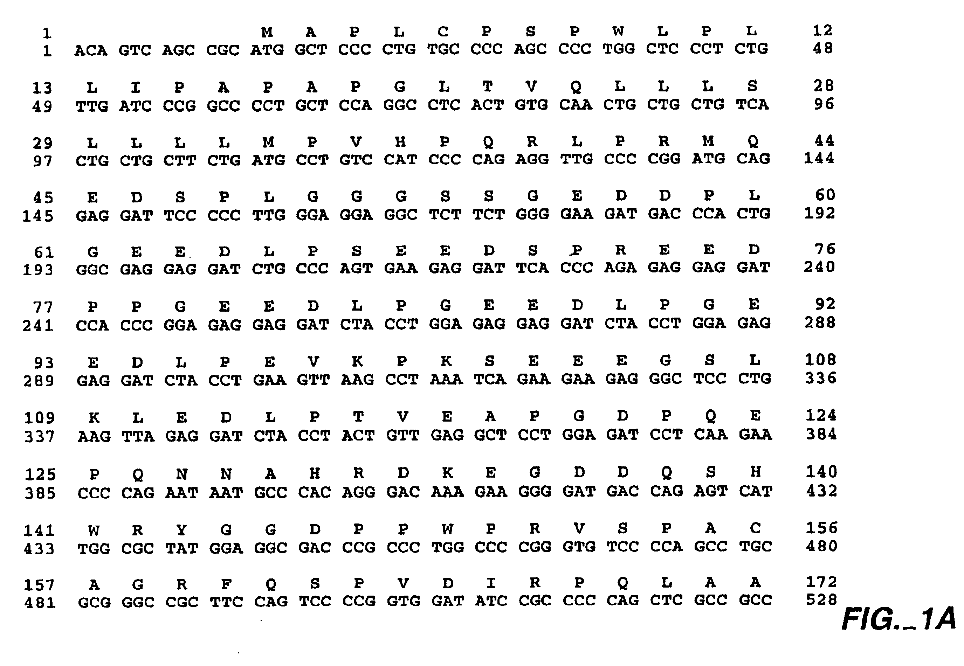

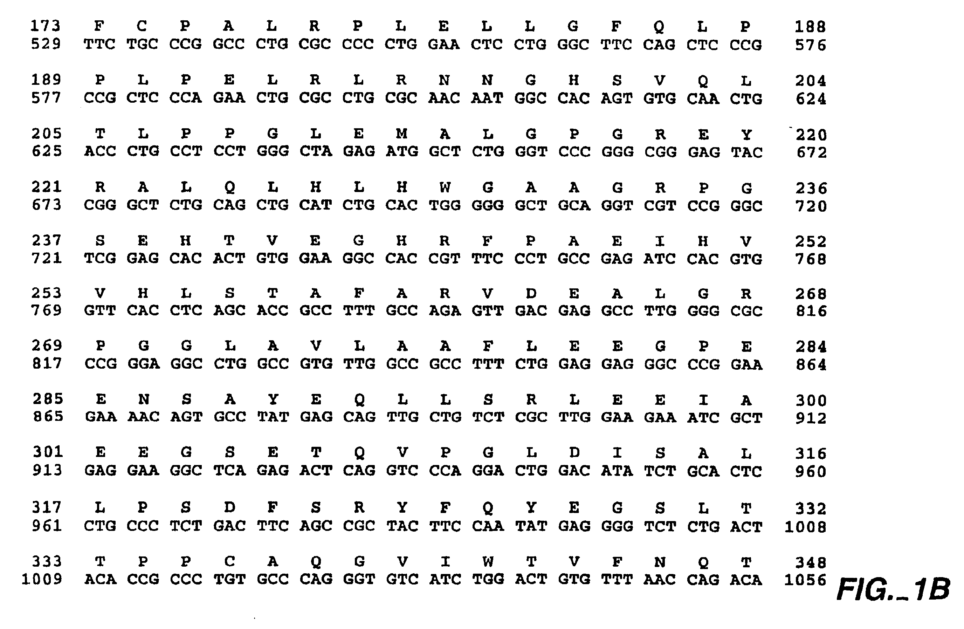

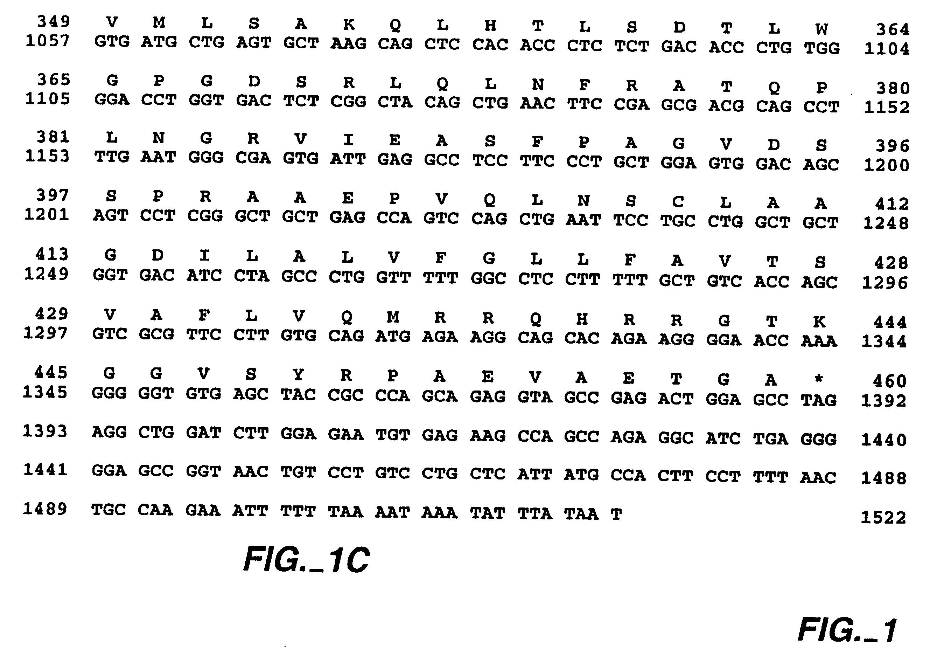

There are twenty main amino acids, each of which is specified by a different arrangement of three adjacent nucleotides (triplet code or codon), and which are linked together in a specific order to form a characteristic protein. A three-letter or one-letter convention is used herein to identify said amino acids, as, for example, in Figure 1 as follows:

| Amino acid name | 3 Ltr. Abbrev. | 1 Ltr. Abbrev. |
|---|---|---|
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Unknown or other | | X |

### BRIEF DESCRIPTION OF THE FIGURES

Figure 1A-C provides the nucleotide sequence for a MN cDNA [SEQ ID NO: 1] clone isolated as described herein. Figure 1 A-C also sets forth the predicted amino acid sequence [SEQ ID NO: 2] encoded by the cDNA.

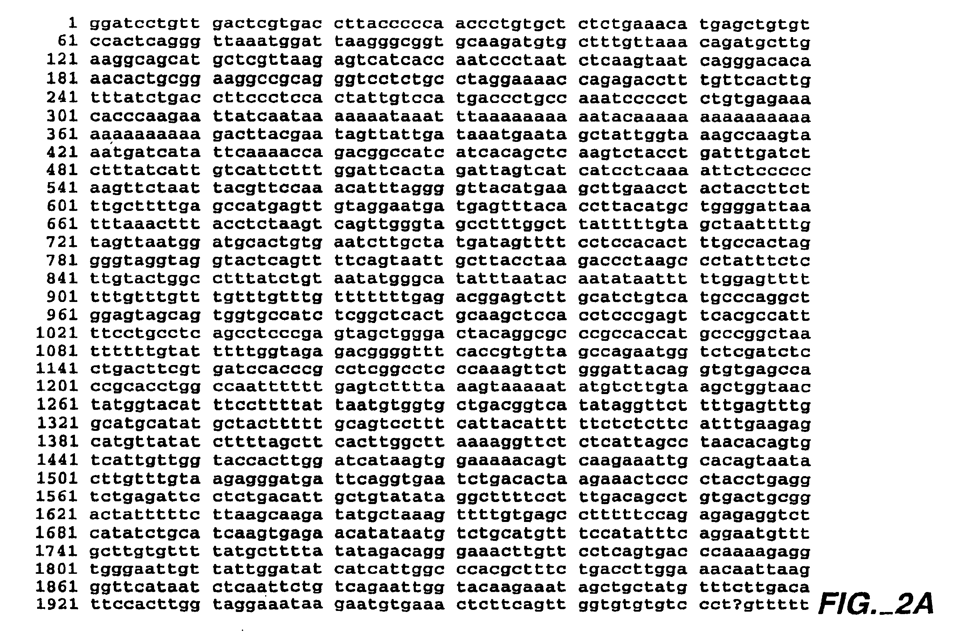

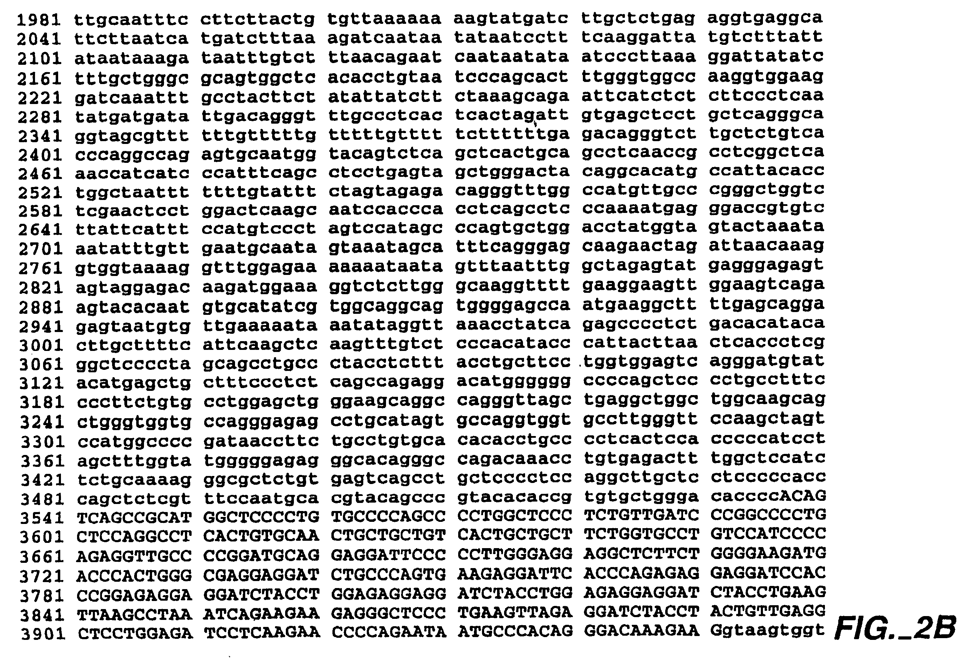

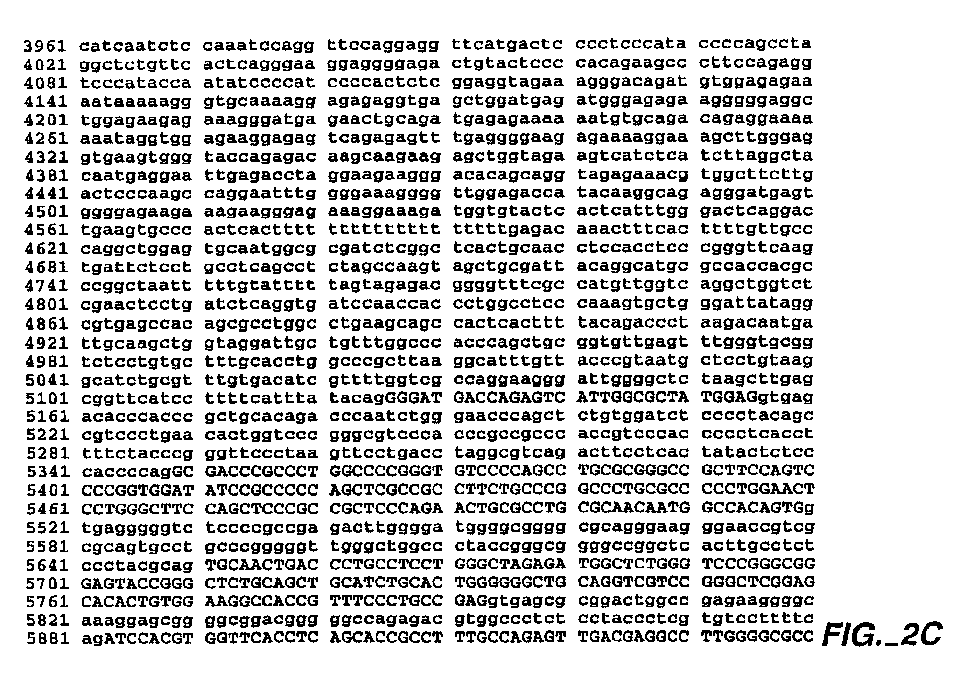

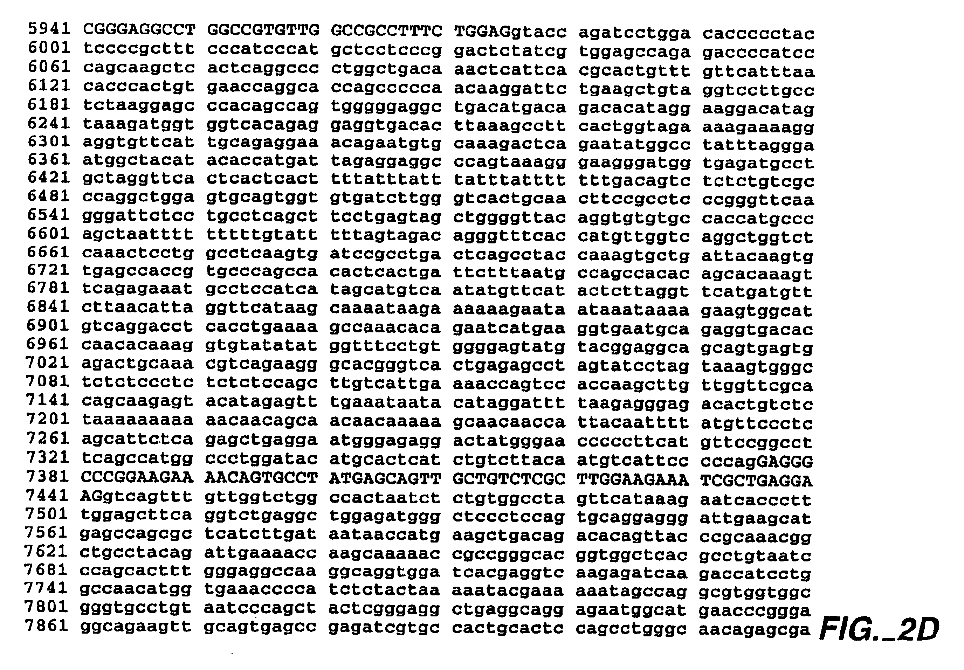

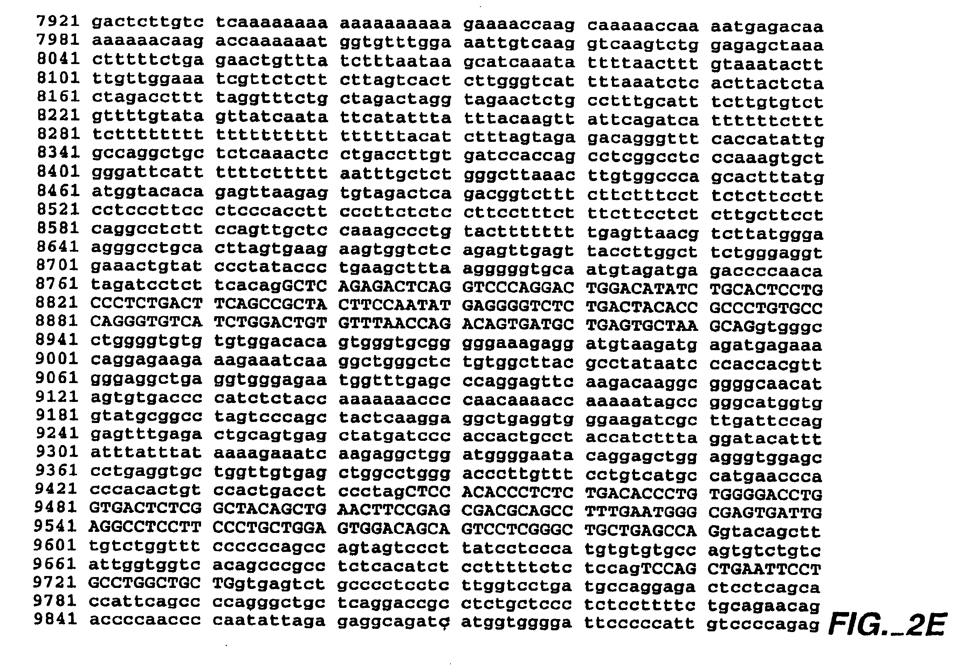

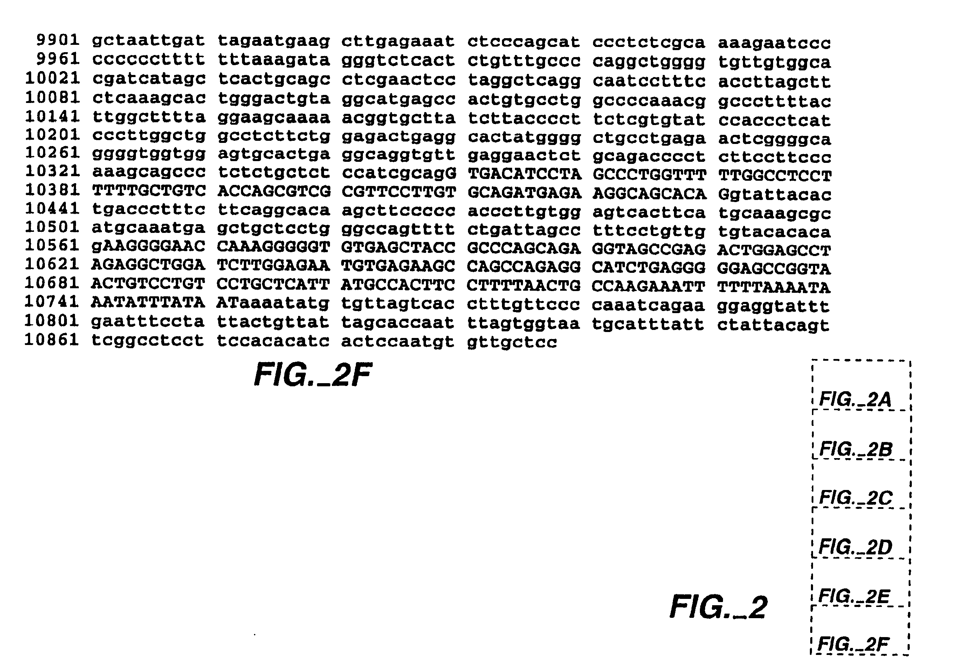

Figure 2A-F provides a 10,898 bp complete genomic sequence of MN [SEQ ID NO: 5]. The base count is as follows: 2654 A; 2739 C; 2645 G; and 2859 T. The 11 exons are in general shown in capital letters, but exon 1 is considered to begin at position 3507 as determined by RNase protection assay.

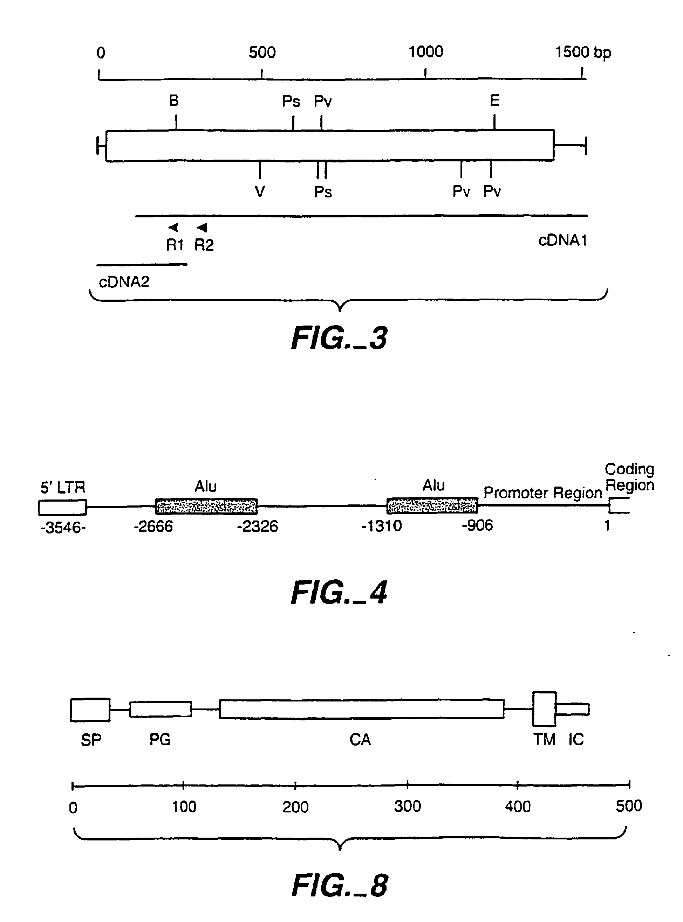

Figure 3 is a restriction map of the full-length MN cDNA. The open reading frame is shown as an open box. The thick lines below the restriction map illustrate the sizes and positions of two overlapping cDNA clones. The horizontal arrows indicate the positions of primers R1 [SEQ ID NO: 7] and R2 [SEQ ID NO: 8] used for the 5' end RACE. Relevant restriction sites are BamHl (B), EcoRV (V), EcoRI (E), Pstl (Ps), Pvull (Pv).
Figure 4 schematically represents the 5' MN genomic region of a MN genomic clone wherein the numbering corresponds to transcription initiation sites estimated by RACE.

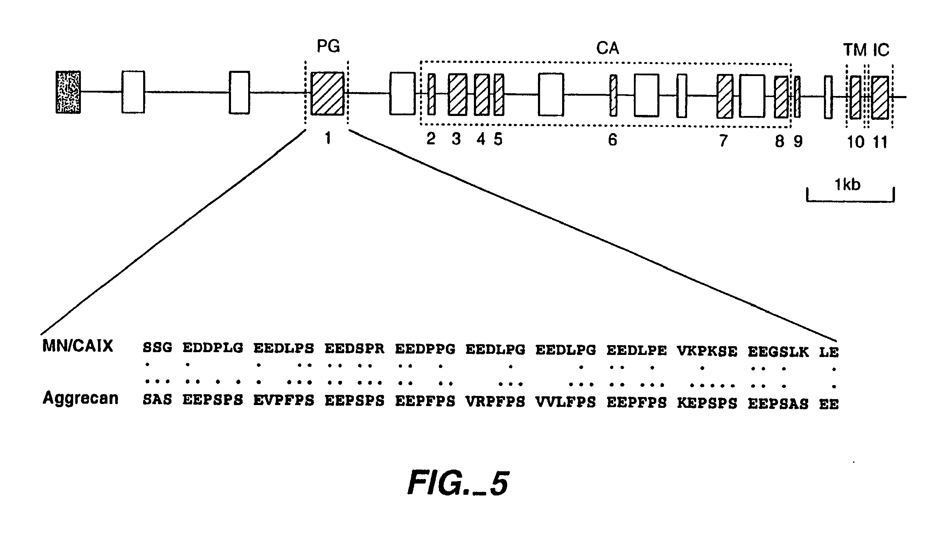

Figure 5 provides an exon-intron map of the human MN/CA IX gene. The positions and sizes of the exons (numbered, cross-hatched boxes), Alu repeat elements (open boxes) and an LTR-related sequence (first unnumbered stippled box) are adjusted to the indicated scale. The exons corresponding to individual MN/CA IX protein domains are enclosed in dashed frames designated PG (proteoglycan-like domain), CA (carbonic anhydrase domain), TM (transmembrane anchor) and IC (intracytoplasmic tail). Below the map, the alignment of amino acid sequences illustrates the extent of homology between the MN/CA IX protein PG region (aa 53-111) [SEQ ID NO: 50] and the human aggrecan (aa 781-839) [SEQ ID NO: 54].

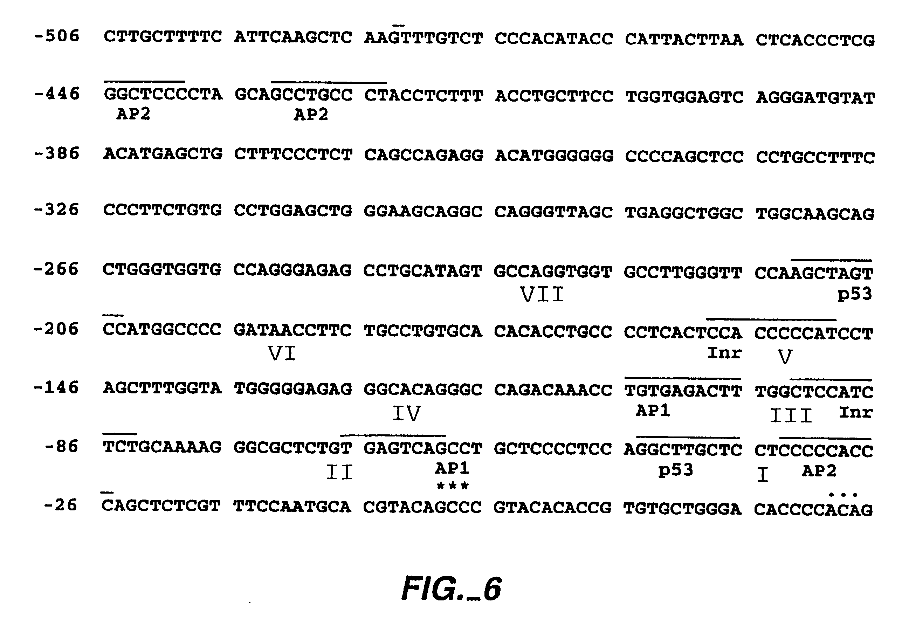

Figure 6 is a nucleotide sequence for the proposed promoter of the human MN gene [SEQ ID NO: 27]. The nucleotides are numbered from the transcription initiation site according to RNase protection assay. Potential regulatory elements are overlined. Transcription start sites are indicated by asterisks (RNase protection) and dots (RACE) above the corresponding nucleotides. The sequence of the 1st exon begins under the asterisks. FTP analysis of the MN4 promoter fragment revealed 5 regions (I-V) protected at both the coding and noncoding strands, and two regions (VI and VII) protected at the coding strand but not at the noncoding strand.

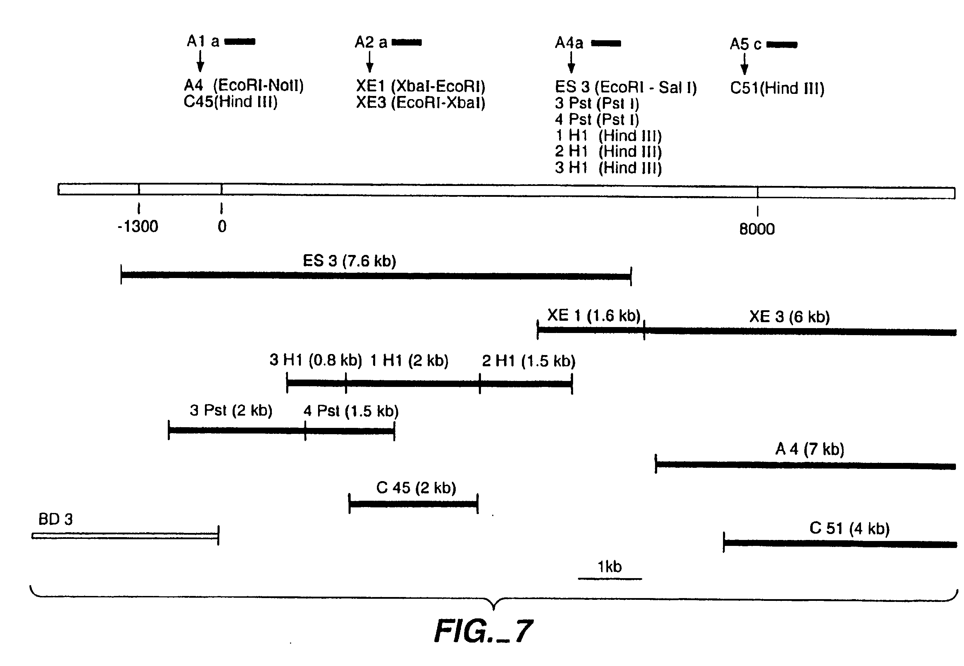

Figure 7 provides a schematic of the alignment of MN genomic clones according to their position related to the transcription initiation site. All the genomic fragments except Bd3 were isolated from a lambda FIX II genomic library derived from HeLa cells. Clone Bd3 was derived from a human fetal brain library.
Figure 8 schematically represents the MN protein structure. The abbreviations are the same as used in Figure 5. The scale indicates the number of amino acids.

### DETAILED DESCRIPTION

The terms "MN/CA IX" and "MN/CA9" are herein considered to be synonyms for MN. Also, the G250 antigen is considered to refer to MN protein/polypeptide. [Uemura et al., J. Urol., 154 (4 Suppl.): 377 (Abstract 1475; 1997).]

MN/CA IX was first identified in HeLa cells, derived from human carcinoma of cervix uteri, as both a plasma membrane and nuclear protein with an apparent molecular weight of 58 and 54 kilodaltons (kDA) as estimated by Western blotting. It is N-glycosylated with a single 3kDa carbohydrate chain and under non-reducing conditions forms S-S-linked oligomers [Pastorekova et al., Virology, 187: 620-626 (1992); Pastorek et al., Oncogene 9: 2788-2888 (1994)]. MN/CA IX is a transmembrane protein located at the cell surface, although in some cases it has been detected in the nucleus [Zavada et al., Int. J. Cancer, 54: 268-274 (1993); Pastorekova et al., supra].

MN is manifested in HeLa cells by a twin protein, p54/58N. Immunoblots using a monoclonal antibody reactive with p54/58N (MAb M75) revealed two bands at 54 kd and 58 kd. Those two bands may correspond to one type of protein that most probably differs by post-translational processing. Herein, the phrase "twin protein" indicates p54/58N.

Zavada et al., WO 93/18152 and/or WO 95/34650 disclose the MN cDNA sequence (SEQ ID NO: 1) shown herein in Figure 1A-1C, the MN amino acid sequence (SEQ ID NO: 2) also shown in Figure 1A-1C, and the MN genomic sequence (SEQ ID NO: 5) shown herein in Figure 2A-2F. The MN gene is organized into 11 exons and 10 introns.

The first thirty seven amino acids of the MN protein shown in Figure 1 A-1C is the putative MN signal peptide [SEQ ID NO: 6]. The MN protein has an extracellular domain [amino acids (aa) 38-414 of Figure 1A-1C (SEQ ID NO: 87)], a transmembrane domain [aa 415-434 (SEQ ID NO: 52)] and an intracellular domain [aa 435-459 (SEQ ID NO: 53)]. The extracellular domain contains the proteoglycan-like domain [aa 53-111 (SEQ ID NO: 50)] and the carbonic anhydrase (CA) domain [aa 135-391 (SEQ ID NO: 51].

### Anticancer Drugs and Antibodies that Block

### Interaction of MN Protein and Receptor Molecules

MN protein is considered to be a uniquely suitable target for cancer therapy for a number of reasons including the following. (1) It is localized on the cell surface, rendering it accessible. (2) It is expressed in a high percentage of human carcinomas (e.g., uterine cervical, renal, colon, breast, esophageal, lung, head and neck carcinomas, among others), but is not normal!y expressed to any significant extent in the normal tissues from which such carcinomas originate.
(3) It is normally expressed only in the stomach mucosa and in some epithelia of the digestive tract (epithelium of gallbladder and small intestine). An anatomic barrier thereby exists between the MN-expressing preneoplastic/neoplastic and MN-expressing normal tissues. Drugs, including antibodies, can thus be administered which can reach tumors without interfering with MN-expressing normal tissues.
(4) MAb M75 has a high affinity and specificity to MN protein. (5) MN cDNA and MN genomic clones which encompass the protein-coding and gene regulatory sequences have been isolated. (6) MN-specific antibodies have been shown to have among the highest tumor uptakes reported in clinical studies with antitumor antibodies in solid tumors, as shown for the MN-specific chimeric antibody G250 in animal studies and in phase I clinical trials with renal carcinoma patients. [Steffens et al., J. Clin. Oncol., 15: 1529 (1997).] Also, MN-specific antibodies have low uptake in normal tissues.

Data, e.g. as presented herein, are consistent with the following theory concerning how MN protein acts in normal tissues and in preneoplastic/neoplastic tissues. In normal tissues (e.g., in stomach mucosa), MN protein is considered to be a differentiation factor. It binds with its normal receptor S (for stomach). Stomach carcinomas have been shown not to contain MN protein.

Ectopic expression of MN protein in other tissues causes malignant conversion of cells. Such ectopic expression is considered to be caused by the binding of MN protein with an alternative receptor H (for HeLa cells), coupled to a signal transduction pathway leading to malignancy. Drugs or antibodies which block the binding site of MN protein for receptor H would be expected to cause reversion of prenoplastic/neoplastic cells to normal or induce their death.

### Design and Development of MN-Blocking Drugs or Antibodies

A process to design and develop MN-blocking drugs, e.g., peptides with high affinity to MN protein, or antibodies, has several steps. First, is to test for the binding of MN protein to receptors based on the cell adhesion assay described infra. That same procedure would also be used to assay for drugs blocking the MN protein binding site. In view of the alternative receptors S and H, stomach epithelial cells or revertants (containing preferentially S receptors), HeLa cells (containing the H receptor and lacking the S receptor) would be used in the cell adhesion assay.

To identify the receptor binding site of MN protein, deletion variants of MN protein lacking different domains can be used to identify region(s) responsible for interaction of MN protein with a receptor. Example 2 identifies and illustrates how to detect other binding sites on MN protein. A preferred MN binding site is considered to be closely related or identical to the epitope for MAb M75, which is located in at least 2 copies within the 6-fold tandem repeat of 6 amino acids [aa 61-96 (SEQ ID NO: 97)] in the proteoglycan-like domain of the MN protein. Smaller deletion variants can be prepared within that relevant domain, e.g., fusion proteins with only small segments of MN protein can be prepared. Also, controlled digestion of MN protein with specific proteases followed by separation of the products can be performed.

Further, peptides comprising the expected binding site can be synthesized. All of those products can be tested in cell adhesion assays, as exemplified below. [See, e.g., Pierschbacher and Ruoslahti, PNAS, 81:5985 (1984); Ruoslahti and Pierschbacher, Science, 238: 491.]

Molecules can be constructed to block the MN receptor binding site. For example, use of a phage display peptide library kit [as Ph.D^{®}-7 Peptide 7-Mer Library Kit from New England Biolabs; Beverly, MA (USA)] as exemplified in Examples 2 and 3, can be used to find peptides with high affinity to the target molecules. Biologic activity of the identified peptides will be tested in vitro by inhibition of cell adhesion to MN protein, by effects on cell morphology and growth characteristics of MN-related tumor cells (HeLa) and of control cells. [Symington, J. Biol. Chem., 267: 25744 (1992).] In vivo screening will be carried out in nude mice that have been injected with HeLa cells.

Peptides containing the binding site of the MN protein will be prepared [e.g. MAPs (multiple antigen peptides); Tam, J.P., PNAS (USA) 85: 5409 (1988); Butz et al., Peptide Res., 7: 20 (1994)]. The MAPs will be used to immunize animals to obtain antibodies (polyclonal and/or monoclonal) that recognize and block the binding site. [See, e.g., Brooks et al., Cell, 79: 1157 (1994).] "Vaccination" would then be used to test for protection in animals. Antibodies to the MN binding site could potentially be used to block MN protein's interaction(s) with other molecules.

Computer modeling can also be used to design molecules with specific affinity to MN protein that would mediate steric inhibition between MN protein and its receptor. A computer model of the MN binding site for the receptor will contain spatial, electrostatic, hydrophobic and other characteristics of this structure. Organic molecules complementary to the structure, that best fit into the binding site, will be designed. Inorganic molecules can also be similarly tested that could block the MN binding site.

The use of oncoproteins as targets for developing new cancer therapeutics is considered conventional by those of skill in the art. [See, e.g., Mendelsohn and Lippman, "Growth Factors," pp. 114-133, IN: DeVita et al. (eds.), Cancer: Principles and Practice of Oncology (4th Ed.; Lippincott; Philadelphia, 1993).] In its broadest sense, the design of blocking drugs can be based in competitive inhibition experiments. Such experiments have been used to invent drugs since the discovery of sulfonamides (competitive inhibitors of para-aminobenzoic acid, a precursor of folic acid). Also, some cytostatics are competitive inhibitors (e.g., halogenated pyrimidines, among others).

However, the application of such approaches to MN is new. In comparison to other tumor-related molecules (e.g. growth factors and their receptors), MN has the unique property of being differentially expressed in preneoplastic/neoplastic and normal tissues, which are separated by an anatomic barrier.

### MN Gene - Cloning and Sequencing

Figure 1A-C provides the nucleotide sequence for a full-length MN cDNA clone isolated as described below [SEQ ID NO: 1]. Figure 2A-F provides a complete MN genomic sequence [SEQ ID NO: 5]. Figure 6 shows the nucleotide sequence for a proposed MN promoter [SEQ ID NO: 27].

It is understood that because of the degeneracy of the genetic code, that is, that more than one codon will code for one amino acid [for example, the codons TTA, TTG, CTT, CTC, CTA and CTG each code for the amino acid leucine (leu)], that variations of the nucleotide sequences in, for example, SEQ ID NOS: 1 and 5 wherein one codon is substituted for another, would produce a substantially equivalent protein or polypeptide according to this invention. All such variations in the nucleotide sequences of the MN cDNA and complementary nucleic acid sequences are included within the scope of this invention.

It is further understood that the nucleotide sequences herein described and shown in Figures 1, 2 and 6, represent only the precise structures of the cDNA, genomic and promoter nucleotide sequences isolated and described herein. It is expected that slightly modified nucleotide sequences will be found or can be modified by techniques known in the art to code for substantially similar or homologous MN proteins and polypeptides, for example, those having similar epitopes, and such nucleotide sequences and proteins/ polypeptides are considered to be equivalents for the purpose of this invention. DNA or RNA having equivalent codons is considered within the scope of the invention, as are synthetic nucleic acid sequences that encode proteins/polypeptides homologous or substantially homologous to MN proteins/polypeptides, as well as those nucleic acid sequences that would hybridize to said exemplary sequences [SEQ. ID. NOS. 1, 5 and 27] under stringent conditions, or that, but for the degeneracy of the genetic code would hybridize to said cDNA nucleotide sequences under stringent hybridization conditions. Modifications and variations of nucleic acid sequences as indicated herein are considered to result in sequences that are substantially the same as the exemplary MN sequences and fragments thereof.

Stringent hybridization conditions are considered herein to conform to standard hybridization conditions understood in the art to be stringent. For example, it is generally understood that stringent conditions encompass relatively low salt and/or high temperature conditions, such as provided by 0.02 M to 0.15 M NaCl at temperatures of 50°C to 70°C. Less stringent conditions, such as, 0.15 M to 0.9 M salt at temperatures ranging from 20°C to 55°C can be made more stringent by adding increasing amounts of formamide, which serves to destabilize hybrid duplexes as does increased temperature.

Exemplary stringent hybridization conditions are described in Sambrook et al., Molecular Cloning: A Laboratory Manual, pages 1.91 and 9.47-9.51 (Second Edition, Cold Spring Harbor Laboratory Press; Cold Spring Harbor, NY; 1989); Maniatis et al., Molecular Cloning: A Laboratory Manual, pages 387-389 (Cold Spring Harbor Laboratory; Cold Spring Harbor, NY; 1982); Tsuchiya et al., Oral Surgery, Oral Medicine. Oral Pathology. 71(6): 721-725 (June 1991).

Zavada et al., WO 95/34650 described how a partial MN cDNA clone, a full-length MN cDNA clone and MN genomic clones were isolated and sequenced. Also, Zavada et al., Int. J. Cancer, 54: 268 (1993) describes the isolation and sequencing of a partial MN cDNA of 1397 bp in length. Briefly attempts to isolate a full-length clone from the original cDNA library failed. Therefore, the inventors performed a rapid amplification of cDNA ends (RACE) using MN-specific primers, R1 and R2 [SEQ ID NOS: 7 and 8], derived from the 5' region of the original cDNA clone. The RACE product was inserted into pBluescript, and the entire population of recombinant plasmids was sequenced with an MN-specific primer ODN1 [SEQ ID NO: 3]. In that way, a reliable sequence at the very 5' end of the MN cDNA as shown in Figure 1 [SEQ ID NO: 1] was obtained.

Specifically, RACE was performed using 5' RACE System [GIBCO BRL; Gaithersburg, MD (USA)] as follows. 1 *µ*g of mRNA (the same as above) was used as a template for the first strand cDNA synthesis which was primed by the MN-specific antisense oligonucleotide, R1 (5'-TGGGGTTCTTGAGGATCTCCAGGAG-3') [SEQ ID NO: 7]. The first strand product was precipitated twice in the presence of ammonium acetate and a homopolymeric C tail was attached to its 3' end by TdT. Tailed cDNA was then amplified by PCR using a nested primer, R2 (5'-CTCTAACTTCAGGGAGCCCTCTTCTT-3') [SEQ ID NO: 8] and an anchor primer that anneals to the homopolymeric tail (5'-CUACUACUACUAGGCCACGCGTCGAC TAGTACGGGI IGGGIIGGGIIG-3') [SEQ ID NO: 9]. The amplified product was digested with BamHI and SalI restriction enzymes and cloned into pBluescript II KS plasmid. After transformation, plasmid DNA was purified from the whole population of transformed cells and used as a template for sequencing with the MN-specific primer ODN1 [SEQ ID NO: 3; a 29-mer 5' CGCCCAGTGGGTCATCTTCCCCAGAAGAG 3'].

To study MN regulation, MN genomic clones were isolated. One MN genomic clone (Bd3) was isolated from a human cosmid library prepared from fetal brain using both MN cDNA as a probe and the MN-specific primers derived from the 5' end of the cDNA ODN1 [SEQ ID NO: 3, supra] and ODN2 [SEQ. ID NO.: 4; 19-mer (5' GGAATCCTCCTGCATCCGG 3')]. Sequence analysis revealed that that genomic clone covered a region upstream from a MN transcription start site and ending with the BamHl restriction site localized inside the MN cDNA. Other MN genomic clones can be similarly isolated.

Figure 7 provides a schematic of the alignment of MN genomic clones according to the transcription initiation site. Plasmids containing the A4a clone and the XE1 and XE3 subclones were deposited at the American Type Culture Collection (ATCC) on June 6, 1995, respectively under ATCC Deposit Nos. 97199, 97200, and 97198.

### Exon-Intron Structure of Complete MN Genomic Region

The complete sequence of the overlapping clones contains 10,898 bp (SEQ ID NO: 5). Figure 5 depicts the organization of the human MN gene, showing the location of all 11 exons as well as the 2 upstream and 6 intronic Alu repeat elements. All the exons are small, ranging from 27 to 191 bp, with the exception of the first exon which is 445 bp. The intron sizes range from 89 to 1400 bp. The CA domain is encoded by exons 2-8, while the exons 1, 10 and 11 correspond respectively to the proteoglycan-like domain, the transmembrane anchor and cytoplasmic tail of the MN/CA IX protein. Table 1 below lists the splice donor and acceptor sequences that conform to consensus splice sequences including the AG-GT motif [Mount, Nucleic Acids Res. 10: 459-472 (1982)].

**TABLE 1**

| Exon-Intron Structure of the Human MN Gene | | | | | |
|---|---|---|---|---|---|
| Exon | Size | Genomic Position** | SEQ ID NO | 5'splice acceptor | SEQ ID NO |
| 1 | 445 | *3507-3951 | 28 | AGAAG gtaagt | 67 |
| 2 | 30 | 5126-5155 | 29 | TGGAG gtgaga | 68 |
| 3 | 171 | 5349-5519 | 30 | CAGTC gtgagg | 69 |
| 4 | 143 | 5651-5793 | 31 | CCGAG gtgagc | 70 |
| 5 | 93 | 5883-5975 | 32 | TGGAG gtacca | 71 |
| 6 | 67 | 7376-7442 | 33 | GGAAG gtcagt | 72 |
| 7 | 158 | 8777-8934 | 34 | AGCAG gtgggc | 73 |
| 8 | 145 | 9447-9591 | 35 | GCCAG gtacag | 74 |
| 9 | 27 | 9706-9732 | 36 | TGCTG gtgagt | 75 |
| 10 | 82 | 10350-70431 | 37 | CACAG gtatta | 76 |
| 11 | 191 | 10562-10752 | 38 | ATAAT end | |

| Intron | Size | Genomic Position** | SEQ ID NO | 3'splice acceptor | SEQ ID NO |
|---|---|---|---|---|---|
| 1 | 1174 | 3952-5125 | 39 | atacag GGGAT | 77 |
| 2 | 193 | 5156-5348 | 40 | ccccag GCGAC | 78 |
| 3 | 131 | 5520-5650 | 41 | acgcag TGCAA | 79 |
| 4 | 89 | 5794-5882 | 42 | tttcag ATCCA | 80 |
| 5 | 1400 | 5976-7375 | 43 | ccccag GAGGG | 81 |
| 6 | 1334 | 7443-8776 | 44 | tcacag GCTCA | 82 |
| 7 | 512 | 8935-9446 | 45 | ccctag CTCCA | 83 |
| 8 | 114 | 9592-9705 | 46 | ctccag TCCAG | 84 |
| 9 | 617 | 9733-10349 | 47 | tcgcag GTGACA | 85 |
| 10 | 130 | 10432-10561 | 48 | acacag AAGGG | 86 |

| | | | | | |
|---|---|---|---|---|---|
| ** positions are related to nt numbering in whole genomic sequence including the 5' flanking region [Figure 2A-F] * number corresponds to transcription initiation site determined below by RNase protection assay | | | | | |

### Mapping of MN Gene Transcription Initiation and Termination Sites

Zavada et al., WO 95/34650 describes the process of mapping the MN gene transcription initiation and termination sites. A RNase protection assay was used for fine mapping of the 5' end of the MN gene. The probe was a uniformly labeled 470 nucleotide copy RNA (nt-205 to + 265) [SEQ ID NO: 55], which was hybridized to total RNA from MN-expressing HeLa and CGl.3 cells and analyzed on a sequencing gel. That analysis has shown that the MN gene transcription initiates at multiple sites, the 5' end of the longest MN transcript being 30 nt longer than that previously characterized by RACE.

### Characterization of the 5' Flanking Region

The Bd3 genomic clone isolated from human fetal brain cosmid library was found to cover a region of 3.5 kb upstream from the transcription start site of the MN gene. It contains no significant coding region. Two Alu repeats are situated at positions -2587 to -2296 [SEQ ID NO: 56] and -1138 to -877 [SEQ ID NO: 57] (with respect to the transcription start determined by RNP).

Nucleotide sequence analysis of the DNA 5' to the transcription start (from nt -507) revealed no recognizable TATA box within the expected distance from the beginning of the first exon. However, the presence of potential binding sites for transcription factors suggests that this region might contain a promoter for the MN gene. There are several consensus sequences for transcription factors AP1 and AP2 as well as for other regulatory elements, including a p53 binding site [Locker and Buzard, J., DNA Sequencing and Mapping, 1: 3-11 (1990); Imagawa et al. Cell, 51: 251-260 (1987); El Deiry et al., Nat. Genet., 1: 44-49 (1992)]. Although the putative promoter region contains 59.3% C+G, it does not have additional attributes of CpG-rich islands that are typical for TATA-less promoters of housekeeping genes [Bird, Nature, 321: 209-213 (1986)]. Another class of genes lacking TATA box utilizes the initiator (lnr) element as a promoter. Many of these genes are not constitutively active, but they are rather regulated during differentiation or development. The Inr has a consensus sequence of PyPyPyCAPyPyPyPyPy [SEQ ID NO: 23] and encompasses the transcription start site [Smale and Baltimore, Cell. 57: 103-113 (1989)]. There are two such consensus sequences in the MN putative promoter; however, they do not overlap the transcription start (Figure 6).

An interesting region was found in the middle of the MN gene. The region is about 1.4 kb in length [nt 4,600-6,000 of the genomic sequence; SEQ ID NO: 49] and spans from the 3' part of the 1 st intron to the end of the 5th exon. The region has the character of a typical CpG-rich island, with 62.8% C + G content and 82 CpG: 131 GpC dinucleotides. Moreover, there are multiple putative binding sites for transcription factors AP2 and Sp1 [Locker and Buzard, supra; Briggs et al., Science, 234: 47-52 (1986)] concentrated in the center of this area. Particularly the 3rd intron of 131 bp in length contains three Sp1 and three AP2 consensus sequences. That data indicates the possible involvement of that region in the regulation of MN gene expression. However, functionality of that region, as well as other regulatory elements found in the proposed 5' MN promoter, remains to be determined.

### MN Promoter

Study of the MN promoter has shown that it is TATA-less and contains regulatory sequences for AP-1, AP-2, as well as two p53 binding sites. The sequence of the 5' end of the 3.5 kb flanking region upstream of the MN gene has shown extensive homology to LTR of HERV-K endogenous retroviruses. Basal transcription activity of the promoter is very weak as proven by analyses using CAT and neo reporter genes. However, expression of the reporter genes is severalfold increased when driven from the 3.5 kb flanking region, indicating involvement of putative enhancers.

Functional characterization of the 3.5 kb MN 5' upstream region by deletion analysis lead to the identification of the [-173, +31] fragment [SEQ ID NO: 21] (also alternatively, but less preferably, the nearly identical -172, +31 fragment [SEQ ID NO: 91]) as the MN promoter. *In vitro* DNase I footprinting revealed the presence of five protected regions (PR) within the MN promoter. Detailed deletion analysis of the promoter identified PR 1 and 2 (numbered from the transcription start) as the most critical for transcriptional activity. PR4 [SEQ ID NO: 115] negatively affected transcription as its deletion led to increased promoter activity and was confirmed to function as a promoter-, position- and orientation-independent silencer element. Mutational analysis indicated that the direct repeat AGGGCacAGGGC [SEQ ID NO: 143] is required for efficient repressor binding. Two components of the repressor complex (35 and 42 kDa) were found to be in direct contact with PR4 by UV crosslinking. Increased cell density, known to induce MN expression, did not affect levels of PR4 binding in HeLa cells. Significantly reduced repressor level seems to be responsible for MN up-regulation in the case of tumorigenic CGL3 as compared to non-tumorigenic CGL1 HeLa x normal fibroblast hybrid cells.

### Utility of MN Promoter as a Tumor-Specific

### Promoter for Gene Therapy

Being investigated is whether the MN gene promoter can be used as a tumor-specific promoter to drive the expression of a suicide gene [thymidine kinase (tk) of HSV)] and mediate the direct and bystander killing of tumor cells. HSVtk gene transferred to tumor cells converts nucleoside analogue ganciclovir (GCV) to toxic triphosphates and mediates the death of transduced and also neighboring tumor cells. The control of HSVtk by the MN gene promoter would allow its expression only in tumor cells, which are permissive for the biosynthesis of MN protein, and selectively kill such tumor cells, but not normal cells in which MN expression is repressed.

A plasmid construct in which HSVtk was cloned downstream of the MN promoter region Bd3, containing both proximal and distant regulatory elements of MN, was prepared. That plasmid pMN-HSVtk was transfected to Rat2TK- cells and C33 human cervical carcinoma cells using calcium phosphate precipitation and lipofection, respectively. Transfectants were tested for expression of HSVtk and GVC sensitivity. Analysis of the transfectants has shown the remarkable cytotoxic in vitro effect of GVC even in low concentrations (up to 95% of cells killed).

Polyclonal rabbit antiserum against HSVtk, using fusion protein with GST in pGEX-3X, has been prepared to immunodetect HSVtk synthesized in transfected cells. This model system is being studied to estimate the bystander effect, the inhibition of cloning efficiency and invasiveness of transduced and GVC-treated cells to collagen matrices. A recombinant retroviral vector with the MN promoter-driven HSVtk is to be prepared to test its in vivo efficacy using an animal model (e.g., SCID-mouse).

### MN Promoter Analysis

Since the MN promoter is weak, a classical approach to study it would be limited due to the relatively low efficiency of transient transfections (up to 10%). Therefore, stable clonal cell lines expressing constructs containing the MN promoter fused to the CAT gene were prepared. In such clonal lines, 100% of the cells express the CAT gene driven from the MN promoter, and thus, the activity of the promoter is detectable easier than in transient experiments. Also, the promoter activity can be analysed repeatedly in the same cells under different conditions or treated by different factors and drugs. This approach allows for the study of the mechanisms underlying MN regulation at the level of transcription initiation.

Several types of transfections with promoter constructs linked to a reporter CAT gene (calcium precipitation, DEAE dextran combined with DMSO shock and/or chloroquine, as well as electroporation), different methods of CAT activity assay (scintillation method, thin layer chromatography) and several recipient cell lines differing in the level of MN expression and in transfection efficiency (HeLa, SiHa, CGL3, KATO III, Rat2TK and C33 cells). Activity of the MN promoter was detected preferably by the electroporation of CGL3 cells and thin layer chromatography. Further preferably, C33 cells cotransfected with MN promoter-CAT constructs and pSV2neo were used.
1. To detect basal activity of the MN promoter and to estimate the position of the core promoter, expression of the CAT gene from constructs pMN1 to pMN7 after transfection to CGL3 cells was analyzed. Plasmids with progressive 5' deletions were transfected into CGL3 cells and activity was analyzed by CAT assay. [8 µg of DNA was used for transfection in all cases except pBLV-LTR (2 µg).]
   Only very weak CAT activity was detected in cells transfected by pMN1 and pMN2 (containing respectively 933 bp and 600 bp of the promoter sequence). A little higher activity was exhibited with the constructs pMN3, pMN4 and pMN6 (containing respectively 446 bp, 243 bp and 58 bp of the promoter). A slight peak of activity was obtained with pMN5 (starting at position -172 with respect to the transcription start.) Thus, the function of the MN core promoter can be assigned to a region of approximately 500 bp immediately upstream from the MN transcription initiation site.
   Interestingly, the activity of the large Bd3 region (covering 3.5 kbp upstream of the transcription start) was severalfold higher than the activity of the core promoter. However, its level was still much iower than that exhibited by a positive control, i.e., BLV-LTR transactivated by Tax, and even lower than the activity of BLV-LTR without transactivation. That the activity of Bd3 was elevated in comparison to the core promoter suggests the presence of some regulatory elements. Such elements are most probably situated in the sequence between pMN1 and Bd3 (i.e. from -1 kbp to - 3.5 kbp) [SEQ ID NO: 58]. The cloning and transfection of several deletion versions of Bd3 covering the indicated region can be used to determine the location of the putative regulatory elements.
   Similar results were obtained from transfecting KATO III cells with Bd3 and pMN4. The transfected cells expressed a lower level of MN than the CGL3 cells. Accordingly, the activity of the MN promoter was found to be lower than in CGL3 cells.
2. In a parallel approach to study the MN promoter, an analysis based on G418 selection of cells transfected by plasmids containing the promoter of interest cloned upstream from the neo gene was made. This approach is suitable to study weak promoters, since its sensitivity is much higher than that of a standard CAT assay. The principle underlying the method is as follows: an active promoter drives expression of the neo gene which protects transfected cells from the toxic effect of G418, whereas an inactive promoter results in no neo product being made and the cells transfected thereby die upon the action of G418. Therefore, the activity of the promoter can be estimated according to the number of cell colonies obtained after two weeks of selection with G418. Three constructs were used in the initial experiments - pMN1 neo, pMN4neo and pMN7neo. As pMN7neo contains only 30 bp upstream of the transcription start site, it was considered a negative control. As a positive control, pSV2neo with a promoter derived from SV40 was used. Rat2TK cells were chosen as the recipient cells, since they are transfectable with high efficiency by the calcium precipitation method.
   After transfection, the cells were subjected to two weeks of selection. Then the medium was removed, the cells were rinsed with PBS, and the colonies were rendered visible by staining with methylene blue. The results obtained from three independent experiments corroborated the data from the CAT assays. The promoter construct pMN4neo exhibited higher transcriptional activity than pMN1neo. However, the difference between the positive control and pMN4neo was not so striking as in the CAT assay. That may have been due to both lower promoter activity of pSV2neo compared to Tax-transactivated pBLV-LTR and to different conditions for cell growth after transfection. From that point of view, stable transfection is probably more advantageous for MN expression, since the cells grow in colonies with close cell to cell contact, and the experiment lasts much longer, providing a better opportunity to detect promoter activity.
3. Stable transfectants expressing MN promoter-CAT chimeric genes were prepared by the cotransfection of relevant plasmids with pSV2neo. As recipient cells, HeLa cells were used first. However, no clones expressing the promoter-CAT constructs were obtained. That negative result was probably caused by homologic recombination of the transfected genomic region of MN (e.g. the promoter) with the corresponding endogenous sequence. On the basis of that experience, C33 cells derived from a HPV-negative cervical carcinoma were used. C33 cells do not express MN, since during the process of tumorigenesis, they lost genetic material including chromosomal region 9p which contains the MN gene. In these experiments, the absence of the MN gene may represent an advantage as the possibility of homologic recombinations is avoided.

### C33 Cells Transfected with MN Promoter-CAT Constructs

C33 cells expressing the CAT gene under MN promoter regions Bd3 (-3500/+31) [SEQ ID NO: 90] and MN5 (-172/+31) [SEQ ID NO: 91] were used for initial experiments to analyze the influence of cell density on the transcriptional activity of the MN promoter. The results indicated that signals generated after cells come into close contact activate transcription of the CAT protein from the MN promoter in proportion to the density of the cell culture. Interestingly, the data indicated that the MN protein is not required for this phase of signal transduction, since the influence of density is clearly demonstrated in MN-negative C33 cells. Rather, it appears that MN protein acts as an effector molecule produced in dense cells in order to perform a certain biological function (i.e., to perturb contact inhibition). Also interestingly, the MN promoter activity is detectable even in very sparse cell cultures suggesting that MN is expressed at a very low level also is sparse subconfluent culture.

Deletion Variants. Deletion variants of the Bd3-CAT promoter construct were then prepared. The constructs were cotransfected with pSV2neo into C33 cervical cells. After selection with G418, the whole population of stably transfected cells were subjected to CAT ELISA analysis. Expression of the deletion constructs resulted in the synthesis of similar levels of CAT protein to that obtained with the Bd3-CAT construct. On the basis of that preliminary data, the inventors proposed that sequences stimulating transcription of MN are located between -3506 and -3375 bp [SEQ ID NO: 92] upstream from the transcription start. That is the sequence exhibiting homology to HERV-K LTR.

However, transient transfection studies in CGL3 cells repeatedly revealed that the LTR region is not required for the enhancement of basal MN promoter activity. Further, results obtained in CGL3 cells indicate that the activating element is localized in the region from -933 to -2179 [SEQ ID NO: 110] with respect to transcription initiation site (the position of the region having been deduced from overlapping sequences in the Bd3 deletion mutants).

### Interaction of Nuclear Proteins with MN Promoter Sequences

In order to identify transcription factors binding to the MN promoter and potentially regulating its activity, a series of analyses using an electrophoretic mobility shift assay (EMSA) and DNase I footprinting analysis (FTP) were performed.

### EMSA

in the EMSA, purified promoter fragments MN4 (-243/+ 31) [SEQ ID NO: 93], MN5 (-172/+31) [SEQ ID NO: 91], MN6 (-58/+31) [SEQ ID NO: 94] and pMN7 (-30/+31) [SEQ ID NO: 95], labeled at the 3' ends by Klenow enzyme, were allowed to interact with proteins in nuclear extracts prepared from CGL1 and CGL3 cells. [40 µg of nuclear proteins were incubated with 30,000 cpm end-labeled DNA fragments in the presence of 2 µg poly(dldC).] DNA-protein complexes were analysed by PAGE (native 6%), where the complexes created extra bands that migrated more slowly than the free DNA fragments, due to the shift in mobility which is dependent on the moiety of bound protein.

The EMSA of the MN4 and MN5 promoter fragments revealed several DNA-protein complexes; however, the binding patterns obtained respectively with CGL1 and CGL3 nuclear extracts were not identical. There is a single CGL-1 specific complex.

The EMSA of the MN6 promoter fragment resulted in the formation of three identical complexes with both CGL1 and CGL3 nuclear extracts, whereas the MN7 promoter fragment did not bind any nuclear proteins.

The EMSA results indicated that the CGL1 nuclear extract contains a specific factor, which could participate in the negative regulation of MN expression in CGL1 cells. Since the specific DNA-protein complex is formed with MN4 (-243/+31) [SEQ. ID NO.: 93] and MN5 (-172/+31) [SEQ. ID NO.: 91] promoter fragments, but not with MN6 (-58/+31) [SEQ ID NO: 94], it appears that the binding site of the protein component of that specific complex is located between -173 and -58 bp [SEQ. ID NO.: 96] with respect to transcription initiation.

The next step was a series of EMSA analyses using double stranded (ds) oligonucleotides designed according to the protected regions in FTP analysis. A ds oligonucleotide derived from the protected region PR2 [covering the sequence from -72 to -56 bp (SEQ ID NO: 111)] of the MN promoter provided confirmation of the binding of the AP-1 transcription factor in competitive EMSA using commercial ds olignucleotides representing the binding site for AP-1.

EMSA of ds oligonucleotides derived from the protected regions of PR1 1 [-46 to -24 bp (SEQ ID NO: 112)], PR2 [-72 to -56 bp (SEQ ID NO: 111)], PR3 [-102 to - 85 (SEQ ID NO: 113)] and PR5 [-163 to -144 (SEQ ID NO: 114)] did not reveal any differences in the binding pattern of nuclear proteins extracted from CGL1 and CGL3 cells, indicating that those regions do not bind crucial transcription factors which control activation of the MN gene in CGL3, or its negative regulation in CGL1. However, EMSA of ds oligonucleotides from the protected region PR4 [-133 to -108; SEQ ID NO: 115] repeatedly showed remarkable quantitative differences between binding of CGL1 and CGL3 nuclear proteins. CGL1 nuclear proteins formed a substantially higher amount of DNA-protein complexes, indicating that the PR4 region contains a binding site for specific transcription factor(s) that may represent a negative regulator of MN gene transcription in CGL1 cells. That fact is in accord with the previous EMSA data which showed CCL-1 specific DNA-protein complex with the promoter fragments pMN4 (-243/+31; SEQ ID NO: 93) and pMN5 (-172/+31; SEQ ID NO: 91), but not with pMN6 (-58/+31; SEQ ID NO: 94).

To identify the protein involved or the formation of a specific complex with the MN promoter in the PR4 region, relevant ds oligonucleotides covalently bound to magnetic beads will be used to purify the corresponding transcription factor. Alternatively the ONE Hybrid System^{®} [Clontech (Palo Alto, CA (USA)] will be used to search for and clone transcription factors involved in regulation of the analysed promoter region. A cDNA library from HeLa cells will be used for that investigation.

### FTP

To determine the precise location of cis regulatory elements that participate in the transcriptional regulation of the MN gene, FTP was used. Proteins in nuclear extracts prepared respectively from CGL1 and CGL3 cells were allowed to interact with a purified ds DNA fragment of the MN promoter (MN4, -243/+ 31) [SEQ ID NO: 93] which was labeled at the 5' end of one strand. [MN4 fragments were labeled either at Xho1 site (-243/+ 31*) or at Xba1 site (*-243/+ 31).] The DNA-protein complex was then subjected to DNase I attack, which causes the DNA chain to break at certain bases if they are not in contact with proteins. [A control used BSA instead of DNase.] Examination of the band pattern of the denatured DNA after gel electrophoresis [8% denaturing gel] indicates which of the bases on the labeled strand were protected by protein.

FTP analysis of the MN4 promoter fragment revealed 5 regions (I-V) protected at both the coding and noncoding strand, as well as two regions (VI and VII) protected at the coding strand but not at the noncoding strand. Figure 6 indicates the general regions on the MN promoter that were protected.

The sequences of the identified protected regions (PR) were subjected to computer analysis using the SIGNALSCAN program to see if they corresponded to known consensus sequences for transcription factors. The data obtained by that computer analyses are as follows:
- PR I -: coding strand - AP-2, p53, GAL4 noncoding strand - JCV-repeated
- PR II -: coding strand - AP-1, CGN4 noncoding strand - TCF-1, dFRA, CGN4
- PR III -: coding strand - no known consensus sequence, only partial overlap of AP1 noncoding strand - 2 TCF-1 sites
- PR IV -: coding strand - TCF-1, ADR-1 noncoding strand - CTCF, LF-A1, LBP-1
- PR V -: coding strand - no known consensus motif noncoding strand - JCV repeated
- PR VI-: coding strand - no known consensus motif noncoding strand - T antigen of SV 40, GAL4
- PR VII-: coding strand - NF-uE4, U2snRNA.2 noncoding strand - AP-2, lgHC.12, MyoD.

In contrast to EMSA, the FTP analysis did not find any differences between CGL1 and CGL3 nuclear extracts. However, the presence of specific DNA-protein interactions detected in the CGL1 nuclear extracts by EMSA could have resulted from the binding of additional protein to form DNA protein-protein complex. If that specific protein did not contact the DNA sequence directly, its presence would not be detectable by FTP.

### EMSA Supershift Analysis

The results of the FTP suggests that transcription factors AP-1, AP-2 as well as tumor suppressor protein p53 are potentially involved in the regulation of MN expression. To confirm binding of those particular proteins to the MN promoter, a supershift analysis using antibodies specific for those proteins was performed. For this analysis, DNA-protein complexes prepared as described for EMSA were allowed to interact with MAbs or polyclonal antibodies specific for proteins potentially included in the complex. The binding of antibody to the corresponding protein results in an additional shift (supershift) in mobility of the DNA-protein-antibody complex which is PAGE visualized as an additional, more slowly migrating band.

By this method, the binding of AP-2 to the MN promoter was confirmed. However, this method did not evidence binding of the AP-1 transcription factor. It is possible that MN protein binds AP-1-related protein, which is antigenically different from the AP-1 recognized by the antibodies used in this assay.

Also of high interest is the possible binding of the p53 tumor suppressor protein to the MN promoter. It is well known that wt p53 functions as a transcription factor, which activates expression of growth-restricting genes and down-modulates, directly or indirectly, the expression of genes that are required for ongoing cell proliferation. Transient co-transfection experiments using the pMN4-CAT promoter construct in combination with wt p53 cDNA and mut p53 cDNA, respectively, suggested that wt p53, but not mut p53, negatively regulates expression of MN. In addition, one of two p53-binding sites in the MN promoter is protected in FTP analysis (Figure 6), indicating that it binds to the corresponding protein. Therefore, supershift analysis to prove that p53 binds to the MN promoter with two p53-specific antibodies, e.g. Mabs 421 and DO-1 [the latter kindly provided by Dr. Vojtesek from Masaryk Memorial Cancer Institute in Brno, Czech Republic] are to be performed with appropriate nuclear extracts, e.g. from MCF-7 breast carcinoma cells which express wt p53 at a sufficient level.

### Regulation of MN Expression and MN Promoter

MN appears to be a novel regulatory protein that is directly involved in the control of cell proliferation and in cellular transformation. In HeLa cells, the expression of MN is positively regulated by cell density. Its level is increased by persistent infection with LCMV. In hybrid cells between HeLa and normal fibroblasts, MN expression correlates with tumorigenicity. The fact that MN is not present in nontumorigenic hybrid cells (CGL1), but is expressed in a tumorigenic segregant lacking chromosome 11, indicates that MN is negatively regulated by a putative suppressor in chromosome 11.

Evidence supporting the regulatory role of MN protein was found in the generation of stable transfectants of NIH 3T3 cells that constitutively express MN protein. As a consequence of MN expression, the NIH 3T3 cells acquired features associated with a transformed phenotype: altered morphology, increased saturation density, proliferative advantage in serum-reduced media, enhanced DNA synthesis and capacity for anchorage-independent growth. Further, flow cytometric analyses of asynchronous cell populations indicated that the expression of MN protein leads to accelerated progression of cells through G1 phase, reduction of cell size and the loss of capacity for growth arrest under inappropriate conditions. Also, MN expressing cells display a decreased sensitivity to the DNA damaging drug mitomycin C.

Nontumorigenic human cells, CGL1 cells, were also transfected with the full-length MN cDNA. The same pSGSC-MN construct in combination with pSV2neo plasmid as used to transfect the NIH 3T3 cells was used. Out of 15 MN-positive clones (tested by SP-RIA and Western blotting), 3 were chosen for further analysis. Two MN-negative clones isolated from CGL1 cells transfected with empty plasmid were added as controls. Initial analysis indicates that the morphology and growth habits of MN-transfected CGL1 cells are not changed dramatically, but their proliferation rate and plating efficiency is increased.

### MN Promoter - Sense/Antisense Constructs

When the promoter region from the MN genomic clone, isolated as described above, was linked to MN cDNA and transfected into CGL1 hybrid cells, expression of MN protein was detectable immediately after selection. However, then it gradually ceased, indicating thus an action of a feedback regulator. The putative regulatory element appeared to be acting via the MN promoter, because when the full-length cDNA (not containing the promoter) was used for transfection, no similar effect was observed.

An "antisense" MN cDNA/MN promoter construct was used to transfect CGL3 cells. The effect was the opposite of that of the CGL1 cells transfected with the "sense" construct. Whereas the transfected CGL1 cells formed colonies several times larger than the control CGL1, the transfected CGL3 cells formed colonies much smaller than the control CGL3 cells. The same result was obtained by antisense MN cDNA transfection in SiHa and HeLa cells.

For those experiments, the part of the promoter region that was linked to the MN cDNA through a BamHl site was derived from a NcoI - BamHI fragment of the MN genomic clone [Bd3] and represents a region a few hundred bp upstream from the transcription initiation site. After the ligation, the joint DNA was inserted into a pBK-CMV expression vector [Stratagene]. The required orientation of the inserted sequence was ensured by directional cloning and subsequently verified by restriction analysis. The tranfection procedure was the same as used in transfecting the NIH 3T3 cells, but co-transfection with the pSV2neo plasmid was not necessary since the neo selection marker was already included in the pBK-CMV vector.

After two weeks of selection in a medium containing G418, remarkable differences between the numbers and sizes of the colonies grown were evident as noted above. Immediately following the selection and cloning, the MN-transfected CGL1 and CGL3 cells were tested by SP-RIA for expression and repression of MN, respectively. The isolated transfected CGL1 clones were MN positive (although the level was lower than obtained with the full-length cDNA), whereas MN protein was almost absent from the transfected CGL3 clones. However, in subsequent passages, the expression of MN in transfected CGL1 cells started to cease, and was then blocked perhaps evidencing a control feedback mechanism.

As a result of the very much lowered proliferation of the transfected CGL3 cells, it was difficult to expand the majority of cloned cells (according to SP-RIA, those with the lowest levels of MN), and they were lost during passaging. However, some clones overcame that problem and again expressed MN. It is possible that once those cells reached a higher quantity, that the level of endogenously produced MN mRNA increased over the amount of ectopically expressed antisense mRNA.

### Identification of Specific Transcription

### Factors Involved in Control of MN Expression

Control of MN expression at the transcription level involves regulatory elements of the MN promoter. Those elements bind transcription factors that are responsible for MN activation in tumor cells and/or repression in normal cells. The identification and isolation of those specific transcription factors and an understanding of how they regulate MN expression could result in their therapeutic utility in modulating MN expression.

EMSA experiments indicate the existence of an MN gene repressor. Using the One Hybrid System^{®} [Clontech (Palo Alto, CA); an in vivo yeast genetic assay for isolating genes encoding proteins that bind to a target, cis-acting regulatory element or any other short DNA-binding sequence; Fields and Song, Nature, 340: 245 (1989); Wu et al., EMBO J., 13: 4823 (1994)] and subtractive suppressive PCR (SSH). SSH allows the cloning of genes that are differentially expressed under conditions which are known to up or down regulate MN expression such as density versus sparsity of HeLa cells, and suspension versus adherent HeLa cells.

In experiments with HPV immobilized cervical cells (HCE 16/3), it was found that the regulation of MN expression differs from that in fully transformed carcinoma cells. For example, glucocorticoid hormones, which activate HPV transcription, negatively regulate MN expression in HCE, but stimulate MN in HeLa and SiHa. Further keratinocyte growth factors, which down regulates transcription of HPV oncogenes, stimulates MN expression in suspension HCE but not in adherent cells.

EGF and insulin are involved in the activation of MN expression in both immortalized and carcinoma cells. All the noted facts can be used in the search for MN-specific transcription factors and in the modulation of MN expression for therapeutic purposes.

### Deduced Amino Acid Sequence

The ORF of the MN cDNA shown in Figure 1 has the coding capacity for a 459 amino acid protein with a calculated molecular weight of 49.7 kd. The overall amino acid composition of the MN/CA IX protein is rather acidic, and predicted to have a pi of 4.3. Analysis of native MN/CA IX protein from CGL3 cells by two-dimensional electrophoresis followed by immunoblotting has shown that in agreement with computer prediction, the MN/CA IX is an acidic protein existing in several isoelectric forms with pls ranging from 4.7 to 6.3.

As assessed by amino acid sequence analysis, the deduced primary structure of the MN protein can be divided into four distinct regions. The initial hydrophobic region of 37 amino acids (aa) corresponds to a signal peptide. The mature protein has an N-terminal or extracellular part of 377 amino acids [aa 38-414 (SEQ ID NO: 87], a hydrophobic transmembrane segment of 20 amino acids [aa 415-434 (SEQ ID NO: 52)] and a C-terminal region of 25 amino acids [aa 435-459 (SEQ ID NO: 53)].

The extracellular part is composed of two distinct domains: (1) a proteoglycan-like domain [aa 53-111 (SEQ ID NO: 50)]; and (2) a CA domain, located close to the plasma membrane [aa 135-391 (SEQ ID NO: 51)]. [The amino acid numbers are keyed to those of Figure 1.]

More detailed insight into MN protein primary structure disclosed the presence of several consensus sequences. One potential N-glycosylation site was found at position 346 of Figure 1. That feature, together with a predicted membrane-spanning region are consistent with the results, in which MN was shown to be an N-glycosylated protein localized in the plasma membrane. MN protein sequence deduced from cDNA was also found to contain seven S/TPXX sequence elements [SEQ ID NOS: 25 AND 26] (one of them is in the signal peptide) defined by Suzuki, J. Mol. Biol., 207: 61-84 (1989) as motifs frequently found in gene regulatory proteins. However, only two of them are composed of the suggested consensus amino acids.

Experiments have shown that the MN protein is able to bind zinc cations, as shown by affinity chromatography using Zn-charged chelating sepharose. MN protein immunoprecipitated from HeLa cells by Mab M75 was found to have weak catalytic activity of CA. The CA-like domain of MN has a structural predisposition to serve as a binding site for small soluble domains. Thus, MN protein could mediate some kind of signal transduction.

MN protein from LCMV-infected HeLA cells was shown by using DNA cellulose affinity chromatography to bind to immobilized double-stranded salmon sperm DNA. The binding activity required both the presence of zinc cations and the absence of a reducing agent in the binding buffer.

### CA Domain Required for Anchorage

### Independence But for Increased

### Proliferation of Transfected NIH 3T3 Fibroblasts

In transfected NIH 3T3 fibroblasts, MN protein induces morphologic transformation, increased proliferation and anchorage independence. The consequences of constitutive expression of two MN-truncated variants in NIH 3T3 cells were studied. It was found that the proteoglycan-like region is sufficient for the morphological alteration of transfected cells and displays the growth-promoting activity presumably related to perturbation of contact inhibition.

The CA domain is essential for induction of anchorage independence, whereas the TM anchor and IC tail are dispensable for that biological effect. The MN protein is also capable of causing plasma membrane ruffling in the transfected cells and appears to participate in their attachment to the solid support. The data evince the involvement of MN in the regulation of cell proliferation, adhesion and intercellular communication.

### Sequence Similarities

Computer analysis of the MN cDNA sequence was carried out using DNASIS and PROSIS (Pharmacia Software packages). GenBank, EMBL, Protein Identification Resource and SWISS-PROT databases were searched for all possible sequence similarities. In addition, a search for proteins sharing sequence similarities with MN was performed in the MIPS databank with the FastA program [Pearson and Lipman, PNAS (USA), 85: 2444 (1988)].

The proteoglycan-like domain [aa 53-111 (SEQ ID NO: 50)], which is between the signal peptide and the CA domain, shows significant homology (38% identity and 44% positivity) with a keratan sulphate attachment domain of a human large aggregating proteoglycan aggrecan [Doege et al., J. Biol. Chem., 266: 894-902 (1991)].

The CA domain [aa 135-391 (SEQ ID NO: 51)] is spread over 265 aa and shows 38.9% amino acid identity with the human CA VI isoenzyme [Aldred et al., Biochemistry, 30: 569-575 (1991)]. The homology between MN/CA IX and other isoenzymes is as follows: 35.2% with CA II in a 261 aa overlap [Montgomery et al., Nucl. Acids. Res., 15: 4687 (1987)], 31.8% with CA I in a 261 aa overlap [Barlow et al., Nucl. Acids Res., 15: 2386 (1987)], 31.6% with CA IV in a 266 aa overlap [Okuyama et al., PNAS (USA) 89: 1315-1319 (1992)], and 30.5% with CA III in a 259 aa overlap (Lloyd et al., Genes. Dev., 1: 594-602 (1987)].

In addition to the CA domain, MN/CA IX has acquired both N-terminal and C-terminal extensions that are unrelated to the other CA isoenzymes. The amino acid sequence of the C-terminal part, consisting of the transmembrane anchor and the intracytoplasmic tail, shows no significant homology to any known protein sequence.

The MN gene was clearly found to be a novel sequence derived from the human genome. The overall sequence homology between the cDNA MN sequence and cDNA sequences encoding different CA isoenzymes is in a homology range of 48-50% which is considered by ones in the art to be low. Therefore, the MN cDNA sequence is not closely related to any CA cDNA sequences.

Only very closely related nt sequences having a homology of at least 80-90% would hybridize to each other under stringent conditions. A sequence comparison of the MN cDNA sequence shown in Figure 1 and a corresponding cDNA of the human carbonic anhydrase II (CA II) showed that there are no stretches of identity between the two sequences that would be long enough to allow for a segment of the CA II cDNA sequence having 25 or more nucleotides to hybridize under stringent hybridization conditions to the MN cDNA or vice versa.

A search for nt sequences related to MN gene in the EMBL Data Library did not reveal any specific homology except for 6 complete and 2 partial Alu-type repeats with homology to Alu sequences ranging from 69.8% to 91 % [Jurka and Milosavljevic, J. Mol. Evol. 32: 105-121 (1991)]. Also a 222 bp sequence proximal to the 5' end of the genomic region is shown to be closely homologous to a region of the HERV-K LTR.

In general, nucleotide sequences that are not in the Alu or LTR-like regions, of preferably 25 bases or more, or still more preferably of 50 bases or more, can be routinely tested and screened and found to hybridize under stringent conditions to only MN nucleotide sequences. Further, not all homologies within the Alu-like MN genomic sequences are so close to Alu repeats as to give a hybridization signal under stringent hybridization conditions. The percent of homology between MN Alu-like regions and a standard Alu-J sequence are indicated as follows:

| Region of Homology within MN Genomic Sequence [SEQ ID NO: 5: Figure 2A-F] | SEQ. ID. NOS. | % Homology to Entire Alu-I Sequence |
|---|---|---|
| 921-1212 | 59 | 89.1% |
| 2370-2631 | 60 | 78.6% |
| 4587-4880 | 61 | 90.1% |
| 6463-6738 | 62 | 85.4% |
| 7651-7939 | 63 | 91.0% |
| 9020-9317 | 64 | 69.8% |

| | | % Homology to One Half of Alu-I Sequence |
|---|---|---|
| 8301-8405 | 65 | 88.8% |
| 10040-10122 | 66 | 73.2%. |

### MN Proteins and/or Polypeptides

The phrase "MN proteins and/or polypeptides" (MN proteins/polypeptides) is herein defined to mean proteins and/or polypeptides encoded by an MN gene or fragments thereof. An exemplary and preferred MN protein according to this invention has the deduced amino acid sequence shown in Figure 1. Preferred MN proteins/polypeptides are those proteins and/or polypeptides that have substantial homology with the MN protein shown in Figure 1. For example, such substantially homologous MN proteins/ polypeptides are those that are reactive with the MN-specific antibodies of this invention, preferably the Mabs M75, MN12, MN9 and MN7 or their equivalents.

A "polypeptide" or "peptide" is a chain of amino acids covalently bound by peptide linkages and is herein considered to be composed of 50 or less amino acids. A "protein" is herein defined to be a polypeptide composed of more than 50 amino acids. The term polypeptide encompasses the terms peptide and oligopeptide.

MN proteins exhibit several interesting features: cell membrane localization, cell density dependent expression in HeLa cells, correlation with the tumorigenic phenotype of HeLa x fibroblast somatic cell hybrids, and expression in several human carcinomas among other tissues. MN protein can be found directly in tumor tissue sections but not in general in counterpart normal tissues (exceptions noted infra as in normal gastric mucosa and gallbladder tissues). MN is also expressed sometimes in morphologically normal appearing areas of tissue specimens exhibiting dysplasia and/or malignancy. Taken together, these features suggest a possible involvement of MN in the regulation of cell proliferation, differentiation and/or transformation.

It can be appreciated that a protein or polypeptide produced by a neoplastic cell in vivo could be altered in sequence from that produced by a tumor cell in cell culture or by a transformed cell. Thus, MN proteins and/or polypeptides which have varying amino acid sequences including without limitation, amino acid substitutions, extensions, deletions, truncations and combinations thereof, fall within the scope of this invention. It can also be appreciated that a protein extant within body fluids is subject to degradative processes, such as, proteolytic processes; thus, MN proteins that are significantly truncated and MN polypeptides may be found in body fluids, such as, sera. The phrase "MN antigen" is used herein to encompass MN proteins and/or polypeptides.

It will further be appreciated that the amino acid sequence of MN proteins and polypeptides can be modified by genetic techniques. One or more amino acids can be deleted or substituted. Such amino acid changes may not cause any measurable change in the biological activity of the protein or polypeptide and result in proteins or polypeptides which are within the scope of this invention, as well as, MN muteins.

The MN proteins and polypeptides of this invention can be prepared in a variety of ways according to this invention, for example, recombinantly, synthetically or otherwise biologically, that is, by cleaving longer proteins and polypeptides enzymatically and/or chemically. A preferred method to prepare MN proteins is by a recombinant means. Particularly preferred methods of recombinantly producing MN proteins are described below for the GST-MN, MN 20-19, MN-Fc and MN-PA proteins.

### Recombinant Production of MN Proteins and Polypeptides

A representative method to prepare the MN proteins shown in Figure 1 or fragments thereof would be to insert the full-length or an appropriate fragment of MN cDNA into an appropriate expression vector as exemplified below. In Zavada et al., WO 93/18152, supra, production of a fusion protein GEX-3X-MN (now termed GST-MN) using the partial cDNA clone (described above) in the vector pGEX-3X (Pharmacia) is described. Nonglycosylated GST-MN (the MN fusion protein MN glutathione S-transferase) from XL1-Blue cells.

Zavada et al., WO 95/34650 describes the recombinant production of both a glycosylated MN protein expressed from insect cells and a nonglycosylated MN protein expressed from E. coli using the expression plasmid pEt-22b [Novagen Inc.; Madison, WI (USA)]. Recombinant baculovirus express vectors were used to infect insect cells. The glycosylated MN 20-19 protein was recombinantly produced in baculovirus-infected sf9 cells [Clontech; Palo Alto, CA (USA)]. The MN 20-19 protein misses the putative signal peptide (aas 1-37) of SEQ ID NO: 6 (Figure 1), has a methionine (Met) at the N-terminus for expression, and a Leu-Glu-His-His-His-His-His-His [SEQ. ID NO.: 22] added to the C-terminus for purification.

In order to insert the portion of the MN coding sequence for the GST-MN fusion protein into alternate expression systems, a set of primers for PCR was designed. The primers were constructed to provide restriction sites at each end of the coding sequence, as well as in-frame start and stop codons. The sequences of the primers, indicating restriction enzyme cleavage sites and expression landmarks, are shown below.

### Primer #20:N-terminus

Translation start
5'GTCGCTAGCTCCATGGGTCATATGCAGAGGTTGCCCCGGATGCAG 3'
NheI NcoI NdeI MN cDNA #1 [SEQ. ID. NO. 17]

### Primer #19:C-terminus

Translation stop
5'GAAGATCTCTTACTCGAGCATTCTCCAAGATCCAGCCTCTAGG 3'
BglII XhoI MN cDNA [SEQ. ID. NO. 18]

The SEQ ID NOS: 17 and 18 primers were used to amplify the MN coding sequence present in the GEX-3X-MN vector using standard PCR techniques. The resulting PCR product (termed MN 20-19) was electrophoresed on a 0.5% agarose/1X TBE gel; the 1.3 kb band was excised; and the DNA recovered using the Gene Clean II kit according to the manufacturer's instructions [Bio101; Lajolla, CA (USA)].

### Identification of MN Protein Partner(s)

A search for protein(s) interacting with MN was initiated using expression cloning of the corresponding cDNA(s) and a MN-Fc fusion protein as a probe. The chimerical MN-Fc cDNA was constructed in pSG5C vector by substitution of MN cDNA sequences encoding both the transmembrane anchor and the intracellular tail of MN protein with the cDNA encoding Fc fragment of the mouse IgG. The Fc fragment cDNA was prepared by RT-PCR from the mouse hybridoma producing IgG2a antibody.

The chimerical MN-Fc cDNA was expressed by transient transfection in COS cells. COS cells were transfected using leptofection. Recombinant MN-Fc protein was released to TC medium of the transfected cells (due to the lack of the transmembrane region), purified by affinity chromatography on a Protein A Sepharose and used for further experiments.

Protein extracts from mock-transfected cells and the cells transfected with pSG5C-MN-Fc were analysed by immunoblotting using the M75 MAb, SwaM-Px and ECL detection^{®} [ECL^{®} - enhanced chemoluminescent system to detect phosphorylated tyrosine residues; Amersham; Arlington, Hts., IL (USA)]. The size of MN-Fc protein expressed from the pSGSC vector corresponds to its computer predicted molecular weight.

³⁵S-labeled MN-Fc protein was employed in cell surface binding assay. It was found to bind to several mammalian cells, e.g., HeLa, Raji, COS, QT35, BL3. Similar results were obtained in cell adhesion assay using MN-Fc protein dropped on bacterial Petri dishes. These assays revealed that KATO III human stomach adenocarcinoma cell line is lacking an ability to interact with MN-Fc protein. This finding allowed us to use KATO III cells for expression cloning and screening of the cDNA coding for MN-binding protein.

The cDNA expression library in pBK-CMV vector was prepared from dense HeLa cells and used for transfection of KATO III cells. For the first round of screening, KATO III cells were transfected by electroporation. After two days of incubation, the ligand-expressing cells were allowed to bind to MN-Fc protein, then to Protein A conjugated with biotin and finally selected by pulling down with streptavidin-coated magnetic beads. Plasmid DNA was extracted from the selected cells and transformed to E. coli. Individual E. coli colonies were picked and pools of 8-10 clones were prepared. Plasmid DNA from the pools was isolated and used in the second round of screening.

In the second round of screening, KATO III cells were transfected by DEAE dextran method. To identify the pool containing the cDNA for MN-binding protein, an ELISA method based on the binding of MN-Fc to the transfected cells, and detection using peroxidase labelled Protein A were used. Pools are selected by ability to bind MN-Fc.

In the third round of screening, plasmid DNAs isolated from individual bacterial colonies of selected pools are transfected to KATO III cells. The transfected cells are subjected to binding with MN-Fc and detection with Protein A as before. Such exemplary screening is expected to identify a clone containing the cDNA which codes for the putative MN protein partner. That clone would then be sequenced and the expression product confirmed as binding to MN protein by cell adhesion assay. (Far-Western blotting, co-precipitation etc.) Hybridomas producing Mabs to the expression product would then be prepared which would allow the analysis of the biological characteristics of the protein partner of MN.

### Preparation of MN-Specific Antibodies

The term "antibodies" is defined herein to include not only whole antibodies but also biologically active fragments of antibodies, preferably fragments containing the antigen binding regions. Further included in the definition of antibodies are bispecific antibodies that are specific for MN protein and to another tissue-specific antigen.

Zavada et al., WO 93/18152 and WO 95/34650 describe in detail methods to produce MN-specific antibodies, and detail steps of preparing representative MN-specific antibodies as the M75, MN7, MN9, and MN12 monoclonal antibodies. Preferred MN antigen epitopes comprise: aa 62-67 (SEQ ID NO: 10); aa 61-66, aa 79-84, aa 85-90 and aa 91-96 (SEQ ID NO: 98); aa 62-65, aa 80-83, aa 86-89 and aa 92-95 (SEQ ID NO: 99); aa 62-66, aa 80-84, aa 86-90 and aa 92-96 (SEQ ID NO: 100); aa 63-68 (SEQ ID NO: 101); aa 62-68 (SEQ ID NO: 102); aa 82-87 and aa 88-93 (SEQ ID NO: 103); aa 55-60 (SEQ ID NO: 11); aa 127-147 (SEQ ID NO: 12); aa 36-51 (SEQ ID NO: 13); aa 68-91 (SEQ ID NO: 14); aa 279-291 (SEQ ID NO: 15); and aa 435-450 (SEQ ID NO: 16). Example 2 provides further description concerning preferred MN antigen epitopes.

Bispecific Antibodies. Bispecific antibodies can be produced by chemically coupling two antibodies of the desired specificity. Bispecific MAbs can preferably be developed by somatic hybridization of 2 hybridomas. Bispecific MAbs for targeting MN protein and another antigen can be produced by fusing a hybridoma that produces MN-specific MAbs with a hybridoma producing MAbs specific to another antigen. For example, a cell (a quadroma), formed by fusion of a hybridoma producing a MN-specific MAb and a hybridoma producing an anti-cytotoxic cell antibody, will produce hybrid antibody having specificity of the parent antibodies. [See, e.g., Immunol. Rev. (1979); Cold Spring Harbor Symposium Quant. Biol., 41: 793 (1977); van Dijk et al., lnt. J. Cancer, 43: 344-349 (1989).] Thus, a hybridoma producing a MN-specific MAb can be fused with a hybridoma producing, for example, an anti-T3 antibody to yield a cell line which produces a MN/T3 bispecific antibody which can target cytotoxic T cells to MN-expressing tumor cells.

It may be preferred for therapeutic and/or imaging uses that the antibodies be biologically active antibody fragments, preferably genetically engineered fragments, more preferably genetically engineered fragments from the V_{H} and/or V_{L} regions, and still more preferably comprising the hypervariable regions thereof. However, for some therapeutic uses bispecific antibodies targeting MN protein and cytotoxic cells would be preferred.

### Epitopes

The affinity of a MAb to peptides containing an epitope depends on the context, e.g. on whether the peptide is a short sequence (4-6 aa), or whether such a short peptide is flanked by longer aa sequences on one or both sides, or whether in testing for an epitope, the peptides are in solution or immobilized on a surface. Therefore, it would be expected by ones of skill in the art that the representative epitopes described herein for the MN-specific MAbs would vary in the context of the use of those MAbs.

The term "corresponding to an epitope of an MN protein/polypeptide" will be understood to include the practical possibility that, in some instances, amino acid sequence variations of a naturally occurring protein or polypeptide may be antigenic and confer protective immunity against neoplastic disease and/or anti-tumorigenic effects. Possible sequence variations include, without limitation, amino acid substitutions, extensions, deletions, truncations, interpolations and combinations thereof. Such variations fall within the contemplated scope of the invention provided the protein or polypeptide containing them is immunogenic and antibodies elicited by such a polypeptide or protein cross-react with naturally occurring MN proteins and polypeptides to a sufficient extent to provide protective immunity and/or anti-tumorigenic activity when administered as a vaccine.

### Epitope for M75 MAb

The M75 epitope is considered to be present in at least two copies within the 6X tandem repeat of 6 amino acids [aa 61-96 (SEQ ID NO: 97)] in the proteglycan domain of the MN protein. Exemplary peptides representing that epitope depending on the context may include the following peptides from that tandem repeat: EEDLPS (SEQ ID NO: 10; aa 62-67); GEEDLP (SEQ ID NO: 98; aa 61-66; aa 79-84; aa 85-90; aa 91-96); EEDL (SEQ ID NO: 99; aa 62-65; aa 80-83; aa 86-89; aa 92-95); EEDLP (SEQ ID NO. 100; aa 62-66; aa 80-84; aa 86-90; aa 92-96); EDLPSE (SEQ ID NO: 101; aa 63-68); EEDLPSE (SEQ ID NO: 102; aa 62-68); and DLPGEE (SEQ ID NO: 103; aa 82-87, aa 88-93).

Three synthetic peptides from the deduced aa sequence for the EC domain of the MN protein shown in Figure 1 were prepared. Those synthetic peptides are represented by aa 51-72 (SEQ ID NO: 104), aa 61-85 (SEQ ID NO: 105) and aa 75-98 (SEQ ID NO.: 106). Each of those synthetic peptides contains the motif EEDLP (SEQ ID NO: 100) and were shown to be reactive with the M75 MAb.

### Other Epitopes

Mab MN9. Monoclonal antibody MN9 (Mab MN9) reacts to the same epitope as Mab M75, as described above. As Mab M75, Mab MN9 recognizes both the GST-MN fusion protein and native MN protein equally well.

Mabs corresponding to Mab MN9 can be prepared reproducibly by screening a series of mabs prepared against an MN protein/polypeptide, such as, the GST-MN fusion protein, against the peptides representing the epitope for Mabs M75 and MN9. Alternatively, the Novatope system [Novagen] or competition with the deposited Mab M75 could be used to select mabs comparable to Mabs M75 and MN9.

Mab MN12. Monoclonal antibody MN12 (Mab MN12) is produced by the mouse lymphocytic hybridoma MN 12.2.2 which was deposited under ATCC HB 11647. Antibodies corresponding to Mab MN12 can also be made, analogously to the method outlined above for Mab MN9, by screening a series of antibodies prepared against an MN protein/polypeptide, against the peptide representing the epitope for Mab MN12. That peptide is aa 55 - aa 60 of Figure 1 [SEQ ID NO: 11]. The Novatope system could also be used to find antibodies specific for said epitope.

Mab MN7. Monoclonal antibody MN7 (Mab MN7) was selected from mabs prepared against nonglycosylated GST-MN as described above. It recognizes the epitope represented by the amino acid sequence from aa 127 to aa 147 [SEQ ID NO: 12] of the Figure 1 MN protein. Analogously to methods described above for Mabs MN9 and MN12, mabs corresponding to Mab MN7 can be prepared by selecting mabs prepared against an MN protein/polypeptide that are reactive with the peptide having SEQ ID NO: 12, or by the stated alternative means.

### MN-Specific Intrabodies - Targeted Tumor Killing Via Intracellular

### Expression of MN-Specific Antibodies to

### Block Transport of MN Protein to Cell Surface

The gene encoding antibodies can be manipulated so that the antigen-binding domain can be expressed intracellularly. Such "intrabodies" that are targeted to the lumen of the endoplasmic reticulum provide a simple and effective mechanism for inhibiting the transport of plasma membrane proteins to the cell surface. [ Marasco, W.A., "Review - Intrabodies: turning the humoral immune system outside in or intracellular immunization," Gene Therapy. 4: 11-15 (1997); Chen et al., "intracellular antibodies as a new class of therapeutic molecules for gene therapy," Hum. Gene Ther., 5(5): 595-601 (1994); Mhashilkar et al., EMBO J., 14: 1542-1551 (1995); Mhashilkar et al., J. Virol., 71: 6486-6494 (1997); Marasco (Ed.), Intrabodies: Basic Research and Clinical Gene Therapy Applications, (Springer Life Sciences 1998; ISBN 3-540-64151-3) (summarizes preclinical studies from laboratories worldwide that have used intrabodies); Zanetti and Capra (Eds.), "Intrabodies: From Antibody Genes to Intracellular Communication," The Antibodies: Volume 4, [Harwood Academic Publishers; ISBN 90-5702-559-0 (Dec. 1997)]; Jones and Marasco, Advanced Drug Delivery Reviews, 31 (1-2): 153-170 (1998); Pumphrey and Marasco, Biodrugs. 9(3): 179-185 (1998); Dachs et al., Oncology Res., 9(6-7); 313-325 (1997); Rondon and Marasco, Ann. Rev. Microbiol.. 51: 257-283 (1997)]; Marasco, W.A., Immunotechnology, 1(1): 1-19 (1995); and Richardson and Marasco, Trends in Biotechnology, 13(8): 306-310 (1995).]

MN-specific intrabodies may prevent the maturation and transport of MN protein to the cell surface and thereby prevent the MN protein from functioning in an oncogenic process. Antibodies directed to MN's EC, TM or IC domains may be useful in this regard. MN protein is considered to mediate signal transduction by transferring signals from the EC domain to the IC tail and then by associating with other intracellular proteins within the cell's interior. MN-specific intrabodies could disrupt that association and perturb that MN function.

Inactivating the function of the MN protein could result in reversion of tumor cells to a non-transformed phenotype. [Marasco et al. (1997), supra.] Antisense expression of MN cDNA in cervical carcinoma cells, as demonstrated herein, has shown that loss of MN protein has led to growth suppression of the transfected cells. It is similarly expected that inhibition of MN protein transport to the cell surface would have similar effects. Cloning and intracellular expression of the M75 MAb's variable region is to be studied to confirm that expectation.

Preferably, the intracellularly produced MN-specific antibodies are single-chain antibodies, specifically single-chain variable region fragments or sFv, in which the heavy- and light-chain variable domains are synthesized as a single polypeptide and are separated by a flexible linker peptide, preferably (Gly₄-Ser)₃ [SEQ ID NO: 116].

MN-specific intracellularly produced antibodies can be used therapeutically to treat preneoplastic/neoplastic disease by transfecting preneoplastic/neoplastic cells that are abnormally expressing MN protein with a vector comprising a nucleic acid encoding MN-specific antibody variable region fragments, operatively linked to an expression control sequence. Preferably said expression control sequence would comprise the MN gene promoter.

### Antibody-Mediated Gene Transfer Using MN-Specific

### Antibodies or Peptides for Targeting MN-Expressing Tumor Cells

An MN-specific antibody or peptide covalently linked to polylysine, a polycation able to compact DNA and neutralize its negative charges, would be expected to deliver efficiently biologically active DNA into an MN-expressing tumor cell. If the packed DNA contains the HSVtk gene under control of the MN promoter, the system would have double specificity for recognition and expression only in MN-expressing tumor cells. The packed DNA could also code for cytokines to induce CTL activity, or for other biologically active molecules. The M75 MAb (or, for example, as a single chain antibody, or as its variable region) is exemplary of such a MN-specific antibody.

The following examples are for purposes of illustration only and are not meant to limit the invention in any way.

### Example 1

### Transient Transformation of

### Mammalian Cells by MN Protein

This example (1) examines the biological consequences of transfecting human or mouse cells with MN-cDNA inserted into expression vectors, mainly from the viewpoint of the involvement of MN protein in oncogenesis; (2) determines if MN protein exerts carbonic anhydrase activity, and whether such activity is relevant for morphologic transformation of cells; and (3) tests whether MN protein is a cell adhesion molecule (CAM).

### Synopsis

Methods: MN-cDNA was inserted into 3 expression vectors and was used for transfecting human or mouse cells. MN protein was detected by Western blotting, radioimmunoassay or immunoperoxidase staining; in all tests the MN-specific monoclonal antibody M75 (MAb M75) was used. Carbonic anhydrase activity was determined by the acidification velocity of carbonate buffer in CO₂ atmosphere.

Results: (1) Cells (human CGL-1 and mouse NIH3T3 cells) transfected with MN-cDNA showed morphologic transformation, but reverted to normal phenotype after 4-5 weeks. (2) This reversion was not due to the loss, silencing or mutation of the MN insert. (3) MN protein has the enzyme activity of a carbonic anhydrase, which can be inhibited with acetazolamide; however, the inhibition of the carbonic anhydrase enzyme activity did not affect transformation. (4) MN protein is an adhesion protein, involved in cell-to-cell contacts.

### Background

This example concerns transformation of mammalian cells by MN-cDNA inserted into expression vectors derived from retroviruses. Such vectors are suitable for efficient and stable integration into cellular DNA and for continuous expression of MN protein. Cells transfected with these constructs showed morphologic transformation, but after some time, they reverted to normal phenotype.

Sulfonamides, including acetazolamide, are very potent inhibitors of known carbonic anhydrases [Maren and Ellison, Mol. Pharmacol.. 3: 503-508 (1967)]. Acetazolamide was tested to determine if it inhibited also the MN-carbonic anhydrase, and if so, whether inhibition of the enzyme affected cell transformation.

There are reasons to believe that MN protein could be involved in direct cell-to-cell interactions: A) previous observations indicated a functional resemblance of MN protein to surface glycoproteins of enveloped viruses, which mediate virus adsorption to cell surface receptors, and MN participated in the formation of phenotypically mixed virions of vesicular stomatitis virus. B) Inducibility of MN protein expression by growing HeLa cells in densely packed monolayers suggests that it may be involved in direct interactions between cells. C) Finally, there is a structural similarity between the MN protein and receptor tyrosine phosphatase β, which also contains proteoglycan and carbonic anhydrase domains; those domains mediate direct contacts between cells of the developing nervous system [Peles et al., Cell. 82: 251-260 (1995)]. Therefore, MN protein was tested to see if it bound to cell surface receptors; the result was clearly positive that it does.

### Materials and Methods

### Cell Lines

Cells used in this example were: CGL1 and CGL3 - respectively non-tumorigenic and tumorigenic HeLa x fibroblast hybrids [Stanbridge et al., Somat. Cell Genet., 7: 699-712 (1981)], mouse cell line NIH3T3, HeLa cells and monkey Vero cells. The NIH3T3 cells were seeded at very low density to obtain colonies started from single cells. The most normal appearing colony, designated subclone 2, was picked for use in the experiments reported in this example.

### Expression Vectors

Full-length MN cDNA was acquired from a pBluescript subclone [Pastorek et al., Oncogene, 9: 2877-2888 (1994)]. To remove 5' and 3' noncoding sequences, that might reduce subsequent gene expression, a polymerase chain reaction (PCR) was performed. The 5' primer TAGACAGATCTACGATGGCTCCCCTGTGCCCCAG [SEQ ID NO: 88] encompasses a translation start site and Bg1II cloning site, and the 3' primer ATTCCTCTAGACAGTTACCGGCTCCCCCTCAGAT [SEQ ID NO: 89] encompasses a stop codon and XbaI cloning site. Full-length MN-cDNA as a template and Pfu DNA Polymerase [Stratagene; Lajolla, CA (USA)] were used in the reaction.

The PCR product was sequenced and found to be identical with the template; it carried no mutations. The PCR product harbouring solely the MN coding sequence was inserted into three vectors: 1. pMAMneo [Clontech; Palo Alto, CA (USA)] plasmid allowing dexamethasone-inducible expression driven by the MMTV-Long Terminal Repeat (LTR) promoter and containing a neo gene for selection of transformants in media supplemented with Geneticin (G418) antibiotics. 2. Retroviral expression vector pGD [Daley et al., Science, 247: 824-829 (1990); kindly provided by Prof. David Baltimore, New York-Cambridge)] containing MLV-LTR promoter and neo gene for G418 antibiotics selection. 3. Vaccinia virus expression vector pSC1 1 [Chakrabarti et al., Mol. Cell. Biol., 5: 3403-3409 (1985)]. Transfection was performed via a calcium-phosphate precipitate according to Sambrook et al. (eds.), Molecular cloning. A laboratory manual. 2nd ed., Cold Spring Harbor Laboratory Press (1989).

Vaccinia virus strain Praha clone 13 was used as parental virus [Kutinova et al., Vaccine, 13: 487-493 (1995)]. Vaccinia virus recombinant was prepared by a standard procedure [Perkus et al., Virology, 152: 285-297 (1986)]. Recombinant viruses were selected and plaque purified twice in rat thymidine-kinase-less RAT2 cells [Topp, W. C., Virology, 113: 408-411 (1981)] in the presence of 5'-bromodeoxyuridine (100 µg/ml). Blue plaques were identified by overlaying with agar containing 5-bromo-4-chloro-3-indolyl-β-D-galactopyranoside (X-Gal) (200 µg/ml).

### CA Assay

Carbonic anhydrase activity was measured by a micro-method [Brion et al., Anal. Biochem., 175: 289-297 (1988)]. In principle, velocity of the reaction CO₂ + H₂O - H₂CO₃ is measured by the time required for acidification of carbonate buffer, detected with phenol red as a pH indicator. This reaction proceeds even in absence of the enzyme, with to - control time (this was set to 60 seconds). Carbonic anhydrase reduces the time of acidification to t; one unit of the enzyme activity reduces the time to one half of control time: t/t₀= 1/2.

For the experiment, MN protein was immunoprecipitated with Mab M75 from RIPA buffer (1% Triton X-100, 0.1% deoxycholate, 1mM phenylmethylsulfonyl-fluoride and 200 trypsin-inhibiting units/ml of Trasylol in PBS, pH 7.2) extract of Vero cells infected with vaccinia-MN construct, after the cells developed cytopathic effect, or with "empty" vaccinia as a control. The MN + antibody complex was subsequently adsorbed to protein A - Staphylococcus aureus cells [Kessler, S. W., J. Immunol., 115: 1617-1624 (1975)] and rinsed 2x with PBS and 2x with 1 mM carbonate buffer, pH 8.0. The precipitate was resuspended in the same buffer and added to the reaction mixture. Acetazolamide (Sigma) was tested for inhibition of carbonic anhydrase [Maren and Ellison, supra]. In extracts of infected cells used for immunoprecipitation, the concentration of total proteins was determined by the Lowry method [ Lowry et al., L Biol. Chem.. 193: 265-275 (1951)] and that of MN protein by a competition radioimmunoassay as described in Zavada et al., Int. I. Cancer, 54: 268-274 (1993).

### Western Blots

Western blotting and development of the blots using ¹²⁵l-labelled M75 and autoradiography was performed as before [Pastorekova et al., Virology, 187: 620-626 (1992); and Zavada (1993), supra].

### Adhesion Assay

For the adhesion assay [Hoffman S., "Assays of cell adhesion," IN: Cell-cell Interactions, (Stevenson et al. eds.) pp. 1-30 (IRL Press at Oxford University Press; Oxford, N.Y., Tokyo; 1992)], 25 µl aliquots MN protein (affinity purified pGEX-3X MN) [Zavada et al. (1993), supra] or of control proteins were spotted on 5 cm-diameter bacteriological Petri dishes and allowed to bind for 2 hours at room temperature. This yielded circular protein-coated areas of 4-5 mm diameter. MN protein was diluted to 10 µg/ml in 50 mM carbonate buffer, pH 9.2. Patches of adsorbed control proteins were prepared similarly. Those included collagens type I and IV, fibronectin, laminin and gelatin (Sigma products), diluted and adsorbed according to the manufacturer's recommendations; FCS and BSA were also included. After aspiration of the drops, the dishes were rinsed 2x with PBS and saturated for 1 hour with DMEM supplied with 5% FCS. The plates were seeded with 5 x 10⁵ cells in 5 ml of DMEM + 5% FCS and incubated overnight at 37°C. The plates were rinsed with PBS, and the attached cells were fixed with formaldehyde, post-fixed with methanol and Giemsa stained.

### Results

### 1. Transformation and reversion of CGL1 cells transfected with MN-cDNA

Since the expression of MN protein correlated with the tumorigenicity of HeLa x fibroblast hybrids [Zavada et al. (1993), supra], the non-tumorigenic hybrid CGL1 cells were first tested. Those cells, transfected with the pMAM.MN construct, after selection with Geneticin, formed colonies with varying degrees of transformation; some of them appeared normal. While normal CGL1 cells are contact inhibited, growing in a parallel orientation, the transformed cells formed very dense colonies, showing the loss of contact inhibition. Such colonies grew more slowly than the original CGL 1.

After subcloning, the cells isolated from transformed colonies segregated revertants. The reversion was a gradual, step-wise process; there were colonies with different degrees of reversion. After 2 passages, all the cell population became a morphologically indistinguishable from normal CGL1. This was due to the reversion of some cells and to the selective advantage of the revertants, which grew faster than the transformed cells. Despite repeated attempts, not even one single stably transformed cell clone was obtained. No transformed colonies were found in CGL1 cells transfected with an "empty" pMAM control plasmid. Growth of the CGL1 + pMAM.MN revertants in media supplied with 5 µg/ml of dexamethasone for 7 days enhanced the production of MN protein, but the morphology of the cells did not return to transformed.

### 2. Rescue of transforming MN from the revertants

The reversion of MN-transformed cells to normal phenotype could have at least 4 causes: A) loss of the MN insert; B) silencing of the MN insert, e.g., by methylation; C) mutation of the MN insert; D) activation of a suppressor gene, coding for a product which neutralizes transforming activity of MN protein; E) loss of a MN-binding protein. To decide among those alternatives, the following experiment was designed.

MN-cDNA was inserted into pGD, a vector derived from mouse leukemia virus - MLV. A defective virus was thereby engineered, which contained the MN gene and the selective marker neo instead of genes coding for viral structural proteins. With this construct, mouse NIH3T3 cells were transfected. In media supplied with Geneticin, the cells formed colonies with phenotypes ranging from strongly transformed to apparently normal. All of the transformed colonies and about 50°/° of the normal colonies expressed MN protein. Contrasting with normal NIH3T3 cells, the transformants were also able to form colonies in soft agar, reflective of the loss of anchorage dependence, characteristic of cell transformation. Upon passaging, the cells isolated from transformed colonies reverted to normal morphology, and a same time, they lost the capacity to form colonies in soft agar, while still expressing the MN protein. This permanent presence of MN protein in revertants ruled out alternatives A) and B) supra, that is, loss or silencing of the MN gene as a cause of reversion.

To decide among the other 3 alternatives, the revertants were superinfected with live, replication competent MLV. This virus grows in NIH3T3 cells without any morphologic manifestations, and it works as a "helper" for the pGD.MN construct. Virus progeny from MLV-infected revertants represents an artificial virus complex [pGD.MN + MLV]. This consists of 2 types of virions: of standard type MLV particles and virions containing the pGD.MN genome, enveloped in structural proteins provided by the "helper" virus. This virus complex was infectious for fresh NIH3T3 cells; it again induced in them morphologic transformation and the capacity to form agar colonies.

Contrasting with NIH3T3 transfected with pGD.MN, all the colonies of cells infected with [pGD.MN + MLV] complex, which grew in the presence of Geneticin, were uniformly transformed and contained MN proteins. The transformants once more reverted to normal phenotype although they kept producing infectious [pGD.MN + MLV] complex, which induced transformation in fresh NIH3T3 cells. This cycle of infection-transformation-reversion was repeated 3 times with the same result. This ruled out alternative C) - mutation of MN-cDNA as a cause of reversion.

Normal NIH3T3 cells formed a contact inhibited monolayer of flat cells, which did not stain with Mab M75 and immunoperoxidase. Cells infected with [pGD.MN + MLV] complex were clearly transformed: they grew in a chaotic pattern and showed loss of contact inhibition. Some of the cells showed signs of apoptosis. Two passages later, the cell population totally reverted to original phenotype as a result of frequent emergence of revertants and of their selective advantages (faster growth and a higher efficiency of plating). In fact, the revertants appeared to grow to a somewhat lower saturation density than the original NIH3T3 cells, showing a higher degree of contact inhibition.

The control NIH3T3 cells did not contain any MN protein (Western blot); while both transformed cells and revertants contained the same amount and the same proportion of 54 and 58 kDa bands of MN protein. In a non-reducing gel, MN protein was present in the form of oligomers of 153 kDa. Consistently, by competition RIA, approximately 40 ng MN/mg total protein was found in both of the transformed cells and revertants.

### 3. Carbonic anhydrase activity and its inhibition

Since the carbonic anhydrase domain represents a considerable part of the MN protein (see Figure 8), tests were performed to determine whether it is indeed enzymatically active. Vero cells infected with the vaccinia.MN construct, which contained more of the MN protein than other cells used in the present experiments, served as a source of MN protein. The cells were extracted with RIPA buffer, and MN protein was concentrated and partially purified by precipitation with MAb M75 and SAC. The immunoprecipitate was tested for CA activity. 78 µl of precipitate contained 1 unit of the enzyme. From the extract, the concentration of total proteins and of MN protein was determined; 1 unit of enzyme corresponded to 145 ng of MN protein or to 0.83 mg of total protein. The immunoprecipitate from Vero cells infected with control virus had no enzyme activity. Activity of MN carbonic anhydrase was inhibited by acetazolamide; 1.53 x 10⁻⁸ M concentration of the drug reduced enzyme activity to 50%.

Preliminary tests showed that confluent cultures of HeLa or of NIH3T3 cells tolerated 10⁻⁵- 10⁻³M concentration of acetazolamide for 3 days without any signs of toxicity and without any effect on cell morphology. In sparse cultures, 10⁻⁵M acetazolamide did not inhibit cell growth, but 10⁻⁴M already caused a partial inhibition. Thus, 10⁻⁵M acetazolamide was added to NIH3T3 cells freshly transformed with the [pGD.MN + MLV] complex. After 4 days of incubation, the colonies were fixed and stained. No difference was seen between cells growing in the presence or absence of acetazolamide; both were indistinguishable from correctly transformed NIH3T3 cells. Thus, the enzymatic activity of carbonic anhydrase is not relevant for the transforming activity of MN protein.

### 4. Cell adhesion assay

To determine whether or not MN protein is a cell adhesion molecule (CAM), adhesion assays were performed in plastic bacteriological Petri dishes (not treated for use with tissue culture). Cells do not adhere to the surfaces of such dishes. unless the dishes are coated with a binding protein. NIH3T3 cells adhered, spread and grew on patches of adsorbed MN protein. Only very few cells attached outside the areas coated with MN protein.

Other variants of the experiment demonstrated that NIH3T3 cells adhered and spread on patches of adsorbed collagen I and IV, fibronectin and laminin. NIH3T3 cells did not attach to dots of adsorbed gelatin, FCS or BSA.

CGL1, HeLa and Vero cells also adhered to MN protein, but 3 leukemia cell lines showed no adherence. CGL3 cells, strongly expressing MN protein adhered less efficiently to MN protein dots then did CGL1. The presence of 10⁻⁴M acetazolamide in the media did not affect the cell adhesion.

To confirm the specificity of adhesion, MN protein was absorbed with SAC loaded with MAb M75 (directed to MN) or MAb M67, directed to an unrelated antigen (Pastorekova et al., supra), before it was applied to the surface of the Petri dishes. Absorption with the SAC-M75 complex totally abrogated the cell binding activity, whereas absorption with SAC-M67 was without any effect.

### Additional Cell Adhesion Results

A shortened MN, missing TM and IC segments, is shed into the medium by 5ET1 cells (a HeLa X fibroblast hybrid, analogous to CGL3 cells that express MN protein abundantly) or by Vero cells infected with W carrying MN-cDNA with deleted TM and IC sequences. The shed MN protein was purified from the media, and tested in cell adhesion assays. The cells adhered, spread and grew only on the patches covered with adsorbed complete MN protein, but not on the dots of MN lacking TM and IC regions. Analogous results have been described also for some other adhesion molecules. A variety of cells (NIH3T3, CGL1, CGL3, HeLa, XC) attached to MN protein dots suggesting that the MN receptor(s) is common on the surface of vertebrate cells.

Tests were also performed with extracellular matrix proteins or control proteins dotted on nitrocellulose. The dot-blots were treated with MN protein solution. Bound MN protein was detected with MAb M75. MN protein absorbed to the dots of collagen I and IV, but not to fibronectin, laminin, gelatine or BSA.

Prospects for therapy. There are many new principles of cancer therapy employing oncoproteins or molecules that interact with them as targets [Mendelsohn and Lippman, "Principles of molecular cell biology of cancer: growth factors," In: DeVita et al., eds., Cancer principles and practice of oncology pp. 114-133 4th ed., Philadelphia: Lippinocott (1993); DeVita et al., eds., Biologic therapy of cancer, 2nd ed., Philadelphia: Lippinocott (1995)]. The MN protein and at least some of its ligands (or receptors) appear to be particularly suitable for such purposes.

### Example 2

### Identification of MN's Binding Site

MN protein is a tumor-associated cell adhesion molecule (CAM). To identify its binding site, a series of overlapping oligopeptides, spanning the N-terminal domain of the MN protein were synthesized. The N-terminal domain is homologous to that of proteoglycans and contains a tandem repeat of six amino acids.

The series of oligopeptides were tested by the cell adhesion assay procedure essentially as described above in Example 1. The synthetic oligopeptides were immobilized on hydrophobic plastic surfaces to see if they would mediate the attachment, spreading and growth of cells. Also investigated were whether the oligopeptides or antibodies inhibited attachment of cells (NIH3T3, HeLa and CGL1) to purified MN protein coated onto such plastic surfaces. The MN protein was affinity purified on agarose covalently linked to sulfonamide, as the MN protein encompasses a CA domain.

Several of the oligopeptides were found to be biologically active: (i) when immobilized onto the plastic, they mediate attachment of cells (NIH3T3, HeLa and to CGL1); (ii) when added to the media, they compete for attachment to cells with the immobilized MN protein; (iii) these oligopeptides, present in the media do not inhibit attachment of cells to TC plastic, but they prevent cell-cell adhesion and formation of intercellular contacts; (iv) treatment of immobilized MN protein and of active peptides with MAb M75 abrogates their affinity for the cells; and (v) the binding site of MN was determined to be closely related or identical to the epitope for MAb M75, at least two copies of which are located in the 6-fold tandem repeat of 6 amino acids [aa 61-96 (SEQ ID NO: 97)] in the proteoglycan-like domain of MN protein.

It was concluded that ectopically expressed MN protein most likely participates in oncogenesis by intervention into normal cell-cell contacts. MN's binding site represents a potential target for which therapeutic agents can be designed.

### Materials and Methods

Affinity chromatography of MN/CA IX. MN/CA IX was purified by a single cycle of adsorption - elution on sulfonamide-agarose, as described for other CAs [Falkbring et al., FEBS Letters, 24: 229 (1972)]. We used columns of p-aminoethylbenzenesulfonamide-agarose (Sigma). Columns with adsorbed MN/CA IX were extensively washed with PBS (NaCl 8.0 g/l, KCI 0.2 g/l, KH₂PO₄ 0.2 g/l, Na₂HPO₄ 1.15 g/l, pH = 7.2) and eluted with 0.1 mM acetazolamide (Sigma). All steps of purification were carried out at 0 - 5 °C, pH 7.2, at physiological concentration of salts. Complete MN/CA IX+ was extracted with 1% Triton X-100 in PBS from Vero cells infected with vaccinia virus containing an insert of complete coding region of MN/CA IX as described in Zavada et al., Int. J. Oncol., 10: 857 (1997). Before chromatography, the extract was diluted 1:6 with PBS and centrifuged for that 1500 xg. Truncated MN/CA IX ΔTM ΔIC was produced from an analogous construct except that the 3' downstream primer for PCR was: 5' CGT CTA GAA GGA ATT CAG CTA GAC TGG CTC AGC A 3' [SEQ ID NO: 117]. MN/CA IXΔ was shed into the medium, from which it was affinity purified after centrifugation as above. All steps of purification were monitored by dot-blots.

Cells and media. The following cell lines were used: HeLa, CGL1 = non-tumorigenic hybrid HeLa x fibroblast, CGL3 = tumorigenic segregant from this hybrid, NIH3T3 cells = mouse fibroblasts. The origin of the cells and growth media are described in Zavada et al., Int. J. Cancer, 54: 268 (1993) and Zavada et al., Int. J. Oncol., 10: 857 (1997). In addition, we used also HT29, a cell line derived from colorectal carcinoma (ATCC No. HBT-38).

Cell adhesion assay. The conditions of the assay are basically as described in Example 1. Briefly, 1*µ*g/ml of purified MN/CA IX in 50 mM mono/bicarbonate buffer, pH 9.2, was adsorbed in 30*µ*l drops on the bottom of bacteriological 5 cm Petri dishes for 1.5 hr. Then the drops were removed by aspiration and the dishes were 3x rinsed with PBS and blocked with 50% FCS in culture medium for 30 min. There were two variants of the test. In the first one, the whole bottom of the Petri dish was blocked with 50% FCS, and the dishes were seeded with 5 ml of cell suspension (10⁵ cells/ml). After overnight incubation, the cultures were rinsed with PBS, fixed and stained. In the other variant, only the area of adsorbed MN/CA IX was blocked and on top of MN/CA IX dots were added 30 µl drops of cell suspension in growth medium, containing added oligopeptides (or control without peptides). After incubation, rinsing and fixation, the cultures were stained with 0.5% Trypan blue in 50 mM Tris buffer pH 8.5 for 1 h, rinsed with water and dried. Stained areas of attached cells were extracted with 10% acetic acid, the extracts transferred to 96-well plates and absorbance was measured at 630 nm on microplate reader.

ELISA. Purified GST-MN [Zavada et al. (1993), supra] at concentration 10 ng /ml in carbonate buffer pH 9.2 was adsorbed for 3 h in Maxisorb strips (NUNC). After washing and blocking (1 h) with 0.05% Tween 20 in PBS, 50*µ*l/well of the antibody + antigen mixtures were added. Final dilution of MAb 75 ascites fluid was 10⁻⁶; concentration of the peptides varied according to their affinity for M75 so as to allow determination of 50% end-point. These mixtures were adsorbed for 1.5 h, followed by washing with Tween-PBS. Bound antibody was detected by anti mouse IgG conjugate with peroxidase (SwAM-Px, SEVAC, Prague), diluted 1:1000. In the color reaction OPD (o-phenylenediamine dihydrochloride, Sigma) 1mg/ml in 0.1 M citrate buffer pH 5.0 was used. To this H₂O₂ was added to final concentration 0.03%. This system is balanced so as to allow assay for antigen competing for M75 as well as for peptides binding to the epitope of immobilized GST-MN.

Peptides. The peptides used in this study were prepared by the solid phase method [Merrifield et al., IN: Gutte, B. (ed.), Peptides: Synthesis, Strucures and Applications, pp. 93-169 (San Diego; Academic Press; 1995)] using the Boc/Bzl strategy. The peptide acids were prepared on PAM-resin and peptide amides on MeBHA resin. Deprotection and splitting from the resin was done by liquid hydrogen fluoride. The peptides were purified by C18 RP HPLC and characterized by amino acid analysis and FAB MS spectroscopy.

Western blots. MN/CA IX antigens from PAGE gels were transferred to PVDF membranes (immobilon P, Millipore) and developed with M75, followed by SwAM-Px (see above) and diaminobenzidine (Sigma) with H₂O₂. For dot-blots we used nitrocellulose membranes.

Phage display. Ph.D.-7 Phage Display Peptide Library kit was used for screening as recommended by manufacturer (New England Biolabs). 96-well plate was coated with peptide SEQ ID NO: 106. Biopanning was carried out by incubating 2x10¹¹ phage with target coated plate for 1 h. Unbound phages were washed away with TBST (50mM Tris-HCl pH 7.5, 150 mM NaCl, 0.1% Tween-20) and specifically bound phages were eluted with M75 antibody (2*µ*g in 100*µ*l of TBS/well). Eluted phage was amplified and used for additional binding and amplification cycles to enrich the pool in favour of binding sequence. After 5 rounds, individual clones were picked, amplified and sequenced using T7 sequencing kit (Pharmacia).

### Results

Affinity chromatography of MN/CA IX protein. For purification of MN/CA IX protein we decided to use affinity chromatography on sulfonamide-agarose column, described previously for other CAs [Falkbring et al., supra]. The advantages of this method are simplicity and the fact that the whole procedure is carried out under non-denaturing conditions. Vaccinia virus vector with an insert of the complete MN/CA9 cDNA, or with truncated cDNA (lacking transmembrane and intracellular domains) was employed as a source of MN/CA IX protein.

A single cycle of adsorption - elution yielded relatively pure proteins: MN/CA IX+ gave 2 bands of 54 and 58 kDa, MN/CA IXΔ of 54.5 and 56 kDa. These proteins strongly reacted with MAb M75 on Western blots. In extracts from HeLa, CGL3 and HT29 the blot revealed 2 bands of the same size as MN/CA IX+ purified from vaccinia virus construct.

Adhesion of cells to MN/CA IX protein. MN/CA IX immobilized on hydrophobic plastic enabled attachment, spreading and growth of cells. Extremely low concentrations of MN/CA IX corresponding to 1 µg/ml of purified protein in adsorption buffer were sufficient to cause this effect; other cell adhesion molecules are used in 10-50x higher concentrations. Only complete MN/CA IX protein was active in cell adhesion test, truncated MN/CA IX did not support cell adhesion at all or it showed only a low adhesion activity and in some instances it even acted as a cell "repellent".

Treatment of the dots of immobilized MN/CA IX with MAb M75 abrogated its capacity to attach the cells, but the control MAb M16, irrelevant for MN/CA IX had no effect. Blocking of cell attachment by M75 shows that the epitope is identical to or overlapping with the binding site of MN/CA IX for cell receptors.

Identification of the epitope recognized by Mab M75. Preliminary mapping of M75 epitope employing partial sequences of extracellular parts of MN/CA9 cDNA expressed from bacterial vectors and tested on Western blots located it in PG region. For exact mapping, our strategy was to synthesize partially overlapping oligopeptides of 15-25 aa covering the PG domain and test them in competition ELISA with M75. According to the results, this was followed by a series of 6-12 aa oligopeptides. A major part of the PG domain consists of a 6-fold tandem repeat of 6 aa (aa 61 - 96) [SEQ ID NO: 97]; 4 repeats are identical (GEEDLP) [SEQ ID NO: 98] and 2 contain 2 aa exchanged (SEEDSP [SEQ ID NO: 141] and REEDPP [SEQ ID NO: 142]).

Following are the results of competition ELISA with recombinant MN/CA IX and oligopetides synthesized according to partial sequences of the PG region. MN/CA IX+ and Δ produced in mammalian cells possessed a higher serological activity than any other protein or peptide included in this experiment; fusion protein GST-MN synthesized in bacteria was less active. The following peptides span the PG region: GGSSGEDDPLGEEDLPSEEDSPC (aa 51-72) [SEQ ID NO: 104]; GEEDLPSEEDSPREEDPPGEEDLPGEC (aa 61-35) [SEQ ID NO: 105]; EDPPGEEDLPGEEDLPGEEDLPEVC (aa 75-98) [SEQ ID NO: 106]; and EVKPKSEEEGSLKLE (aa 97-111) [SEQ ID NO: 118]. SEQ ID NOS: 104 and 106 caused 50% inhibition at 1ng/ml. Those 2 oligopeptides are mutually non-overlapping, thus the epitope is repeated in both of them. SEQ ID NO: 105 was 1000x less active, probably due to a different conformation. SEQ ID NO: 118 was inactive; thus it does not contain the M75 epitope.

The next step for identifying the epitope was to synthesize oligopeptides containing all circular permutations of the motif GEEDLP [SEQ ID NO: 98] repeated twice. All 6 of the following dodecapeptides [SEQ ID NOS: 119-124] were serologically active (2 more and 4 less so): GEEDLPGEEDLP [SEQ ID NO: 119]; EEDLPGEEDLPG [SEQ ID NO: 120]; EDLPGEEDLP [SEQ ID NO: 121]; DLPGEEDLPGEE [SEQ ID NO: 122]; LPGEEDLPGEED [SEQ ID NO: 123]; and PGEEDLPGEEDL [SEQ ID NO: 124]. The following series of 7 aa sequences, flanked by alanine on both ends were tested: APGEEDLPA [SEQ ID NO: 125]; AGEEDLPGA [SEQ ID NO: 126]; AEEDLPGEA [SEQ ID NO: 127]; AEDLPGEEA [SEQ ID NO: 128]; ADLPGEEDA [SEQ ID NO. 129]; and ALPGEEDLA [SEQ ID NO: 130]. The results showed that the minimum serologically active sequence is the oligopeptide APGEEDLPA [SEQ ID NO: 125]. SEQ ID NOS: 127-130 proved negative in competition at 100*µ*g/ml. Further, none of the following still shorter oligopeptides (6 + 2aa) competed in ELISA for M75: AGEEDLPA [SEQ ID NO: 131]; AEEDLPGA [SEQ ID NO: 132]; AEDLPGEA [SEQ ID NO: 133]; ADLPGEEA [SEQ ID NO: 134]; ALPGEEDA [SEQ ID NO: 135]; and APGEEDLA [SEQ ID NO: 136].

In the oligopeptides of SEQ ID NOS: 104, 105, 106 and 118, the C-terminal amino acid was present as an acid, whereas in all the other oligopeptides, the C-terminal amino acid was present as an amide. It is clear that the affinity between these oligopeptides and MAb M75 very strongly increases with the size of peptide molecule.

### Attempts to demonstrate adhesion of cells to immobilized oligopeptides.

Our initial plan was to follow the pioneering work of Piersbacher and Ruoslahti, PNAS, 81: 5985 (1984). They linked tested oligopeptides to adsorbed bovine serum albumin by cross-linking agent SPDP (N-succinimidyl 3[pyridylhydro] propionate). This is why we added onto the C-end of oligopeptides SEQ ID NOS: 104-106 cysteine, which would enable oriented linking to adsorbed albumin. We demonstrated linking of the peptides directly in Petri dishes by immunoperoxidase staining with M75. Unfortunately, CGL1 or CGL3 cells adhered to control albumin treated with SPDP and blocked with ethanolamine (in place of oligopeptides) as strongly as to BSA dots with linked oligopeptides. We were unable to abrogate this non-specific adhesion. Oligopeptides SEQ ID NOS: 104-106 adsorb only very poorly to bacteriological Petri dishes, thereby not allowing the performance of the cell adhesion assay.

Alternatively, we tested inhibition of cell adhesion to MN/CA IX dots by oligopeptides added to the media together with the cell suspension, as described by Piersbacher and Ruoslahti, supra.. All peptides SEQ ID NOS: 104-106 and 118-136, were tested at concentrations of 100 and 10 µg/ml. None of them inhibited reproducibly the adhesion of CGL1 cells.

### Oligopeptides with affinity to M75 epitope which inhibit cell adhesion to

MN/CA IX. As an alternative to monoclonal antibodies, we set out to select oligopeptides exerting affinity to M75 epitope as well as to MN/CA IX receptor binding site from a phage display library of random heptapeptides - Ph.D.-7. Our aim was to select phages containing the desired heptapeptides by panning on immobilized peptide SEQ ID NO: 106 and subsequent elution with M75. Eluted phage was multiplied in appropriate bacteria and subjected to 4 more cycles of panning and elution. From the selected phage population, 10 plaques were picked, amplified and the heptapeptide-coding region was sequenced. Only 3 heptapeptides were represented. Those three heptapeptides, after adding alanine on both sides, are the following nonapeptides: AKKMKRRKA [SEQ ID NO: 137]; AITFNAQYA [SEQ ID NO: 138]; and ASASAPVSA [SEQ ID NO: 139]. The last heptapeptide, synthesized again with added terminal alanines as nonapeptide AGQTRSPLA [SEQ ID NO: 140], was identified by panning on GST-MN and eluted with acetazolamide. This last peptide has affinity to the active site of MN/CA IX carbonic anhydrase. We synthesized these peptides of 7 + 2 aa and tested them in competition ELISA and in cell adhesion inhibition. Both tests yielded essentially consistent results: peptide SEQ ID NO: 138 showed the highest activity, peptide SEQ ID NO: 137 was less active, peptide SEQ ID NO: 139 was marginally positive only in ELISA, and peptide SEQ ID NO: 140 was inactive. In all of those 4 nonapeptides, the C-terminal amide was present as amide.

### Discussion

Purification of transmembrane proteins like MN/CA IX often poses technical problems because they tend to form aggregates with other membrane proteins due to their hydrophobic TM segments. To avoid this, we engineered truncated MN/CA IX ΔICΔTM, which is secreted into the medium. Indeed, truncated MN/CA IX was obtained in higher purity than MN/CA IX+. Unfortunately, this protein was of little use for our purposes, since it was inactive in the cell adhesion assay. Such a situation has also been described for other cell adhesion molecules: their shed, shortened form either assumes an inactive conformation, or it adsorbs to hydrophobic plastic "upside down," while complete proteins adsorb by hydrophobic TM segments in the "correct" position.

MN/CA IX protein forms oligomers of 150 kDa, linked by disulfidic bonds. It was not known whether these are homo- or hetero-oligomers, but PAGE and Western blot analysis suggest that these are probably homo-oligomers, most likely trimers, since on the gel stained with Coomassie Blue no additional bands of intensity comparable to 2 bands specific for MN/CA IX appeared. It is also unlikely that there could exist an additional protein co-migrating with one of the 2 major MN/CA IX bands, since the intensity of their staining on the gel and on Western blots is well comparable.

There can be no doubt on the specificity of cell attachment to purified MN/CA IX+. It is abrogated by specific MAb M75, at a dilution 1:1000 of ascites fluid. This is a correction to our previous report in Zavada et al., Int. J. Oncol., 10: 857 (1997) in which we observed that MN/CA IX produced by vaccinia virus vector and fusion protein GST-MN support cell adhesion, but we did not realize that GST anchor itself contains another binding site, which is not blocked by M75.

MAb M75 reacts excellently with MN/CA IX under any circumstances - with native antigen on the surface of living cells, with denatured protein on Western blots and with antigen in paraffin sections of biopsies fixed with formaldehyde, suggesting that the epitope is small and contiguous. In competition ELISA the smallest sequence reactive with M75 was 7 + 2 aa, but the affinity between M75 and tested peptides strongly depended on their molecular weight. Complete MN/CA IX was 100,000x more active than the smallest serologically active peptide in terms of weight/volume concentration. In terms of molar concentration this difference would be 150,000,000x. Oligopeptides of intermediate size also showed intermediate activities. It remains to be elucidated whether such differences in activity are due to the conformation depending on the size of the molecule, or to the fact that complete MN/CA IX contains several copies of the epitope, but the smallest molecule only one.

Considering the possibility that the epitope is identical with the cell adhesion structure in MN/CA IX, we can understand why we failed to detect inhibition of cell adhesion by the oligopeptides. The binding site is just not as simple as the prototype peptide, RGD [Winter, J., IN Cleland and Craik (eds.), Protein Engineering. Principles and Practice, pp. 349-369 (N.Y.; Wiley-Liss; 1996)].

Naturally, one can argue that the size of MN/CA IX is about the same as of immunoglobulin molecule, and that binding of M75 to its epitope may sterically hinder a different sequence of cell attachment site. This objection has been made unlikely by blocking of both M75 epitope and of cell binding site by nonapeptides 7 + 2 aa. That result strongly suggests that the epitope and the binding site are indeed identical.

MN/CA IX and its PG region in particular appears to be a potential target molecule for therapy for the following reasons: (i) it is exposed on the cell surface; (ii) it is present in high percentage of certain human carcinomas; (iii) it is normally expressed MN/CA IX in the mucosa of alimentary tract which is not accessible to circulating antibodies, in contrast with the tumors; (iv) it is not shed (or only minimally) into the body fluids; (v) the motif GEEDLP [SEQ ID NO: 98] is repeated 18 x on the surface of every MN/CA IX molecule. Oligopeptide display libraries are being employed in the first steps to develop new drugs [Winter, J., supra]. Selected oligopeptides can serve as lead compounds for the computerized design of new molecules, with additional properties required from a drug [DeCamp et al., IN Cleland and Craik (eds.), supra at pp. 467-505].

### Example 3

### Identification of Peptides Binding to

### MN Protein Using Phage Display

(a) To identify peptides that are recognized by MN protein, a heptapeptide phage display library [Ph.D.^{®}-7 Peptide 7-mer Library Kit (phage display peptide library kit); New England Biolabs; Beverly, MA (USA)] was screened. In screening the library, a selection process, i.e., biopanning [Parmley and Smith, Gene, 73: 308 (1988); Noren, C.J., NEB Transcript. 8(1): 1 (1996)] was carried out by incubating the phages encoding the peptides with a plate coated with MN protein, washing away the unbound phage, eluting and amplifying the specifically bound phage.
   The target MN protein in this process was a glutathione-S-transferase (GST) MN fusion protein (GST-MN). GST-MN is a recombinantly produced fusion protein expressed from pGEX-3X-MN containing the cDNA for the MN protein without the signal peptide. GST-MN was produced in bacteria under modified cultivation conditions (decreased optical density, decreased temperature). Such cultivation prevented premature termination of translation and resulted in synthesis of the protein molecules which were in vast majority of the full length. The GST-MN protein was used for coating of the wells and binding the relevant phages. The bound phages were then eluted by acetazolamide, amplified and used for two additional rounds of screening.
   After sequencing of several independent phage clones obtained after the third round of screening, the following heptapeptides were obtained:
   (1) GETRAPL (SEQ ID NO: 107)
   (2) GETREPL (SEQ ID NO: 108)
   (3) GQTRSPL (SEQ ID NO: 109)
   (4) GQTRSPL ( " )
   (5) GQTRSPL ( " )
   (6) GQTRSPL ( " )
   (7) GQTRSPL ( " )
   The heptapeptides show very similar or identical sequences indicating that the binding is specific. The fact that phages bearing these heptapeptides were eluted by acetazolamide, an inhibitor of carbonic anhydrase activity, indicates that the peptides bind to the CA domain of MN protein.
(b) Analogous screening of the heptapeptide phage display library is done using collagen I, shown to bind MN protein, for elution of phages. Different peptide(s) binding to different part(s) of the MN protein molecule are expected to be identified. After identifying such MN-binding peptides, the corresponding synthetic peptides shall then be analysed for their biological effects.

### Example 4

### Accessibility In Vivo of MN Protein Expressed in Tumor Cells and in Stomach

Lewis rats (384g) carrying a BP6 subcutaneous tumor (about 1 cm in diameter) expressing rat MN protein were injected intraperitoneally (i.p.) with ¹²⁵I-M75 Mab (2.5 x 10⁶ cpm). Five days later, 0.5-1g pieces of the tumor and organs were weighed and their radioactivity was measured by a gamma counter.

Table 2? summarizes the results. The highest radioactivity was present in the tumor. Relatively high radioactivity was found in the liver and kidney, apparently reflecting the clearance of mouse lgG from the blood. The stomach continued a relatively low level of radioactivity, indicating that the M75 Mab had only limited access to MN protein exposed in the gastric mucosa.

**TABLE 2**

| Distribution of radioactivity of ¹²⁵ I-M75 in rat organs and in the tumor | | | | | |
|---|---|---|---|---|---|
| Organ | | cpm/g | | | |
| Kidney | 2153 | 2184 | | | |
| Spleen | 653 | 555 | | | |
| Liver | 1993 | 1880 | | | |
| Lung | 1183 | 1025 | | | |
| Blood | 1449 | | | | |
| Heart | 568 | 477 | | | |
| Stomach | 1184 | 1170 | | | |
| Testis | 812 | 779 | | | |
| Tail | 647 | | | | |
| Tumor | 3646 | 4058 | 3333 | 8653 | 3839 |

### Example 5

### FACS Analysis of MN Protein Expression in CGL3 Cells- Apoptosis

A FACS investigation was designed to determine the conditions that influence the synthesis of MN protein and to analyse the cell cycle distribution of MN-positive versus MN-negative cells in a CGL3 population stimulated to apoptosis. Previous Western blotting analyses have shown CGL3 cells to express a relatively high amount of MN protein under different cultivation conditions. CGL3 cells are considered a constitutive producer of MN proteins. However, Western blotting does not recognize small differences in the level of protein. In contrast FACS allows the detection of individual MN-positive cells, a calculation of their percentage in the analysed population, an estimation of the level of MN protein in the cells, and a determination of the cell cycle distribution.

To study the effect of cultivation conditions on MN expression in CGL3 cells, the CGL3 cells were plated in different relative densities and serum concentrations. Three days after plating, the cells were collected, surface labeled by M75 Mab followed by FITC-conjugated anti-mouse IgG and immediately analysed by FACS.

The analysis showed that in adherent cells, MN expression is dependent on cell density as is HeLa cells. However, low density cultures still produced detectable amounts of MN protein. In low density cultures, serum concentration does not seem to play a role. In relatively high density cultures, a decreasing serum concentration resulted in slightly diminished MN expression, probably due to a lower density that the cells were able to reach during the three days of cultivation.

The effect of the actual cell density is remarkable, and MN expression (detectable in 15-90% of the cells) represents a very sensitive monitoring factor. In all experiments, there was about a 5% higher percentage of cycling cells in the MN-positive part of the population, compared to the MN-negative part. That fact prompted the analysis of the cell cycle distribution of MN-positive CGL3 cells under unfavorable growth conditions, that is, after induction of apoptosis.

### Apoptosis

CGL3 cells were stimulated to apoptotic death by several drugs, including cycloheximide, actimonycin D and dexamethasone. The FACS study showed that the onset of apoptosis is delayed in MN-positive cells suggesting a protective role of MN in this process. It was also observed that the induction of apoptosis resulted in the down-regulation of MN expression in a time-dependent manner. That same phenomenon was described for Bcl-2 anti-apoptotic protein, and there is existing opinion that the down-regulation of certain regulatory genes during apoptosis sensitizes the cells to undergo apoptotic death. To prove the role of MN in apoptosis, a similar study with cells transfected by MN cDNA is to be performed.

The preliminary results indicate the possible involvement of MN in the suppression of apoptosis. The recent view that tumors arise both as a consequence of increased proliferation and decreased cell death appears to be consistent with the association of the MN protein with tumors in vivo.

### ATCC Deposits

The materials listed below were deposited with the American Type Culture Collection (ATCC) now at 10810 University Blvd., Manassus, Virginia 20110-2209 (USA). The deposits were made under the provisions of the Budapest Treaty on the International Recognition of Deposited Microorganisms for the Purposes of Patent Procedure and Regulations thereunder (Budapest Treaty). Maintenance of a viable culture is assured for thirty years from the date of deposit. The hybridomas and plasmids will be made available by the ATCC under the terms of the Budapest Treaty, and subject to an agreement between the Applicants and the ATCC which assures unrestricted availability of the deposited hybridomas and plasmids to the public upon the granting of patent from the instant application. Availability of the deposited strain is not to be construed as a license to practice the invention in contravention of the rights granted under the authority of any Government in accordance with its patent laws.

| Hybridoma Deposit Date | | ATCC # |
|---|---|---|
| VU-M75 | September 17, 1992 | HB 11128 |
| MN 12.2.2 | June 9, 1994 | HB 11647 |
| | | |

| Plasmid | Deposit Date | ATCC # |
|---|---|---|
| A4a | June 6, 1995 | 97199 |
| XE1 | June 6, 1995 | 97200 |
| XE3 | June 6, 1995 | 97198 |

The description of the foregoing embodiments of the invention have been presented for purposes of illustration and description. They are not intended to be exhaustive or to limit the invention to the precise form disclosed, and obviously many modifications and variations are possible in light of the above teachings. The embodiments were chosen and described in order to explain the principles of the invention and its practical application to enable thereby others skilled in the art to utilize the invention in various embodiments and with various modifications as are suited to the particular use contemplated.

All references cited herein are hereby incorporated by reference.

## Claims

**1.** A polypeptide for use in inhibiting the growth of vertebrate preneoplastic or neoplastic cells that abnormally express MN protein, wherein said polypeptide binds specifically to a site on the MN protein to which vertebrate cells adhere in a cell adhesion assay, wherein said polypeptide when tested in vitro inhibits the adhesion of vertebrate cells to MN protein, and wherein said polypeptide comprises a peptide selected by screening a phage display peptide library for specific binding to the MN protein.

**2.** The polypeptide of Claim 1 wherein said peptide, selected by screening a phage display peptide library for specific binding to the MN protein, is from the group consisting of SEQ ID NOS: 107, 108, 109, 137 and 138.

**4.** The polypeptide of Claim 1 wherein the site on the MN protein, to which vertebrate cells adhere in a cell adhesion assay, is within the proteoglycan-like domain or within the carbonic anhydrase domain of the MN protein.

**5.** The polypeptide of Claim 1 wherein the site on the MN protein, to which vertebrate cells adhere in a cell adhesion assay, comprises an amino acid sequence selected from the group consisting of SEQ ID NOS: 10 and 97-106.

**6.** A repressor complex, comprising two proteins having molecular weights of 35 and 42 kilodaltons, respectively, that binds to SEQ ID NO: 115 of the MN gene promoter.

**7.** The repressor complex of Claim 6 that binds to SEQ ID NO: 143 of the MN gene promoter.

**8.** A vector comprising a nucleic acid that encodes a cytotoxic protein or cytotoxic polypeptide operatively linked to the MN gene promoter, wherein said vector, when transfected into a vertebrate preneoplastic or neoplastic cell that abnormally expresses MN protein, inhibits the growth of said cell.

**9.** The vector of Claim 8 wherein said cytoxic protein is HSV thymidine kinases.

**10.** The vector according to Claim 9 wherein said vector further comprises a nucleic acid encoding a cytokine operatively linked to said MN gene promoter.


SEQUENCE LISTING

<110> Zavada, Jan
      Pastorekova, Silvia
      Pastorek, Jaromir

<120> MN Gene and Protein

<130> D-0021.5 PCT

<140>
<141>

<150> 09/177,776
<151> 1998-10-23

<150> 09/178,115
<151> 1998-10-23

<160> 143

<170> PatentIn Ver. 2.0

<210> 1
<211> 1522
<212> DNA
<213> HUMAN

<220>
<221> CDS
<222> (13)..(1389)

<220>
<221> mat_peptide
<222> (124)..(1389)

<400> 1
acagtcagcc gc atg gct ccc ctg tgc ccc agc ccc tgg ctc cct ctg ttg 51
              Met Ala Pro Leu Cys Pro Ser Pro Trp Leu Pro Leu Leu
                      -35                 -30                 -25

atc ccg gcc cct gct cca ggc ctc act gtg caa ctg ctg ctg tca ctg    99
Ile Pro Ala Pro Ala Pro Gly Leu Thr Val Gln Leu Leu Leu Ser Leu
                -20                 -15                 -10

ctg ctt ctg atg cct gtc cat ccc cag agg ttg ccc cgg atg cag gag    147
Leu Leu Leu Met Pro Val His Pro Gln Arg Leu Pro Arg Met Gln Glu
            -5                  -1   1               5

gat tcc ccc ttg gga gga ggc tct tct ggg gaa gat gac cca ctg ggc 195
Asp Ser Pro Leu Gly Gly Gly Ser Ser Gly Glu Asp Asp Pro Leu Gly
10 15 20

gag gag gat ctg ccc agt gaa gag gat tca ccc aga gag gag gat cca 243
Glu Glu Asp Leu Pro Ser Glu Glu Asp Ser Pro Arg Glu Glu Asp Pro
25 30 35 40

ccc gga gag gag gat cta cct gga gag gag gat cta cct gga gag gag 291
Pro Gly Glu Glu Asp Leu Pro Gly Glu Glu Asp Leu Pro Gly Glu Glu
45 50 55

gat cta cct gaa gtt aag cct aaa tca gaa gaa gag ggc tcc ctg aag 339
Asp Leu Pro Glu Val Lys Pro Lys Ser Glu Glu Glu Gly Ser Leu Lys
60 65 70

tta gag gat cta cct act gtt gag gct cct gga gat cct caa gaa ccc 387
Leu Glu Asp Leu Pro Thr Val Glu Ala Pro Gly Asp Pro Gln Glu Pro
75 80 85

cag aat aat gcc cac agg gac aaa gaa ggg gat gac cag agt cat tgg 435
Gln Asn Asn Ala His Arg Asp Lys Glu Gly Asp Asp Gln Ser His Trp
90 95 100

cgc tat gga ggc gac ccg ccc tgg ccc cgg gtg tcc cca gcc tgc gcg 483
Arg Tyr Gly Gly Asp Pro Pro Trp Pro Arg Val Ser Pro Ala Cys Ala
105 110 115 120

ggc cgc ttc cag tcc ccg gtg gat atc cgc ccc cag ctc gcc gcc ttc 531
Gly Arg Phe Gln Ser Pro Val Asp Ile Arg Pro Gln Leu Ala Ala Phe
125 130 135

tgc ccg gcc ctg cgc ccc ctg gaa ctc ctg ggc ttc cag ctc ccg ccg 579
Cys Pro Ala Leu Arg Pro Leu Glu Leu Leu Gly Phe Gln Leu Pro Pro
140 145 150

ctc cca gaa ctg cgc ctg cgc aac aat ggc cac agt gtg caa ctg acc 627
Leu Pro Glu Leu Arg Leu Arg Asn Asn Gly His Ser Val Gln Leu Thr
155 160 165

ctg cct cct ggg cta gag atg gct ctg ggt ccc ggg cgg gag tac cgg 675
Leu Pro Pro Gly Leu Glu Met Ala Leu Gly Pro Gly Arg Glu Tyr Arg
170 175 180

gct ctg cag ctg cat ctg cac tgg ggg gct gca ggt cgt ccg ggc tcg 723
Ala Leu Gln Leu His Leu His Trp Gly Ala Ala Gly Arg Pro Gly Ser
185 190 195 200

gag cac act gtg gaa ggc cac cgt ttc cct gcc gag atc cac gtg gtt 771
Glu His Thr Val Glu Gly His Arg Phe Pro Ala Glu Ile His Val Val
205 210 215

cac ctc agc acc gcc ttt gcc aga gtt gac gag gcc ttg ggg cgc ccg 819
His Leu Ser Thr Ala Phe Ala Arg Val Asp Glu Ala Leu Gly Arg Pro
220 225 230

gga ggc ctg gcc gtg ttg gcc gcc ttt ctg gag gag ggc ccg gaa gaa 867
Gly Gly Leu Ala Val Leu Ala Ala Phe Leu Glu Glu Gly Pro Glu Glu
235 240 245

aac agt gcc tat gag cag ttg ctg tct cgc ttg gaa gaa atc gct gag 915
Asn Ser Ala Tyr Glu Gln Leu Leu Ser Arg Leu Glu Glu Ile Ala Glu
250 255 260

gaa ggc tca gag act cag gtc cca gga ctg gac ata tct gca ctc ctg 963
Glu Gly Ser Glu Thr Gln Val Pro Gly Leu Asp Ile Ser Ala Leu Leu
265 270 275 280

ccc tct gac ttc agc cgc tac ttc caa tat gag ggg tct ctg act aca 1011
Pro Ser Asp Phe Ser Arg Tyr Phe Gln Tyr Glu Gly Ser Leu Thr Thr
285 290 295

ccg ccc tgt gcc cag ggt gtc atc tgg act gtg ttt aac cag aca gtg 1059
Pro Pro Cys Ala Gln Gly Val Ile Trp Thr Val Phe Asn Gln Thr Val
300 305 310

atg ctg agt gct aag cag ctc cac acc ctc tct gac acc ctg tgg gga 1107
Met Leu Ser Ala Lys Gln Leu His Thr Leu Ser Asp Thr Leu Trp Gly
315 320 325

cct ggt gac tct cgg cta cag ctg aac ttc cga gcg acg cag cct ttg 1155
Pro Gly Asp Ser Arg Leu Gln Leu Asn Phe Arg Ala Thr Gln Pro Leu
330 335 340

aat ggg cga gtg att gag gcc tcc ttc cct gct gga gtg gac agc agt 1203
Asn Gly Arg Val Ile Glu Ala Ser Phe Pro Ala Gly Val Asp Ser Ser
345 350 355 360

cct cgg gct gct gag cca gtc cag ctg aat tcc tgc ctg gct gct ggt 1251
Pro Arg Ala Ala Glu Pro Val Gln Leu Asn Ser Cys Leu Ala Ala Gly
365 370 375

gac atc cta gcc ctg gtt ttt ggc ctc ctt ttt gct gtc acc agc gtc 1299
Asp Ile Leu Ala Leu Val Phe Gly Leu Leu Phe Ala Val Thr Ser Val
380 385 390

gcg ttc ctt gtg cag atg aga agg cag cac aga agg gga acc aaa ggg 1347
Ala Phe Leu Val Gln Met Arg Arg Gln His Arg Arg Gly Thr Lys Gly
395 400 405

ggt gtg agc tac cgc cca gca gag gta gcc gag act gga gcc 1389
Gly Val Ser Tyr Arg Pro Ala Glu Val Ala Glu Thr Gly Ala
410 415 420

tagaggctgg atcttggaga atgtgagaag ccagccagag gcatctgagg gggagccggt 1449

aactgtcctg tcctgctcat tatgccactt ccttttaact gccaagaaat tttttaaaat 1509

aaatatttat aat 1522

<210> 2
<211> 459
<212> PRT
<213> HUMAN

<400> 2
Met Ala Pro Leu Cys Pro Ser Pro Trp Leu Pro Leu Leu Ile Pro Ala
-35 -30 -25

Pro Ala Pro Gly Leu Thr Val Gln Leu Leu Leu Ser Leu Leu Leu Leu
-20 -15 -10

Met Pro Val His Pro Gln Arg Leu Pro Arg Met Gln Glu Asp Ser Pro
-5 -1 1 5 10

Leu Gly Gly Gly Ser Ser Gly Glu Asp Asp Pro Leu Gly Glu Glu Asp
15 20 25

Leu Pro Ser Glu Glu Asp Ser Pro Arg Glu Glu Asp Pro Pro Gly Glu
30 35 40

Glu Asp Leu Pro Gly Glu Glu Asp Leu Pro Gly Glu Glu Asp Leu Pro
45 50 55

Glu Val Lys Pro Lys Ser Glu Glu Glu Gly Ser Leu Lys Leu Glu Asp
60 65 70 75

Leu Pro Thr Val Glu Ala Pro Gly Asp Pro Gln Glu Pro Gln Asn Asn
80 85 90

Ala His Arg Asp Lys Glu Gly Asp Asp Gln Ser His Trp Arg Tyr Gly
95 100 105

Gly Asp Pro Pro Trp Pro Arg Val Ser Pro Ala Cys Ala Gly Arg Phe
110 115 120

Gln Ser Pro Val Asp Ile Arg Pro Gln Leu Ala Ala Phe Cys Pro Ala
125 130 135

Leu Arg Pro Leu Glu Leu Leu Gly Phe Gln Leu Pro Pro Leu Pro Glu
140 145 150 155

Leu Arg Leu Arg Asn Asn Gly His Ser Val Gln Leu Thr Leu Pro Pro
160 165 170

Gly Leu Glu Met Ala Leu Gly Pro Gly Arg Glu Tyr Arg Ala Leu Gln
175 180 185

Leu His Leu His Trp Gly Ala Ala Gly Arg Pro Gly Ser Glu His Thr
190 195 200

Val Glu Gly His Arg Phe Pro Ala Glu Ile His Val Val His Leu Ser
205 210 215

Thr Ala Phe Ala Arg Val Asp Glu Ala Leu Gly Arg Pro Gly Gly Leu
220 225 230 235

Ala Val Leu Ala Ala Phe Leu Glu Glu Gly Pro Glu Glu Asn Ser Ala
240 245 250

Tyr Glu Gln Leu Leu Ser Arg Leu Glu Glu Ile Ala Glu Glu Gly Ser
255 260 265

Glu Thr Gln Val Pro Gly Leu Asp Ile Ser Ala Leu Leu Pro Ser Asp
270 275 280

Phe Ser Arg Tyr Phe Gln Tyr Glu Gly Ser Leu Thr Thr Pro Pro Cys
285 290 295

Ala Gln Gly Val Ile Trp Thr Val Phe Asn Gln Thr Val Met Leu Ser
300 305 310 315

Ala Lys Gln Leu His Thr Leu Ser Asp Thr Leu Trp Gly Pro Gly Asp
320 325 330

Ser Arg Leu Gln Leu Asn Phe Arg Ala Thr Gln Pro Leu Asn Gly Arg
335 340 345

Val Ile Glu Ala Ser Phe Pro Ala Gly Val Asp Ser Ser Pro Arg Ala
350 355 360

Ala Glu Pro Val Gln Leu Asn Ser Cys Leu Ala Ala Gly Asp Ile Leu
    365                 370                 375

Ala Leu Val Phe Gly Leu Leu Phe Ala Val Thr Ser Val Ala Phe Leu
380                 385                 390                 395

Val Gln Met Arg Arg Gln His Arg Arg Gly Thr Lys Gly Gly Val Ser
                400                 405                 410

Tyr Arg Pro Ala Glu Val Ala Glu Thr Gly Ala
            415                 420

<210> 3
<211> 29
<212> DNA
<213> HUMAN

<400> 3
cgcccagtgg gtcatcttcc ccagaagag                                    29

<210> 4
<211> 19
<212> DNA
<213> HUMAN

<400> 4
ggaatcctcc tgcatccgg                                               19

<210> 5
<211> 10898
<212> DNA
<213> HUMAN

<220>
<221> gene
<222> (1)..(10898)

<400> 5
ggatcctgtt gactcgtgac cttaccccca accctgtgct ctctgaaaca tgagctgtgt 60

ccactcaggg ttaaatggat taagggcggt gcaagatgtg ctttgttaaa cagatgcttg 120

aaggcagcat gctcgttaag agtcatcacc aatccctaat ctcaagtaat cagggacaca 180

aacactgcgg aaggccgcag ggtcctctgc ctaggaaaac cagagacctt tgttcacttg 240

tttatctgac cttccctcca ctattgtcca tgaccctgcc aaatccccct ctgtgagaaa 300

cacccaagaa ttatcaataa aaaaataaat ttaaaaaaaa aatacaaaaa aaaaaaaaaa 360

aaaaaaaaaa gacttacgaa tagttattga taaatgaata gctattggta aagccaagta 420

aatgatcata ttcaaaacca gacggccatc atcacagctc aagtctacct gatttgatct 480

ctttatcatt gtcattcttt ggattcacta gattagtcat catcctcaaa attctccccc 540

aagttctaat tacgttccaa acatttaggg gttacatgaa gcttgaacct actaccttct 600

ttgcttttga gccatgagtt gtaggaatga tgagtttaca ccttacatgc tggggattaa 660

tttaaacttt acctctaagt cagttgggta gcctttggct tatttttgta gctaattttg 720

tagttaatgg atgcactgtg aatcttgcta tgatagtttt cctccacact ttgccactag 780

gggtaggtag gtactcagtt ttcagtaatt gcttacctaa gaccctaagc cctatttctc 840

ttgtactggc ctttatctgt aatatgggca tatttaatac aatataattt ttggagtttt 900

tttgtttgtt tgtttgtttg tttttttgag acggagtctt gcatctgtca tgcccaggct 960

ggagtagcag tggtgccatc tcggctcact gcaagctcca cctcccgagt tcacgccatt 1020

ttcctgcctc agcctcccga gtagctggga ctacaggcgc ccgccaccat gcccggctaa 1080

ttttttgtat ttttggtaga gacggggttt caccgtgtta gccagaatgg tctcgatctc 1140

ctgacttcgt gatccacccg cctcggcctc ccaaagttct gggattacag gtgtgagcca 1200

ccgcacctgg ccaatttttt gagtctttta aagtaaaaat atgtcttgta agctggtaac 1260

tatggtacat ttccttttat taatgtggtg ctgacggtca tataggttct tttgagtttg 1320

gcatgcatat gctacttttt gcagtccttt cattacattt ttctctcttc atttgaagag 1380

catgttatat cttttagctt cacttggctt aaaaggttct ctcattagcc taacacagtg 1440

tcattgttgg taccacttgg atcataagtg gaaaaacagt caagaaattg cacagtaata 1500

cttgtttgta agagggatga ttcaggtgaa tctgacacta agaaactccc ctacctgagg 1560

tctgagattc ctctgacatt gctgtatata ggcttttcct ttgacagcct gtgactgcgg 1620

actatttttc ttaagcaaga tatgctaaag ttttgtgagc ctttttccag agagaggtct 1680

catatctgca tcaagtgaga acatataatg tctgcatgtt tccatatttc aggaatgttt 1740

gcttgtgttt tatgctttta tatagacagg gaaacttgtt cctcagtgac ccaaaagagg 1800

tgggaattgt tattggatat catcattggc ccacgctttc tgaccttgga aacaattaag 1860

ggttcataat ctcaattctg tcagaattgg tacaagaaat agctgctatg tttcttgaca 1920

ttccacttgg taggaaataa gaatgtgaaa ctcttcagtt ggtgtgtgtc cctngttttt 1980

ttgcaatttc cttcttactg tgttaaaaaa aagtatgatc ttgctctgag aggtgaggca 2040

ttcttaatca tgatctttaa agatcaataa tataatcctt tcaaggatta tgtctttatt 2100

ataataaaga taatttgtct ttaacagaat caataatata atcccttaaa ggattatatc 2160

tttgctgggc gcagtggctc acacctgtaa tcccagcact ttgggtggcc aaggtggaag 2220

gatcaaattt gcctacttct atattatctt ctaaagcaga attcatctct cttccctcaa 2280

tatgatgata ttgacagggt ttgccctcac tcactagatt gtgagctcct gctcagggca 2340

ggtagcgttt tttgtttttg tttttgtttt tcttttttga gacagggtct tgctctgtca 2400

cccaggccag agtgcaatgg tacagtctca gctcactgca gcctcaaccg cctcggctca 2460

aaccatcatc ccatttcagc ctcctgagta gctgggacta caggcacatg ccattacacc 2520

tggctaattt ttttgtattt ctagtagaga cagggtttgg ccatgttgcc cgggctggtc 2580

tcgaactcct ggactcaagc aatccaccca cctcagcctc ccaaaatgag ggaccgtgtc 2640

ttattcattt ccatgtccct agtccatagc ccagtgctgg acctatggta gtactaaata 2700

aatatttgtt gaatgcaata gtaaatagca tttcagggag caagaactag attaacaaag 2760

gtggtaaaag gtttggagaa aaaaataata gtttaatttg gctagagtat gagggagagt 2820

agtaggagac aagatggaaa ggtctcttgg gcaaggtttt gaaggaagtt ggaagtcaga 2880

agtacacaat gtgcatatcg tggcaggcag tggggagcca atgaaggctt ttgagcagga 2940

gagtaatgtg ttgaaaaata aatataggtt aaacctatca gagcccctct gacacataca 3000

cttgcttttc attcaagctc aagtttgtct cccacatacc cattacttaa ctcaccctcg 3060

ggctccccta gcagcctgcc ctacctcttt acctgcttcc tggtggagtc agggatgtat 3120

acatgagctg ctttccctct cagccagagg acatgggggg ccccagctcc cctgcctttc 3180

cccttctgtg cctggagctg ggaagcaggc cagggttagc tgaggctggc tggcaagcag 3240

ctgggtggtg ccagggagag cctgcatagt gccaggtggt gccttgggtt ccaagctagt 3300

ccatggcccc gataaccttc tgcctgtgca cacacctgcc cctcactcca cccccatcct 3360

agctttggta tgggggagag ggcacagggc cagacaaacc tgtgagactt tggctccatc 3420

tctgcaaaag ggcgctctgt gagtcagcct gctcccctcc aggcttgctc ctcccccacc 3480

cagctctcgt ttccaatgca cgtacagccc gtacacaccg tgtgctggga caccccacag 3540

tcagccgcat ggctcccctg tgccccagcc cctggctccc tctgttgatc ccggcccctg 3600

ctccaggcct cactgtgcaa ctgctgctgt cactgctgct tctggtgcct gtccatcccc 3660

agaggttgcc ccggatgcag gaggattccc ccttgggagg aggctcttct ggggaagatg 3720

acccactggg cgaggaggat ctgcccagtg aagaggattc acccagagag gaggatccac 3780

ccggagagga ggatctacct ggagaggagg atctacctgg agaggaggat ctacctgaag 3840

ttaagcctaa atcagaagaa gagggctccc tgaagttaga ggatctacct actgttgagg 3900

ctcctggaga tcctcaagaa ccccagaata atgcccacag ggacaaagaa ggtaagtggt 3960

catcaatctc caaatccagg ttccaggagg ttcatgactc ccctcccata ccccagccta 4020

ggctctgttc actcagggaa ggaggggaga ctgtactccc cacagaagcc cttccagagg 4080

tcccatacca atatccccat ccccactctc ggaggtagaa agggacagat gtggagagaa 4140

aataaaaagg gtgcaaaagg agagaggtga gctggatgag atgggagaga agggggaggc 4200

tggagaagag aaagggatga gaactgcaga tgagagaaaa aatgtgcaga cagaggaaaa 4260

aaataggtgg agaaggagag tcagagagtt tgaggggaag agaaaaggaa agcttgggag 4320

gtgaagtggg taccagagac aagcaagaag agctggtaga agtcatctca tcttaggcta 4380

caatgaggaa ttgagaccta ggaagaaggg acacagcagg tagagaaacg tggcttcttg 4440

actcccaagc caggaatttg gggaaagggg ttggagacca tacaaggcag agggatgagt 4500

ggggagaaga aagaagggag aaaggaaaga tggtgtactc actcatttgg gactcaggac 4560

tgaagtgccc actcactttt tttttttttt tttttgagac aaactttcac ttttgttgcc 4620

caggctggag tgcaatggcg cgatctcggc tcactgcaac ctccacctcc cgggttcaag 4680

tgattctcct gcctcagcct ctagccaagt agctgcgatt acaggcatgc gccaccacgc 4740

ccggctaatt tttgtatttt tagtagagac ggggtttcgc catgttggtc aggctggtct 4800

cgaactcctg atctcaggtg atccaaccac cctggcctcc caaagtgctg ggattatagg 4860

cgtgagccac agcgcctggc ctgaagcagc cactcacttt tacagaccct aagacaatga 4920

ttgcaagctg gtaggattgc tgtttggccc acccagctgc ggtgttgagt ttgggtgcgg 4980

tctcctgtgc tttgcacctg gcccgcttaa ggcatttgtt acccgtaatg ctcctgtaag 5040

gcatctgcgt ttgtgacatc gttttggtcg ccaggaaggg attggggctc taagcttgag 5100

cggttcatcc ttttcattta tacaggggat gaccagagtc attggcgcta tggaggtgag 5160

acacccaccc gctgcacaga cccaatctgg gaacccagct ctgtggatct cccctacagc 5220

cgtccctgaa cactggtccc gggcgtccca cccgccgccc accgtcccac cccctcacct 5280

tttctacccg ggttccctaa gttcctgacc taggcgtcag acttcctcac tatactctcc 5340

caccccaggc gacccgccct ggccccgggt gtccccagcc tgcgcgggcc gcttccagtc 5400

cccggtggat atccgccccc agctcgccgc cttctgcccg gccctgcgcc ccctggaact 5460

cctgggcttc cagctcccgc cgctcccaga actgcgcctg cgcaacaatg gccacagtgg 5520

tgagggggtc tccccgccga gacttgggga tggggcgggg cgcagggaag ggaaccgtcg 5580

cgcagtgcct gcccgggggt tgggctggcc ctaccgggcg gggccggctc acttgcctct 5640

ccctacgcag tgcaactgac cctgcctcct gggctagaga tggctctggg tcccgggcgg 5700

gagtaccggg ctctgcagct gcatctgcac tggggggctg caggtcgtcc gggctcggag 5760

cacactgtgg aaggccaccg tttccctgcc gaggtgagcg cggactggcc gagaaggggc 5820

aaaggagcgg ggcggacggg ggccagagac gtggccctct cctaccctcg tgtccttttc 5880

agatccacgt ggttcacctc agcaccgcct ttgccagagt tgacgaggcc ttggggcgcc 5940

cgggaggcct ggccgtgttg gccgcctttc tggaggtacc agatcctgga caccccctac 6000

tccccgcttt cccatcccat gctcctcccg gactctatcg tggagccaga gaccccatcc 6060

cagcaagctc actcaggccc ctggctgaca aactcattca cgcactgttt gttcatttaa 6120

cacccactgt gaaccaggca ccagccccca acaaggattc tgaagctgta ggtccttgcc 6180

tctaaggagc ccacagccag tgggggaggc tgacatgaca gacacatagg aaggacatag 6240

taaagatggt ggtcacagag gaggtgacac ttaaagcctt cactggtaga aaagaaaagg 6300

aggtgttcat tgcagaggaa acagaatgtg caaagactca gaatatggcc tatttaggga 6360

atggctacat acaccatgat tagaggaggc ccagtaaagg gaagggatgg tgagatgcct 6420

gctaggttca ctcactcact tttatttatt tatttatttt tttgacagtc tctctgtcgc 6480

ccaggctgga gtgcagtggt gtgatcttgg gtcactgcaa cttccgcctc ccgggttcaa 6540

gggattctcc tgcctcagct tcctgagtag ctggggttac aggtgtgtgc caccatgccc 6600

agctaatttt tttttgtatt tttagtagac agggtttcac catgttggtc aggctggtct 6660

caaactcctg gcctcaagtg atccgcctga ctcagcctac caaagtgctg attacaagtg 6720

tgagccaccg tgcccagcca cactcactga ttctttaatg ccagccacac agcacaaagt 6780

tcagagaaat gcctccatca tagcatgtca atatgttcat actcttaggt tcatgatgtt 6840

cttaacatta ggttcataag caaaataaga aaaaagaata ataaataaaa gaagtggcat 6900

gtcaggacct cacctgaaaa gccaaacaca gaatcatgaa ggtgaatgca gaggtgacac 6960

caacacaaag gtgtatatat ggtttcctgt ggggagtatg tacggaggca gcagtgagtg 7020

agactgcaaa cgtcagaagg gcacgggtca ctgagagcct agtatcctag taaagtgggc 7080

tctctccctc tctctccagc ttgtcattga aaaccagtcc accaagcttg ttggttcgca 7140

cagcaagagt acatagagtt tgaaataata cataggattt taagagggag acactgtctc 7200

taaaaaaaaa aacaacagca acaacaaaaa gcaacaacca ttacaatttt atgttccctc 7260

agcattctca gagctgagga atgggagagg actatgggaa cccccttcat gttccggcct 7320

tcagccatgg ccctggatac atgcactcat ctgtcttaca atgtcattcc cccaggaggg 7380

cccggaagaa aacagtgcct atgagcagtt gctgtctcgc ttggaagaaa tcgctgagga 7440

aggtcagttt gttggtctgg ccactaatct ctgtggccta gttcataaag aatcaccctt 7500

tggagcttca ggtctgaggc tggagatggg ctccctccag tgcaggaggg attgaagcat 7560

gagccagcgc tcatcttgat aataaccatg aagctgacag acacagttac ccgcaaacgg 7620

ctgcctacag attgaaaacc aagcaaaaac cgccgggcac ggtggctcac gcctgtaatc 7680

ccagcacttt gggaggccaa ggcaggtgga tcacgaggtc aagagatcaa gaccatcctg 7740

gccaacatgg tgaaacccca tctctactaa aaatacgaaa aaatagccag gcgtggtggc 7800

gggtgcctgt aatcccagct actcgggagg ctgaggcagg agaatggcat gaacccggga 7860

ggcagaagtt gcagtgagcc gagatcgtgc cactgcactc cagcctgggc aacagagcga 7920

gactcttgtc tcaaaaaaaa aaaaaaaaaa gaaaaccaag caaaaaccaa aatgagacaa 7980

aaaaaacaag accaaaaaat ggtgtttgga aattgtcaag gtcaagtctg gagagctaaa 8040

ctttttctga gaactgttta tctttaataa gcatcaaata ttttaacttt gtaaatactt 8100

ttgttggaaa tcgttctctt cttagtcact cttgggtcat tttaaatctc acttactcta 8160

ctagaccttt taggtttctg ctagactagg tagaactctg cctttgcatt tcttgtgtct 8220

gttttgtata gttatcaata ttcatattta tttacaagtt attcagatca ttttttcttt 8280

tctttttttt tttttttttt ttttttacat ctttagtaga gacagggttt caccatattg 8340

gccaggctgc tctcaaactc ctgaccttgt gatccaccag cctcggcctc ccaaagtgct 8400

gggattcatt ttttcttttt aatttgctct gggcttaaac ttgtggccca gcactttatg 8460

atggtacaca gagttaagag tgtagactca gacggtcttt cttctttcct tctcttcctt 8520

cctcccttcc ctcccacctt cccttctctc cttcctttct ttcttcctct cttgcttcct 8580

caggcctctt ccagttgctc caaagccctg tacttttttt tgagttaacg tcttatggga 8640

agggcctgca cttagtgaag aagtggtctc agagttgagt taccttggct tctgggaggt 8700

gaaactgtat ccctataccc tgaagcttta agggggtgca atgtagatga gaccccaaca 8760

tagatcctct tcacaggctc agagactcag gtcccaggac tggacatatc tgcactcctg 8820

ccctctgact tcagccgcta cttccaatat gaggggtctc tgactacacc gccctgtgcc 8880

cagggtgtca tctggactgt gtttaaccag acagtgatgc tgagtgctaa gcaggtgggc 8940

ctggggtgtg tgtggacaca gtgggtgcgg gggaaagagg atgtaagatg agatgagaaa 9000

caggagaaga aagaaatcaa ggctgggctc tgtggcttac gcctataatc ccaccacgtt 9060

gggaggctga ggtgggagaa tggtttgagc ccaggagttc aagacaaggc ggggcaacat 9120

agtgtgaccc catctctacc aaaaaaaccc caacaaaacc aaaaatagcc gggcatggtg 9180

gtatgcggcc tagtcccagc tactcaagga ggctgaggtg ggaagatcgc ttgattccag 9240

gagtttgaga ctgcagtgag ctatgatccc accactgcct accatcttta ggatacattt 9300

atttatttat aaaagaaatc aagaggctgg atggggaata caggagctgg agggtggagc 9360

cctgaggtgc tggttgtgag ctggcctggg acccttgttt cctgtcatgc catgaaccca 9420

cccacactgt ccactgacct ccctagctcc acaccctctc tgacaccctg tggggacctg 9480

gtgactctcg gctacagctg aacttccgag cgacgcagcc tttgaatggg cgagtgattg 9540

aggcctcctt ccctgctgga gtggacagca gtcctcgggc tgctgagcca ggtacagctt 9600

tgtctggttt ccccccagcc agtagtccct tatcctccca tgtgtgtgcc agtgtctgtc 9660

attggtggtc acagcccgcc tctcacatct cctttttctc tccagtccag ctgaattcct 9720

gcctggctgc tggtgagtct gcccctcctc ttggtcctga tgccaggaga ctcctcagca 9780

ccattcagcc ccagggctgc tcaggaccgc ctctgctccc tctccttttc tgcagaacag 9840

accccaaccc caatattaga gaggcagatc atggtgggga ttcccccatt gtccccagag 9900

gctaattgat tagaatgaag cttgagaaat ctcccagcat ccctctcgca aaagaatccc 9960

cccccctttt tttaaagata gggtctcact ctgtttgccc caggctgggg tgttgtggca 10020

cgatcatagc tcactgcagc ctcgaactcc taggctcagg caatcctttc accttagctt 10080

ctcaaagcac tgggactgta ggcatgagcc actgtgcctg gccccaaacg gcccttttac 10140

ttggctttta ggaagcaaaa acggtgctta tcttacccct tctcgtgtat ccaccctcat 10200

cccttggctg gcctcttctg gagactgagg cactatgggg ctgcctgaga actcggggca 10260

ggggtggtgg agtgcactga ggcaggtgtt gaggaactct gcagacccct cttccttccc 10320

aaagcagccc tctctgctct ccatcgcagg tgacatccta gccctggttt ttggcctcct 10380

ttttgctgtc accagcgtcg cgttccttgt gcagatgaga aggcagcaca ggtattacac 10440

tgaccctttc ttcaggcaca agcttccccc accettgtgg agtcacttca tgcaaagcgc 10500

atgcaaatga gctgctcctg ggccagtttt ctgattagcc tttcctgttg tgtacacaca 10560

gaaggggaac caaagggggt gtgagctacc gcccagcaga ggtagccgag actggagcct 10620

agaggctgga tcttggagaa tgtgagaagc cagccagagg catctgaggg ggagccggta 10680

actgtcctgt cctgctcatt atgccacttc cttttaactg ccaagaaatt ttttaaaata 10740

aatatttata ataaaatatg tgttagtcac ctttgttccc caaatcagaa ggaggtattt 10800

gaatttccta ttactgttat tagcaccaat ttagtggtaa tgcatttatt ctattacagt 10860

tcggcctcct tccacacatc actccaatgt gttgctcc 10898

<210> 6
<211> 37
<212> PRT
<213> HUMAN

<400> 6
Met Ala Pro Leu Cys Pro Ser Pro Trp Leu Pro Leu Leu Ile Pro Ala
1               5                   10                  15

Pro Ala Pro Gly Leu Thr Val Gln Leu Leu Leu Ser Leu Leu Leu Leu
            20                  25                  30

Met Pro Val His Pro
        35

<210> 7
<211> 25
<212> DNA
<213> HUMAN

<400> 7
tggggttctt gaggatctcc aggag 25

<210> 8
<211> 26
<212> DNA
<213> HUMAN

<400> 8
ctctaacttc agggagccct cttctt                                          26


<210> 9
<211> 48
<212> DNA
<213> HUMAN

<220>
<221> primer_bind
<222> (1)..(48)

<400> 9
cuacuacuac uaggccacgc gtcgactagt acgggnnggg nngggnng                  48


<210> 10
<211> 6
<212> PRT
<213> HUMAN

<400> 10
Glu Glu Asp Leu Pro Ser
 1               5


<210> 11
<211> 6
<212> PRT
<213> HUMAN

<400> 11
Gly Glu Asp Asp Pro Leu
 1               5


<210> 12
<211> 21
<212> PRT
<213> HUMAN

<400> 12

Asn Asn Ala His Arg Asp Lys Glu Gly Asp Asp Gln Ser His Trp Arg
1               5                   10                  15

Tyr Gly Gly Asp Pro
            20

<210> 13
<211> 16
<212> PRT
<213> HUMAN

<400> 13
His Pro Gln Arg Leu Pro Arg Met Gln Glu Asp Ser Pro Leu Gly Gly
1               5                   10                  15

<210> 14
<211> 24
<212> PRT
<213> HUMAN

<400> 14
Glu Glu Asp Ser Pro Arg Glu Glu Asp Pro Pro Gly Glu Glu Asp Leu
1               5                   10                  15

Pro Gly Glu Glu Asp Leu Pro Gly
            20

<210> 15
<211> 13
<212> PRT
<213> HUMAN

<400> 15
Leu Glu Glu Gly Pro Glu Glu Asn Ser Ala Tyr Glu Gln
1               5                   10

<210> 16
<211> 16
<212> PRT
<213> HUMAN

<400> 16
Met Arg Arg Gln His Arg Arg Gly Thr Lys Gly Gly Val Ser Tyr Arg
1               5                   10                  15

<210> 17
<211> 45
<212> DNA
<213> HUMAN

<400> 17
gtcgctagct ccatgggtca tatgcagagg ttgccccgga tgcag 45

<210> 18
<211> 43
<212> DNA
<213> HUMAN

<400> 18
gaagatctct tactcgagca ttctccaaga tccagcctct agg 43

<210> 19
<211> 10
<212> DNA
<213> HUMAN

<400> 19
ctccatctct 10

<210> 20
<211> 10
<212> DNA
<213> HUMAN

<400> 20
ccacccccat 10

<210> 21
<211> 205
<212> DNA
<213> HUMAN

<400> 21
acctgcccct cactccaccc ccatcctagc tttggtatgg gggagagggc acagggccag 60

acaaacctgt gagactttgg ctccatctct gcaaaagggc gctctgtgag tcagcctgct 120

cccctccagg cttgctcctc ccccacccag ctctcgtttc caatgcacgt acagcccgta 180

cacaccgtgt gctgggacac cccac 205

<210> 22
<211> 8
<212> PRT
<213> HUMAN

<400> 22
Leu Glu His His His His His His
1               5

<210> 23
<211> 10
<212> DNA
<213> HUMAN

<220>
<221> misc_feature
<222> (1)..(10)

<400> 23
yyycayyyyy 10

<210> 24
<211> 10
<212> DNA
<213> HUMAN

<300>
<301> Locker and Buzard,
<303> DNA Sequencing and Mapping
<304> 1
<306> 3-11
<307> 1990

<400> 24
tgtgagactt 10

<210> 25
<211> 4
<212> PRT
<213> HUMAN

<220>
<221> SITE
<222> (1)..(4)

<400> 25
Ser Pro Xaa Xaa
1

<210> 26
<211> 4
<212> PRT
<213> HUMAN

<220>
<221> SITE
<222> (1)..(4)

<400> 26
Thr Pro Xaa Xaa
1

<210> 27
<211> 540
<212> DNA
<213> HUMAN

<220>
<221> promoter
<222> (1)..(540)

<400> 27
cttgcttttc attcaagctc aagtttgtct cccacatacc cattacttaa ctcaccctcg 60

ggctccccta gcagcctgcc ctacctcttt acctgcttcc tggtggagtc agggatgtat 120

acatgagctg ctttccctct cagccagagg acatgggggg ccccagctcc cctgcctttc 180

cccttctgtg cctggagctg ggaagcaggc cagggttagc tgaggctggc tggcaagcag 240

ctgggtggtg ccagggagag cctgcatagt gccaggtggt gccttgggtt ccaagctagt 300

ccatggcccc gataaccttc tgcctgtgca cacacctgcc cctcactcca cccccatcct 360

agctttggta tgggggagag ggcacagggc cagacaaacc tgtgagactt tggctccatc 420

tctgcaaaag ggcgctctgt gagtcagcct gctcccctcc aggcttgctc ctcccccacc 480

cagctctcgt ttccaatgca cgtacagccc gtacacaccg tgtgctggga caccccacag 540

<210> 28
<211> 445
<212> DNA
<213> HUMAN

<220>
<221> exon
<222> (1)
<223> 1st MN exon

<400> 28
gcccgtacac accgtgtgct gggacacccc acagtcagcc gcatggctcc 50

cctgtgcccc agcccctggc tccctctgtt gatcccggcc cctgctccag 100

gcctcactgt gcaactgctg ctgtcactgc tgcttctggt gcctgtccat 150

ccccagaggt tgccccggat gcaggaggat tcccccttgg gaggaggctc 200

ttctggggaa gatgacccac tgggcgagga ggatctg[?]cc agtgaagagg 250

attcacccag agaggaggat ccacccggag aggaggatct acctggagag 300

gaggatctac ctggagagga ggatctacct gaagttaagc ctaaatcaga 350

agaagagggc tccctgaagt tagaggatct acctactgtt gaggctcctg 400

gagatcctca agaaccccag aataatgccc acagggacaa agaag 445

<210> 29
<211> 30
<212> DNA
<213> HUMAN

<220>
<221> exon
<222> (1)
<223> 2nd MN exon

<400> 29
gggatgacca gagtcattgg cgctatggag 30

<210> 30
<211> 171
<212> DNA
<213> HUMAN

<220>
<221> exon
<222> (1)
<223> 3rd MN exon

<400> 30
gcgacccgcc ctggccccgg gtgtccccag cctgcgcggg ccgcttccag 50

tccccggtgg atatccgccc ccagctcgcc gccttctgcc cggccctgcg 100

ccccctggaa ctcctgggct tccagctccc gccgctccca gaactgcgcc 150

tgcgcaacaa tggccacagt g 171


<210> 31
<211> 143
<212> DNA
<213> HUMAN

<220>
<221> exon
<222> (1)
<223> 4th MN exon

<400> 31
tgcaactgac cctgcctcct gggctagaga tggctctggg tcccgggcgg 50

gagtaccggg ctctgcagct gcatctgcac tggggggctg caggtcgtcc 100

gggctcggag cacactgtgg aaggccaccg tttccctgcc gag 143


<210> 32
<211> 93
<212> DNA
<213> HUMAN

<220>
<221> exon
<222> (1)
<223> 5th MN exon

<400> 32
atccacgtgg ttcacctcag caccgccttt gccagagttg acgaggcctt 50

ggggcgcccg ggaggcctgg ccgtgttggc cgcctttctg gag 93

<210> 33
<211> 67
<212> DNA
<213> HUMAN

<220>
<221> exon
<222> (1)
<223> 6th MN exon

<400> 33
gagggcccgg aagaaaacag tgcctatgag cagttgc[?]gt ctcgcttgga 50

agaaatcgct gaggaag 67

<210> 34
<211> 158
<212> DNA
<213> HUMAN

<220>
<221> exon
<222> (1)
<223> 7th MN exon

<400> 34
gctcagagac tcaggtccca ggactggaca tatctgcact cctgccctct 50

gacttcagcc gctacttcca atatgagggg tctctgacta caccgccctg 100

tgcccagggt gtcatctgga ctgtgtttaa ccagacagtg atgctgagtg ctaagcag 158

<210> 35
<211> 145
<212> DNA
<213> HUMAN

<220>
<221> exon

<222> (1)
<223> 8th MN exon

<400> 35
ctccacaccc tctctgacac cctgtgggga cctggtgact ctcggctaca 50

gctgaacttc cgagcgacgc agcctttgaa tgggcgagtg attgaggcct 100

ccttccctgc tggagtggac agcagtcctc gggctgctga gccag 145


<210> 36
<211> 27
<212> DNA
<213> HUMAN

<220>
<221> exon
<222> (1)
<223> 9th MN exon

<400> 36
tccagctgaa ttcctgcctg gctgctg 27


<210> 37
<211> 82
<212> DNA
<213> HUMAN

<220>
<221> exon
<222> (1)
<223> 10th MN exon

<400> 37
gtgacatcct agccctggtt tttggcctcc tttttgctgt caccagcgtc 50

gcgttccttg tgcagatgag aaggcagcac ag 82


<210> 38
<211> 191
<212> DNA
<213> HUMAN

<220>
<221> exon

<222> (1)
<223> 11th MN exon

<400> 38
aaggggaacc aaagggggtg tgagctaccg cccagcagag gtagccgaga 50

ctggagccta gaggctggat cttggagaat gtgagaagcc agccagaggc 100

atctgagggg gagccggtaa ctgtcctgtc ctgctcatta tgccacttcc 150

ttttaactgc caagaaattt tttaaaataa atatttataa t 191

<210> 39
<211> 1174
<212> DNA
<213> HUMAN

<220>
<221> intron
<222> (1)..(1174)
<223> 1st MN intron

<400> 39
gtaagtggtc atcaatctcc aaatccaggt tccaggaggt tcatgactcc cctcccatac 60

cccagcctag gctctgttca ctcagggaag gaggggagac tgtactcccc acagaagccc 120

ttccagaggt cccataccaa tatccccatc cccactctcg gaggtagaaa gggacagatg 180

tggagagaaa ataaaaaggg tgcaaaagga gagaggtgag ctggatgaga tgggagagaa 240

gggggaggct ggagaagaga aagggatgag aactgcagat gagagaaaaa atgtgcagac 300

agaggaaaaa aataggtgga gaaggagagt cagagagttt gaggggaaga gaaaaggaaa 360

gcttgggagg tgaagtgggt accagagaca agcaagaaga gctggtagaa gtcatctcat 420

cttaggctac aatgaggaat tgagacctag gaagaaggga cacagcaggt agagaaacgt 480

ggcttcttga ctcccaagcc aggaatttgg ggaaaggggt tggagaccat acaaggcaga 540

gggatgagtg gggagaagaa agaagggaga aaggaaagat ggtgtactca ctcatttggg 600

actcaggact gaagtgccca ctcacttttt tttttttttt ttttgagaca aactttcact 660

tttgttgccc aggctggagt gcaatggcgc gatctcggct cactgcaacc tccacctccc 720

gggttcaagt gattctcctg cctcagcctc tagccaagta gctgcgatta caggcatgcg 780

ccaccacgcc cggctaattt ttgtattttt agtagagacg gggtttcgcc atgttggtca 840

ggctggtctc gaactcctga tctcaggtga tccaaccacc ctggcctccc aaagtgctgg 900

gattataggc gtgagccaca gcgcctggcc tgaagcagcc actcactttt acagacccta 960

agacaatgat tgcaagctgg taggattgct gtttggccca cccagctgcg gtgttgagtt 1020

tgggtgcggt ctcctgtgct ttgcacctgg cccgcttaag gcatttgtta cccgtaatgc 1080

tcctgtaagg catctgcgtt tgtgacatcg ttttggtcgc caggaaggga ttggggctct 1140

aagcttgagc ggttcatcct tttcatttat acag 1174

<210> 40
<211> 193
<212> DNA
<213> HUMAN

<220>
<221> intron
<222> (1)..(193)
<223> 2nd MN intron

<400> 40
gtgagacacc cacccgctgc acagacccaa tctgggaacc cagctctgtg gatctcccct 60

acagccgtcc ctgaacactg gtcccgggcg tcccacccgc cgcccaccgt cccaccccct 120

caccttttct acccgggttc cctaagttcc tgacctaggc gtcagacttc ctcactatac 180

tctcccaccc cag 193

<210> 41
<211> 131
<212> DNA
<213> HUMAN

<220>
<221> intron
<222> (1)..(131)
<223> 3rd MN intron

<400> 41

gtgagggggt ctccccgccg agacttgggg atggggcggg gcgcagggaa gggaaccgtc 60

gcgcagtgcc tgcccggggg ttgggctggc cctaccgggc ggggccggct cacttgcctc 120

tccctacgca g 131

<210> 42
<211> 89
<212> DNA
<213> HUMAN

<220>
<221> intron
<222> (1)..(89)
<223> 4th MN intron

<400> 42
gtgagcgcgg actggccgag aaggggcaaa ggagcggggc ggacgggggc cagagacgtg 60

gccctctcct accctcgtgt ccttttcag 89

<210> 43
<211> 1400
<212> DNA
<213> HUMAN

<220>
<221> intron
<222> (1)..(1400)
<223> 5th MN intron

<400> 43
gtaccagatc ctggacaccc cctactcccc gctttcccat cccatgctcc tcccggactc 60

tatcgtggag ccagagaccc catcccagca agctcactca ggcccctggc tgacaaactc 120

attcacgcac tgtttgttca tttaacaccc actgtga[?]cc aggcaccagc ccccaacaag 180

gattctgaag ctgtaggtcc ttgcctctaa ggagcccaca gccagtgggg gaggctgaca 240

tgacagacac ataggaagga catagtaaag atggtggtca cagaggaggt gacacttaaa 300

gccttcactg gtagaaaaga aaaggaggtg ttcattgcag aggaaacaga atgtgcaaag 360

actcagaata tggcctattt agggaatggc tacatacacc atgattagag gaggcccagt 420

aaagggaagg gatggtgaga tgcctgctag gttcactcac tcacttttat ttatttattt 480

atttttttga cagtctctct gtcgcccagg ctggagtgca gtggtgtgat cttgggtcac 540

tgcaacttcc gcctcccggg ttcaagggat tctcctgcct cagcttcctg agtagctggg 600

gttacaggtg tgtgccacca tgcccagcta attttttttt gtatttttag tagacagggt 660

ttcaccatgt tggtcaggct ggtctcaaac tcctggcctc aagtgatccg cctgactcag 720

cctaccaaag tgctgattac aagtgtgagc caccgtgccc agccacactc actgattctt 780

taatgccagc cacacagcac aaagttcaga gaaatgcctc catcatagca tgtcaatatg 840

ttcatactct taggttcatg atgttcttaa cattaggttc ataagcaaaa taagaaaaaa 900

gaataataaa taaaagaagt ggcatgtcag gacctcacct gaaaagccaa acacagaatc 960

atgaaggtga atgcagaggt gacaccaaca caaaggtgta tatatggttt cctgtgggga 1020

gtatgtacgg aggcagcagt gagtgagact gcaaacgtca gaagggcacg ggtcactgag 1080

agcctagtat cctagtaaag tgggctctct ccctctctct ccagcttgtc attgaaaacc 1140

agtccaccaa gcttgttggt tcgcacagca agagtacata gagtttgaaa taatacatag 1200

gattttaaga gggagacact gtctctaaaa aaaaaaacaa cagcaacaac aaaaagcaac 1260

aaccattaca attttatgtt ccctcagcat tctcagagct gaggaatggg agaggactat 1320

gggaaccccc ttcatgttcc ggccttcagc catggccctg gatacatgca ctcatctgtc 1380

ttacaatgtc attcccccag 1400

<210> 44
<211> 1334
<212> DNA
<213> HUMAN

<220>
<221> intron
<222> (1)..(1334)
<223> 6th MN intron

<400> 44
   gtcagtttgt tggtctggcc actaatctct gtggcctagt tcataaagaa tcaccctttg 60
   gagcttcagg tctgaggctg gagatgggct ccctccagtg caggagggat tgaagcatga 120
   gccagcgctc atcttgataa taaccatgaa gctgacagac acagttaccc gcaaacggct 180
   gcctacagat tgaaaaccaa gcaaaaaccg ccgggcacgg tggctcacgc ctgtaatccc 240
   agcactttgg gaggccaagg caggtggatc acgaggtcaa gagatcaaga ccatcctggc 300
   caacatggtg aaaccccatc tctactaaaa atacgaaaaa atagccaggc gtggtggcgg 360
   gtgcctgtaa tcccagctac tcgggaggct gaggcaggag aatggcatga acccgggagg 420
   cagaagttgc agtgagccga gatcgtgcca ctgcactcca gcctgggcaa cagagcgaga 480
   ctcttgtctc aaaaaaaaaa aaaaaaaaga aaaccaagca aaaaccaaaa tgagacaaaa 540
   aaaacaagac caaaaaatgg tgtttggaaa ttgtcaaggt caagtctgga gagctaaact 600
   ttttctgaga actgtttatc tttaataagc atcaaatatt ttaactttgt aaatactttt 660
   gttggaaatc gttctcttct tagtcactct tgggtcattt taaatctcac ttactctact 720
   agacctttta ggtttctgct agactaggta gaactctgcc tttgcatttc ttgtgtctgt 780
   tttgtatagt tatcaatatt catatttatt tacaagttat tcagatcatt ttttcttttc 840
   tttttttttt tttttttttt ttttacatct ttagtagaga cagggtttca ccatattggc 900
   caggctgctc tcaaactcct gaccttgtga tccaccagcc tcggcctccc aaagtgctgg 960
   gattcatttt ttctttttaa tttgctctgg gcttaaactt gtggcccagc actttatgat 1020
   ggtacacaga gttaagagtg tagactcaga cggtctttct tctttccttc tcttccttcc 1080
   tcccttccct cccaccttcc cttctctcct tcctttcttt cttcctctct tgcttcctca 1140
   ggcctcttcc agttgctcca aagccctgta cttttttttg agttaacgtc ttatgggaag 1200
   ggcctgcact tagtgaagaa gtggtctcag agttgagtta ccttggcttc tgggaggtga 1260
   aactgtatcc ctataccctg aagctttaag gggtgcaatt gtagatgaga ccccaacata 1320
   gatcctcttc acag 1334

<210> 45
<211> 512
<212> DNA

<213> HUMAN

<220>
<221> intron
<222> (1)..(512)
<223> 7th MN intron

<400> 45
gtgggcctgg ggtgtgtgtg gacacagtgg gtgcggggga aagaggatgt aagatgagat 60

gagaaacagg agaagaaaga aatcaaggct gggctctgtg gcttacgcct ataatcccac 120

cacgttggga ggctgaggtg ggagaatggt ttgagcccag gagttcaaga caaggcgggg 180

caacatagtg tgaccccatc tctaccaaaa aaaccccaac aaaaccaaaa atagccgggc 240

atggtggtat gcggcctagt cccagctact caaggaggct gaggtgggaa gatcgcttga 300

ttccaggagt ttgagactgc agtgagctat gatcccacca ctgcctacca tctttaggat 360

acatttattt atttataaaa gaaatcaaga ggctggatgg ggaatacagg agctggaggg 420

tggagccctg aggtgctggt tgtgagctgg cctgggaccc ttgtttcctg tcatgccatg 480

aacccaccca cactgtccac tgacctccct ag 512


<210> 46
<211> 114
<212> DNA
<213> HUMAN

<220>
<221> intron
<222> (1)..(114)
<223> 8th MN intron

<400> 46
gtacagcttt gtctggtttc cccccagcca gtagtccctt atcctcccat gtgtgtgcca 60

gtgtctgtca ttggtggtca cagcccgcct ctcacatctc ctttttctct ccag 114


<210> 47
<211> 617
<212> DNA
<213> HUMAN

<220>
<221> intron
<222> (1)..(617)
<223> 9th MN intron

<400> 47
gtgagtctgc ccctcctctt ggtcctgatg ccaggagact cctcagcacc attcagcccc 60

agggctgctc aggaccgcct ctgctccctc tccttttctg cagaacagac cccaacccca 120

atattagaga ggcagatcat ggtggggatt cccccattgt ccccagaggc taattgatta 180

gaatgaagct tgagaaatct cccagcatcc ctctcgcaaa agaatccccc cccctttttt 240

taaagatagg gtctcactct gtttgcccca ggctggggtg ttgtggcacg atcatagctc 300

actgcagcct cgaactccta ggctcaggca atcctttcac cttagcttct caaagcactg 360

ggactgtagg catgagccac tgtgcctggc cccaaacggc ccttttactt ggcttttagg 420

aagcaaaaac ggtgcttatc ttaccccttc tcgtgtatcc accctcatcc cttggctggc 480

ctcttctgga gactgaggca ctatggggct gcctgagaac tcggggcagg ggtggtggag 540

tgcactgagg caggtgttga ggaactctgc agacccctct tccttcccaa agcagccctc 600

tctgctctcc atcgcag 617


<210> 48
<211> 130
<212> DNA
<213> HUMAN

<220>
<221> intron
<222> (1)..(130)
<223> 10th MN intron

<400> 48
gtattacact gaccctttct tcaggcacaa gcttccccca cccttgtgga gtcacttcat 60

gcaaagcgca tgcaaatgag ctgctcctgg gccagttttc tgattagcct ttcctgttgt 120

gtacacacag 130


<210> 49

<211> 1401
<212> DNA
<213> HUMAN

<400> 49
   caaactttca cttttgttgc ccaggctgga gtgcaatggc gcgatctcgg ctcactgcaa 60
   cctccacctc ccgggttcaa gtgattctcc tgcctcagcc tctagccaag tagctgcgat 120
   tacaggcatg cgccaccacg cccggctaat ttttgtattt ttagtagaga cggggtttcg 180
   ccatgttggt caggctggtc tcgaactcct gatctcaggt gatccaacca ccctggcctc 240
   ccaaagtgct gggattatag gcgtgagcca cagcgcctgg cctgaagcag ccactcactt 300
   ttacagaccc taagacaatg attgcaagct ggtaggattg ctgtttggcc cacccagctg 360
   cggtgttgag tttgggtgcg gtctcctgtg ctttgcacct ggcccgctta aggcatttgt 420
   tacccgtaat gctcctgtaa ggcatctgcg tttgtgacat cgttttggtc gccaggaagg 480
   gattggggct ctaagcttga gcggttcatc cttttcattt atacagggga tgaccagagt 540
   cattggcgct atggaggtga gacacccacc cgctgcacag acccaatctg ggaacccagc 600
   tctgtggatc tcccctacag ccgtccctga acactggtcc cgggcgtccc acccgccgcc 660
   caccgtccca ccccctcacc ttttctaccc gggttcccta agttcctgac ctaggcgtca 720
   gacttcctca ctatactctc ccaccccagg cgacccgccc tggccccggg tgtccccagc 780
   ctgcgcgggc cgcttccagt ccccggtgga tatccgcccc cagctcgccg ccttctgccc 840
   ggccctgcgc cccctggaac tcctgggctt ccagctcccg ccgctcccag aactgcgcct 900
   gcgcaacaat ggccacagtg gtgagggggt ctccccgccg agacttgggg atggggcggg 960
   gcgcagggaa gggaaccgtc gcgcagtgcc tgcccggggg ttgggctggc cctaccgggc 1020
   ggggccggct cacttgcctc tccctacgca gtgcaactga ccctgcctcc tgggctagag 1080
   atggctctgg gtcccgggcg ggagtaccgg gctctgcagc tgcatctgca ctggggggct 1140
   gcaggtcgtc cgggctcgga gcacactgtg gaaggccacc gtttccctgc cgaggtgagc 1200
   gcggactggc cgagaagggg caaaggagcg gggcggacgg gggccagaga cgtggccctc 1260
   tcctaccctc gtgtcctttt cagatccacg tggttcacct cagcaccgcc tttgccagag 1320
   ttgacgaggc cttggggcgc ccgggaggcc tggccgtgtt ggccgccttt ctggaggtac 1380
   cagatcctgg acacccccta c 1401

<210> 50
<211> 59
<212> PRT
<213> HUMAN

<400> 50
Ser Ser Gly Glu Asp Asp Pro Leu Gly Glu Glu Asp Leu Pro Ser Glu
1               5                   10                  15

Glu Asp Ser Pro Arg Glu Glu Asp Pro Pro Gly Glu Glu Asp Leu Pro
            20                  25                  30

Gly Glu Glu Asp Leu Pro Gly Glu Glu Asp Leu Pro Glu Val Lys Pro
        35                  40                  45

Lys Ser Glu Glu Glu Gly Ser Leu Lys Leu Glu
    50                  55

<210> 51
<211> 257
<212> PRT
<213> HUMAN

<400> 51
Gly Asp Asp Gln Ser His Trp Arg Tyr Gly Gly Asp Pro Pro Trp Pro
1               5                   10                  15

Arg Val Ser Pro Ala Cys Ala Gly Arg Phe Gln Ser Pro Val Asp Ile
            20                  25                  30

Arg Pro Gln Leu Ala Ala Phe Cys Pro Ala Leu Arg Pro Leu Glu Leu
        35                  40                  45

Leu Gly Phe Gln Leu Pro Pro Leu Pro Glu Leu Arg Leu Arg Asn Asn
    50                  55                  60

Gly His Ser Val Gln Leu Thr Leu Pro Pro Gly Leu Glu Met Ala Leu
65                  70                  75                  80

Gly Pro Gly Arg Glu Tyr Arg Ala Leu Gln Leu His Leu His Trp Gly
                85                  90                  95

Ala Ala Gly Arg Pro Gly Ser Glu His Thr Val Glu Gly His Arg Phe
            100                 105                 110

Pro Ala Glu Ile His Val Val His Leu Ser Thr Ala Phe Ala Arg Val
        115                 120                 125

Asp Glu Ala Leu Gly Arg Pro Gly Gly Leu Ala Val Leu Ala Ala Phe
    130                 135                 140

Leu Glu Glu Gly Pro Glu Glu Asn Ser Ala Tyr Glu Gln Leu Leu Ser
145                 150                 155                 160

Arg Leu Glu Glu Ile Ala Glu Glu Gly Ser Glu Thr Gln Val Pro Gly
                165                 170                 175

Leu Asp Ile Ser Ala Leu Leu Pro Ser Asp Phe Ser Arg Tyr Phe Gln
            180                 185                 190

Tyr Glu Gly Ser Leu Thr Thr Pro Pro Cys Ala Gln Gly Val Ile Trp
        195                 200                 205

Thr Val Phe Asn Gln Thr Val Met Leu Ser Ala Lys Gln Leu His Thr
    210                 215                 220

Leu Ser Asp Thr Leu Trp Gly Pro Gly Asp Ser Arg Leu Gln Leu Asn
225                 230                 235                 240

Phe Arg Ala Thr Gln Pro Leu Asn Gly Arg Val Ile Glu Ala Ser Phe
                245                 250                 255

Pro

<210> 52
<211> 20
<212> PRT
<213> HUMAN

<400> 52
Ile Leu Ala Leu Val Phe Gly Leu Leu Phe Ala Val Thr Ser Val Ala
1               5                   10                  15

Phe Leu Val Gln
            20

<210> 53
<211> 25
<212> PRT
<213> HUMAN

<400> 53
Met Arg Arg Gln His Arg Arg Gly Thr Lys Gly Gly Val Ser Tyr Arg
1               5                   10                  15

Pro Ala Glu Val Ala Glu Thr Gly Ala
            20                  25

<210> 54
<211> 59
<212> PRT
<213> HUMAN

<400> 54
Ser Ala Ser Glu Glu Pro Ser Pro Ser Glu Val Pro Phe Pro Ser Glu
1               5                   10                  15

Glu Pro Ser Pro Ser Glu Glu Pro Phe Pro Ser Val Arg Pro Phe Pro
            20                  25                  30

Ser Val Val Leu Phe Pro Ser Glu Glu Pro Phe Pro Ser Lys Glu Pro
        35                  40                  45

Ser Pro Ser Glu Glu Pro Ser Ala Ser Glu Glu
    50                  55

<210> 55
<211> 470
<212> RNA
<213> HUMAN

<400> 55
cauggccccg auaaccuucu gccugugcac acaccugccc cucacuccac ccccauccua 60

gcuuugguau gggggagagg gcacagggcc agacaaaccu gugagacuuu ggcuccaucu 120

cugcaaaagg gcgcucugug agucagccug cuccccucca ggcuugcucc ucccccaccc 180

agcucucguu uccaaugcac guacagcccg uacacaccgu gugcugggac accccacagu 240

cagccgcaug gcuccccugu gccccagccc cuggcucccu cuguugaucc cggccccugc 300

uccaggccuc acugugcaac ugcugcuguc acugcugcuu cuggugccug uccaucccca 360

gagguugccc cggaugcagg aggauucccc cuugggagga ggcucuucug gggaagauga 420

cccacugggc gaggaggauc ugcccaguga agaggauuca cccagagagg 470

<210> 56
<211> 292
<212> DNA
<213> HUMAN

<400> 56
gtttttttga gacggagtct tgcatctgtc atgcccaggc tggagtagca gtggtgccat 60

ctcggctcac tgcaagctcc acctcccgag ttcacgccat tttcctgcct cagcctcccg 120

agtagctggg actacaggcg cccgccacca tgcccggcta attttttgta tttttggtag 180

agacggggtt tcaccgtgtt agccagaatg gtctcgatct cctgacttcg tgatccaccc 240

gcctcggcct cccaaagttc tgggattaca ggtgtgagcc accgcacctg gc 292

<210> 57
<211> 262
<212> DNA
<213> HUMAN

<400> 57
tttctttttt gagacagggt cttgctctgt cacccaggcc agagtgcaat ggtacagtct 60

cagctcactg cagcctcaac cgcctcggct caaaccatca tcccatttca gcctcctgag 120

tagctgggac tacaggcaca tgccattaca cctggctaat ttttttgtat ttctagtaga 180

gacagggttt ggccatgttg cccgggctgg tctcgaactc ctggactcaa gcaatccacc 240

cacctcagcc tcccaaaatg ag 262

<210> 58
<211> 2501
<212> DNA
<213> HUMAN

<220>
<221> misc_feature

<222> (1)..(2501)

<400> 58
tgttgactcg tgaccttacc cccaaccctg tgctctctga aacatgagct gtgtccactc 60

agggttaaat ggattaaggg cggtgcaaga tgtgctttgt taaacagatg cttgaaggca 120

gcatgctcgt taagagtcat caccaatccc taatctcaag taatcaggga cacaaacact 180

gcggaaggcc gcagggtcct ctgcctagga aaaccagaga cctttgttca cttgtttatc 240

tgaccttccc tccactattg tccatgaccc tgccaaatcc ccctctgtga gaaacaccca 300

agaattatca ataaaaaaat aaatttaaaa aaaaaataca aaaaaaaaaa aaaaaaaaaa 360

aaaagactta cgaatagtta ttgataaatg aatagctatt ggtaaagcca agtaaatgat 420

catattcaaa accagacggc catcatcaca gctcaagtct acctgatttg atctctttat 480

cattgtcatt ctttggattc actagattag tcatcatcct caaaattctc ccccaagttc 540

taattacgtt ccaaacattt aggggttaca tgaagcttga acctactacc ttctttgctt 600

ttgagccatg agttgtagga atgatgagtt tacaccttac atgctgggga ttaatttaaa 660

ctttacctct aagtcagttg ggtagccttt ggcttatttt tgtagctaat tttgtagtta 720

atggatgcac tgtgaatctt gctatgatag ttttcctcca cactttgcca ctaggggtag 780

gtaggtactc agttttcagt aattgcttac ctaagaccct aagccctatt tctcttgtac 840

tggcctttat ctgtaatatg ggcatattta atacaatata atttttggag tttttttgtt 900

tgtttgtttg tttgtttttt tgagacggag tcttgcatct gtcatgccca ggctggagta 960

gcagtggtgc catctcggct cactgcaagc tccacctccc gagttcacgc cattttcctg 1020

cctcagcctc ccgagtagct gggactacag gcgcccgcca ccatgcccgg ctaatttttt 1080

gtatttttgg tagagacggg gtttcaccgt gttagccaga atggtctcga tctcctgact 1140

tcgtgatcca cccgcctcgg cctcccaaag ttctgggatt acaggtgtga gccaccgcac 1200

ctggccaatt ttttgagtct tttaaagtaa aaatatgtct tgtaagctgg taactatggt 1260

acatttcctt ttattaatgt ggtgctgacg gtcatatagg ttcttttgag tttggcatgc 1320

atatgctact tttgcagtcc ctttcattac atttttctct cttcatttga agagcatgtt 1380

atatctttta gcttcacttg gcttaaaagg ttctctcatt agcctaacac agtgtcattg 1440

ttggtaccac ttggatcata agtggaaaaa cagtcaagaa attgcacagt aatacttgtt 1500

tgtaagaggg atgattcagg tgaatctgac actaagaaac tcccctacct gaggtctgag 1560

attcctctga cattgctgta tataggcttt tcctttg[?]ca gcctgtgact gcggactatt 1620

tttcttaagc aagatatgct aaagttttgt gagccttttt ccagagagag gtctcatatc 1680

tgcatcaagt gagaacatat aatgtctgca tgtttccata tttcaggaat gtttgcttgt 1740

gttttatgct tttatataga cagggaaact tgttcctcag tgacccaaaa gaggtgggaa 1800

ttgttattgg atatcatcat tggcccacgc tttctgacct tggaaacaat taagggttca 1860

taatctcaat tctgtcagaa ttggtacaag aaatagctgc tatgtttctt gacattccac 1920

ttggtaggaa ataagaatgt gaaactcttc agttggtgtg tgtccctngt ttttttgcaa 1980

tttccttctt actgtgttaa aaaaaagtat gatcttgctc tgagaggtga ggcattctta 2040

atcatgatct ttaaagatca ataatataat cctttcaagg attatgtctt tattataata 2100

aagataattt gtctttaaca gaatcaataa tataatccct taaaggatta tatctttgct 2160

gggcgcagtg gctcacacct gtaatcccag cactttgggt ggccaaggtg gaaggatcaa 2220

atttgcctac ttctatatta tcttctaaag cagaattcat ctctcttccc tcaatatgat 2280

gatattgaca gggtttgccc tcactcacta gattgtgagc tcctgctcag ggcaggtagc 2340

gttttttgtt tttgtttttg tttttctttt ttgagacagg gtcttgctct gtcacccagg 2400

ccagagtgca atggtacagt ctcagctcac tgcagcctca accgcctcgg ctcaaaccat 2460

catcccattt cagcctcctg agtagctggg actacaggca c 2501

<210> 59
<211> 292
<212> DNA
<213> HUMAN

<220>
<221> misc_feature
<222> (1)

<400> 59
tttttttgag acggagtctt gcatctgtca tgcccaggct ggagtagcag tggtgccatc 60

tcggctcact gcaagctcca cctcccgagt tcacgccatt ttcctgcctc agcctcccga 120

gtagctggga ctacaggcgc ccgccaccat gcccggctaa ttttttgtat ttttggtaga 180

gacggggttt caccgtgtta gccagaatgg tctcgat[?]tc ctgacttcgt gatccacccg 240

cctcggcctc ccaaagttct gggattacag gtgtgagcca ccgcacctgg cc 292


<210> 60
<211> 262
<212> DNA
<213> HUMAN

<400> 60
ttcttttttg agacagggtc ttgctctgtc acccaggcca gagtgcaatg gtacagtctc 60

agctcactgc agcctcaacc gcctcggctc aaaccatcat cccatttcag cctcctgagt 120

agctgggact acaggcacat gccattacac ctggcta[?]tt tttttgtatt tctagtagag 180

acagggtttg gccatgttgc ccgggctggt ctcgaactcc tggactcaag caatccaccc 240

acctcagcct cccaaaatga gg 262


<210> 61
<211> 294
<212> DNA
<213> HUMAN

<400> 61
tttttttttg agacaaactt tcacttttgt tgcccaggct ggagtgcaat ggcgcgatct 60

cggctcactg caacctccac ctcccgggtt caagtga[?]tc tcctgcctca gcctctagcc 120

aagtagctgc gattacaggc atgcgccacc acgcccggct aatttttgta tttttagtag 180

agacggggtt tcgccatgtt ggtcaggctg gtctcgaact cctgatctca ggtgatccaa 240

ccaccctggc ctcccaaagt gctgggatta taggcgtgag ccacagcgcc tggc 294


<210> 62

<211> 276
<212> DNA
<213> HUMAN

<400> 62
tgacagtctc tctgtcgccc aggctggagt gcagtggtgt gatcttgggt cactgcaact 60

tccgcctccc gggttcaagg gattctcctg cctcagcttc ctgagtagct ggggttacag 120

gtgtgtgcca ccatgcccag ctaatttttt tttgtatttt tagtagacag ggtttcacca 180

tgttggtcag gctggtctca aactcctggc ctcaagtgat ccgcctgact cagcctacca 240

aagtgctgat tacaagtgtg agccaccgtg cccagc 276


<210> 63
<211> 289
<212> DNA
<213> HUMAN

<400> 63
cgccgggcac ggtggctcac gcctgtaatc ccagcacttt gggaggccaa ggcaggtgga 60

tcacgaggtc aagagatcaa gaccatcctg gccaacatgg tgaaacccca tctctactaa 120

aaatacgaaa aaatagccag gcgtggtggc gggtgcctgt aatcccagct actcgggagg 180

ctgaggcagg agaatggcat gaacccggga ggcagaagtt gcagtgagcc gagatcgtgc 240

cactgcactc cagcctgggc aacagagcga gactcttgtc tcaaaaaaa 289


<210> 64
<211> 298
<212> DNA
<213> HUMAN

<400> 64
aggctgggct ctgtggctta cgcctataat cccaccacgt tgggaggctg aggtgggaga 60

atggtttgag cccaggagtt caagacaagg cggggcaaca tagtgtgacc ccatctctac 120

caaaaaaacc ccaacaaaac caaaaatagc cgggcatggt ggtatgcggc ctagtcccag 180

ctactcaagg aggctgaggt gggaagatcg cttgattcca ggagtttgag actgcagtga 240

gctatgatcc caccactgcc taccatcttt aggatacatt tatttattta taaaagaa 298

<210> 65
<211> 105
<212> DNA
<213> HUMAN

<400> 65
ttttttacat ctttagtaga gacagggttt caccatattg gccaggctgc tctcaaactc 60

ctgaccttgt gatccaccag cctcggcctc ccaaagtgct gggat 105

<210> 66
<211> 83
<212> DNA
<213> HUMAN

<400> 66
cctcgaactc ctaggctcag gcaatccttt caccttagct tctcaaagca ctgggactgt 60

aggcatgagc cactgtgcct ggc 83

<210> 67
<211> 11
<212> DNA
<213> HUMAN

<400> 67
agaaggtaag t 11

<210> 68
<211> 11
<212> DNA
<213> HUMAN

<400> 68
tggaggtgag a 11

<210> 69
<211> 11
<212> DNA
<213> HUMAN

<400> 69

cagtcgtgag g 11

<210> 70
<211> 11
<212> DNA
<213> HUMAN

<400> 70
ccgaggtgag c 11

<210> 71
<211> 11
<212> DNA
<213> HUMAN

<400> 71
tggaggtacc a 11

<210> 72
<211> 11
<212> DNA
<213> HUMAN

<400> 72
ggaaggtcag t 11

<210> 73
<211> 11
<212> DNA
<213> HUMAN

<400> 73
agcaggtggg c 11

<210> 74
<211> 11
<212> DNA
<213> HUMAN

<400> 74
gccaggtaca g 11

<210> 75
<211> 11
<212> DNA
<213> HUMAN

<400> 75
tgctggtgag t                                                                 11


<210> 76
<211> 11
<212> DNA
<213> HUMAN

<400> 76
atacagggga t                                                                 11


<210> 77
<211> 11
<212> DNA
<213> HUMAN

<400> 77
atacagggga t                                                                 11


<210> 78
<211> 11
<212> DNA
<213> HUMAN

<400> 78
ccccaggcga c                                                                 11


<210> 79
<211> 11
<212> DNA
<213> HUMAN

<400> 79
acgcagtgca a                                                                 11


<210> 80
<211> 11
<212> DNA

<213> HUMAN

<400> 80
tttcagatcc a                                                                 11

<210> 81
<211> 11
<212> DNA
<213> HUMAN

<400> 81
ccccaggagg g                                                                 11

<210> 82
<211> 11
<212> DNA
<213> HUMAN

<400> 82
tcacaggctc a                                                                 11

<210> 83
<211> 11
<212> DNA
<213> HUMAN

<400> 83
ccctagctcc a                                                                 11

<210> 84
<211> 11
<212> DNA
<213> HUMAN

<400> 84
ctccagtcca g                                                                 11

<210> 85
<211> 12
<212> DNA
<213> HUMAN

<400> 85

tcgcaggtga ca                                                          12

<210> 86
<211> 11
<212> DNA
<213> HUMAN

<400> 86
acacagaagg g                                                           11

<210> 87
<211> 377
<212> PRT
<213> HUMAN

<400> 87
Gln Arg Leu Pro Arg Met Gln Glu Asp Ser Pro Leu Gly Gly Gly Ser
1               5                   10                  15

Ser Gly Glu Asp Asp Pro Leu Gly Glu Glu Asp Leu Pro Ser Glu Glu
            20                  25                  30

Asp Ser Pro Arg Glu Glu Asp Pro Pro Gly Glu Glu Asp Leu Pro Gly
        35                  40                  45

Glu Glu Asp Leu Pro Gly Glu Glu Asp Leu Pro Glu Val Lys Pro Lys
    50                  55                  60

Ser Glu Glu Glu Gly Ser Leu Lys Leu Glu Asp Leu Pro Thr Val Glu
65                  70                  75                  80

Ala Pro Gly Asp Pro Gln Glu Pro Gln Asn Asn Ala His Arg Asp Lys
                85                  90                  95

Glu Gly Asp Asp Gln Ser His Trp Arg Tyr Gly Gly Asp Pro Pro Trp
            100                 105                 110

Pro Arg Val Ser Pro Ala Cys Ala Gly Arg Phe Gln Ser Pro Val Asp
        115                 120                 125

Ile Arg Pro Gln Leu Ala Ala Phe Cys Pro Ala Leu Arg Pro Leu Glu
    130                 135                 140

Leu Leu Gly Phe Gln Leu Pro Pro Leu Pro Glu Leu Arg Leu Arg Asn
145                 150                 155                 160

Asn Gly His Ser Val Gln Leu Thr Leu Pro Pro Gly Leu Glu Met Ala
                165                 170                 175

Leu Gly Pro Gly Arg Glu Tyr Arg Ala Leu Gln Leu His Leu His Trp
            180                 185                 190

Gly Ala Ala Gly Arg Pro Gly Ser Glu His Thr Val Glu Gly His Arg
        195                 200                 205

Phe Pro Ala Glu Ile His Val Val His Leu Ser Thr Ala Phe Ala Arg
    210                 215                 220

Val Asp Glu Ala Leu Gly Arg Pro Gly Gly Leu Ala Val Leu Ala Ala
225                 230                 235                 240

Phe Leu Glu Glu Gly Pro Glu Glu Asn Ser Ala Tyr Glu Gln Leu Leu
                245                 250                 255

Ser Arg Leu Glu Glu Ile Ala Glu Glu Gly Ser Glu Thr Gln Val Pro
            260                 265                 270

Gly Leu Asp Ile Ser Ala Leu Leu Pro Ser Asp Phe Ser Arg Tyr Phe
        275                 280                 285

Gln Tyr Glu Gly Ser Leu Thr Thr Pro Pro Cys Ala Gln Gly Val Ile
    290                 295                 300

Trp Thr Val Phe Asn Gln Thr Val Met Leu Ser Ala Lys Gln Leu His
305                 310                 315                 320

Thr Leu Ser Asp Thr Leu Trp Gly Pro Gly Asp Ser Arg Leu Gln Leu
                325                 330                 335

Asn Phe Arg Ala Thr Gln Pro Leu Asn Gly Arg Val Ile Glu Ala Ser
            340                 345                 350

Phe Pro Ala Gly Val Asp Ser Ser Pro Arg Ala Ala Glu Pro Val Gln
        355                 360                 365

Leu Asn Ser Cys Leu Ala Ala Gly Asp
    370                 375

<210> 88
<211> 34
<212> DNA
<213> HUMAN

<400> 88
tagacagatc tacgatggct cccctgtgcc ccag                                34

<210> 89
<211> 34
<212> DNA
<213> HUMAN

<400> 89
attcctctag acagttaccg gctccccctc agat                                34

<210> 90
<211> 3532
<212> DNA
<213> HUMAN

<220>
<221> misc_feature
<222> (1)..(3532)

<400> 90
   tgttgactcg tgaccttacc cccaaccctg tgctctctga aacatgagct gtgtccactc 60
   agggttaaat ggattaaggg cggtgcaaga tgtgctttgt taaacagatg cttgaaggca 120
   gcatgctcgt taagagtcat caccaatccc taatctcaag taatcaggga cacaaacact 180
   gcggaaggcc gcagggtcct ctgcctagga aaaccagaga cctttgttca cttgtttatc 240
   tgaccttccc tccactattg tccatgaccc tgccaaatcc ccctctgtga gaaacaccca 300
   agaattatca ataaaaaaat aaatttaaaa aaaaaataca aaaaaaaaaa aaaaaaaaaa 360
   aaaagactta cgaatagtta ttgataaatg aatagctatt ggtaaagcca agtaaatgat 420
   catattcaaa accagacggc catcatcaca gctcaagtct acctgatttg atctctttat 480
   cattgtcatt ctttggattc actagattag tcatcatcct caaaattctc ccccaagttc 540
   taattacgtt ccaaacattt aggggttaca tgaagcttga acctactacc ttctttgctt 600
   ttgagccatg agttgtagga atgatgagtt tacaccttac atgctgggga ttaatttaaa 660
   ctttacctct aagtcagttg ggtagccttt ggcttatttt tgtagctaat tttgtagtta 720
   atggatgcac tgtgaatctt gctatgatag ttttcctcca cactttgcca ctaggggtag 780
   gtaggtactc agttttcagt aattgcttac ctaagaccct aagccctatt tctcttgtac 840
   tggcctttat ctgtaatatg ggcatattta atacaatata atttttggag tttttttgtt 900
   tgtttgtttg tttgtttttt tgagacggag tcttgcatct gtcatgccca ggctggagta 960
   gcagtggtgc catctcggct cactgcaagc tccacctccc gagttcacgc cattttcctg 1020
   cctcagcctc ccgagtagct gggactacag gcgcccgcca ccatgcccgg ctaatttttt 1080
   gtatttttgg tagagacggg gtttcaccgt gttagccaga atggtctcga tctcctgact 1140
   tcgtgatcca cccgcctcgg cctcccaaag ttctgggatt acaggtgtga gccaccgcac 1200
   ctggccaatt ttttgagtct tttaaagtaa aaatatgtct tgtaagctgg taactatggt 1260
   acatttcctt ttattaatgt ggtgctgacg gtcatatagg ttcttttgag tttggcatgc 1320
   atatgctact ttttgcagtc ctttcattac atttttctct cttcatttga agagcatgtt 1380
   atatctttta gcttcacttg gcttaaaagg ttctctcatt agcctaacac agtgtcattg 1440
   ttggtaccac ttggatcata agtggaaaaa cagtcaagaa attgcacagt aatacttgtt 1500
   tgtaagaggg atgattcagg tgaatctgac actaagaaac tcccctacct gaggtctgag 1560
   attcctctga cattgctgta tataggcttt tcctttgaca gcctgtgact gcggactatt 1620
   tttcttaagc aagatatgct aaagttttgt gagccttttt ccagagagag gtctcatatc 1680
   tgcatcaagt gagaacatat aatgtctgca tgtttccata tttcaggaat gtttgcttgt 1740
   gttttatgct tttatataga cagggaaact tgttcctcag tgacccaaaa gaggtgggaa 1800
   ttgttattgg atatcatcat tggcccacgc tttctgacct tggaaacaat taagggttca 1860
   taatctcaat tctgtcagaa ttggtacaag aaatagctgc tatgtttctt gacattccac 1920
   ttggtaggaa ataagaatgt gaaactcttc agttggtgtg tgtccctngt ttttttgcaa 1980
   tttccttctt actgtgttaa aaaaaagtat gatcttgctc tgagaggtga ggcattctta 2040
   atcatgatct ttaaagatca ataatataat cctttcaagg attatgtctt tattataata 2100
   aagataattt gtctttaaca gaatcaataa tataatccct taaaggatta tatctttgct 2160
   gggcgcagtg gctcacacct gtaatcccag cactttgggt ggccaaggtg gaaggatcaa 2220
   atttgcctac ttctatatta tcttctaaag cagaattcat ctctcttccc tcaatatgat 2280
   gatattgaca gggtttgccc tcactcacta gattgtgagc tcctgctcag ggcaggtagc 2340
   gttttttgtt tttgtttttg tttttctttt ttgagacagg gtcttgctct gtcacccagg 2400
   ccagagtgca atggtacagt ctcagctcac tgcagcctca accgcctcgg ctcaaaccat 2460
   catcccattt cagcctcctg agtagctggg actacaggca catgccatta cacctggcta 2520
   atttttttgt atttctagta gagacagggt ttggccatgt tgcccgggct ggtctcgaac 2580
   tcctggactc aagcaatcca cccacctcag cctcccaaaa tgagggaccg tgtcttattc 2640
   atttccatgt ccctagtcca tagcccagtg ctggacctat ggtagtacta aataaatatt 2700
   tgttgaatgc aatagtaaat agcatttcag ggagcaagaa ctagattaac aaaggtggta 2760
   aaaggtttgg agaaaaaaat aatagtttaa tttggctaga gtatgaggga gagtagtagg 2820
   agacaagatg gaaaggtctc ttgggcaagg ttttgaagga agttggaagt cagaagtaca 2880
   caatgtgcat atcgtggcag gcagtgggga gccaatgaag gcttttgagc aggagagtaa 2940
   tgtgttgaaa aataaatata ggttaaacct atcagagccc ctctgacaca tacacttgct 3000
   tttcattcaa gctcaagttt gtctcccaca tacccattac ttaactcacc ctcgggctcc 3060
   cctagcagcc tgccctacct ctttacctgc ttcctggtgg agtcagggat gtatacatga 3120
   gctgctttcc ctctcagcca gaggacatgg ggggccccag ctcccctgcc tttccccttc 3180
   tgtgcctgga gctgggaagc aggccagggt tagctgaggc tggctggcaa gcagctgggt 3240
   ggtgccaggg agagcctgca tagtgccagg tggtgccttg ggttccaagc tagtccatgg 3300
   ccccgataac cttctgcctg tgcacacacc tgcccctcac tccaccccca tcctagcttt 3360
   ggtatggggg agagggcaca gggccagaca aacctgtgag actttggctc catctctgca 3420
   aaagggcgct ctgtgagtca gcctgctccc ctccaggctt gctcctcccc cacccagctc 3480
   tcgtttccaa tgcacgtaca gcccgtacac accgtgtgct gggacacccc ac 3532

<210> 91
<211> 204

<212> DNA
<213> HUMAN

<400> 91
cctgcccctc actccacccc catcctagct ttggtatggg ggagagggca cagggccaga 60

caaacctgtg agactttggc tccatctctg caaaagggcg ctctgtgagt cagcctgctc 120

ccctccaggc ttgctcctcc cccacccagc tctcgtttcc aatgcacgta cagcccgtac 180

acaccgtgtg ctgggacacc ccac 204


<210> 92
<211> 132
<212> DNA
<213> HUMAN

<400> 92
ggatcctgtt gactcgtgac cttaccccca accctgtgct ctctgaaaca tgagctgtgt 60

ccactcaggg ttaaatggat taagggcggt gcaagatgtg ctttgttaaa cagatgcttg 120

aaggcagcat gc 132


<210> 93
<211> 275
<212> DNA
<213> HUMAN

<400> 93
gcatagtgcc aggtggtgcc ttgggttcca agctagtcca tggccccgat aaccttctgc 60

ctgtgcacac acctgcccct cactccaccc ccatcctagc tttggtatgg gggagagggc 120

acagggccag acaaacctgt gagactttgg ctccatctct gcaaaagggc gctctgtgag 180

tcagcctgct cccctccagg cttgctcctc ccccacccag ctctcgtttc caatgcacgt 240

acagcccgta cacaccgtgt gctgggacac cccac 275


<210> 94
<211> 89
<212> DNA
<213> HUMAN

<400> 94
ctgctcccct ccaggcttgc tcctccccca cccagctctc gtttccaatg cacgtacagc 60

ccgtacacac cgtgtgctgg gacacccca 89



<210> 95
<211> 61
<212> DNA
<213> HUMAN

<400> 95
cacccagctc tcgtttccaa tgcacgtaca gcccgtacac accgtgtgct gggacacccc 60

a 61



<210> 96
<211> 116
<212> DNA
<213> HUMAN

<400> 96
acctgcccct cactccaccc ccatcctagc tttggtatgg gggagagggc acagggccag 60

acaaacctgt gagactttgg ctccatctct gcaaaagggc gctctgtgag tcagcc 116



<210> 97
<211> 36
<212> PRT
<213> HUMAN

<400> 97
Gly Glu Glu Asp Leu Pro Ser Glu Glu Asp Ser Pro Arg Glu Glu Asp
1               5                   10                  15

Pro Pro Gly Glu Glu Asp Leu Pro Gly Glu Glu Asp Leu Pro Gly Glu
            20                  25                  30

Glu Asp Leu Pro
        35



<210> 98
<211> 6
<212> PRT
<213> HUMAN

<400> 98
Gly Glu Glu Asp Leu Pro
1               5

<210> 99
<211> 4
<212> PRT
<213> HUMAN

<400> 99
Glu Glu Asp Leu
1

<210> 100
<211> 5
<212> PRT
<213> HUMAN

<400> 100
Glu Glu Asp Leu Pro
1               5

<210> 101
<211> 6
<212> PRT
<213> HUMAN

<400> 101
Glu Asp Leu Pro Ser Glu
1               5

<210> 102
<211> 7
<212> PRT
<213> HUMAN

<400> 102
Glu Glu Asp Leu Pro Ser Glu
1               5

<210> 103
<211> 6

<212> PRT
<213> HUMAN

<400> 103
Asp Leu Pro Gly Glu Glu
1               5

<210> 104
<211> 22
<212> PRT
<213> HUMAN

<400> 104
Gly Gly Ser Ser Gly Glu Asp Asp Pro Leu Gly Glu Glu Asp Leu Pro
1               5                   10                  15

Ser Glu Glu Asp Ser Pro
            20

<210> 105
<211> 25
<212> PRT
<213> HUMAN

<400> 105
Gly Glu Glu Asp Leu Pro Ser Glu Glu Asp Ser Pro Arg Glu Glu Asp
1               5                   10                  15

Pro Pro Gly Glu Glu Asp Leu Pro Gly
            20                  25

<210> 106
<211> 24
<212> PRT
<213> HUMAN

<400> 106
Glu Asp Pro Pro Gly Glu Glu Asp Leu Pro Gly Glu Glu Asp Leu Pro
1               5                   10                  15

Gly Glu Glu Asp Leu Pro Glu Val
            20

<210> 107

<211> 7
<212> PRT
<213> HUMAN

<400> 107
Gly Glu Thr Arg Ala Pro Leu
1               5

<210> 108
<211> 7
<212> PRT
<213> HUMAN

<400> 108
Gly Glu Thr Arg Glu Pro Leu
1               5

<210> 109
<211> 7
<212> PRT
<213> HUMAN

<400> 109
Gly Gln Thr Arg Ser Pro Leu
1               5

<210> 110
<211> 1247
<212> DNA
<213> HUMAN

<220>
<221> misc_feature
<222> (1)..(1247)

<400> 110
tatgctactt tttgcagtcc tttcattaca tttttctctc ttcatttgaa gagcatgtta 60

tatcttttag cttcacttgg cttaaaaggt tctctcatta gcctaacaca gtgtcattgt 120

tggtaccact tggatcataa gtggaaaaac agtcaagaaa ttgcacagta atacttgttt 180

gtaagaggga tgattcaggt gaatctgaca ctaagaaact cccctacctg aggtctgaga 240

ttcctctgac attgctgtat ataggctttt cctttgacag cctgtgactg cggactattt 300

ttcttaagca agatatgcta aagttttgtg agcctttttc cagagagagg tctcatatct 360

gcatcaagtg agaacatata atgtctgcat gtttccatat ttcaggaatg tttgcttgtg 420

ttttatgctt ttatatagac agggaaactt gttcctcagt gacccaaaag aggtgggaat 480

tgttattgga tatcatcatt ggcccacgct ttctgacctt ggaaacaatt aagggttcat 540

aatctcaatt ctgtcagaat tggtacaaga aatagctgct atgtttcttg acattccact 600

tggtaggaaa taagaatgtg aaactcttca gttggtgtgt gtccctngtt tttttgcaat 660

ttccttctta ctgtgttaaa aaaaagtatg atcttgctct gagaggtgag gcattcttaa 720

tcatgatctt taaagatcaa taatataatc ctttcaagga ttatgtcttt attataataa 780

agataatttg tctttaacag aatcaataat ataatccctt aaaggattat atctttgctg 840

ggcgcagtgg ctcacacctg taatcccagc actttgggtg gccaaggtgg aaggatcaaa 900

tttgcctact tctatattat cttctaaagc agaattcatc tctcttccct caatatgatg 960

atattgacag ggtttgccct cactcactag attgtgagct cctgctcagg gcaggtagcg 1020

tttttgtttt ttgtttttgt ttttcttttt tgagacaggg tcttgctctg tcacccaggc 1080

cagagtgcaa tggtacagtc tcagctcact gcagcctcaa ccgcctcggc tcaaaccatc 1140

atcccatttc agcctcctga gtagctggga ctacaggcac atgccattac acctggctaa 1200

tttttttgta tttctagtag agacagggtt tggccatgtt gcccggg 1247

<210> 111
<211> 17
<212> DNA
<213> HUMAN

<400> 111
ctctgtgagt cagcctg 17

<210> 112
<211> 23
<212> DNA
<213> HUMAN

<400> 112
aggcttgctc ctcccccacc cag                                              23

<210> 113
<211> 18
<212> DNA
<213> HUMAN

<400> 113
agactttggc tccatctc                                                    18

<210> 114
<211> 20
<212> DNA
<213> HUMAN

<400> 114
cactccaccc ccatcctagc                                                  20

<210> 115
<211> 26
<212> DNA
<213> HUMAN

<400> 115
gggagagggc acagggccag acaaac                                           26

<210> 116
<211> 20
<212> PRT
<213> HUMAN

<400> 116
Gly Gly Gly Gly Ser Gly Gly Gly Gly Ser Gly Gly Gly Gly Ser Gly
1               5                   10                  15

Gly Gly Gly Ser
            20

<210> 117
<211> 34
<212> DNA
<213> HUMAN

<400> 117
cgtctagaag gaattcagct agactggctc agca                                    34

<210> 118
<211> 15
<212> PRT
<213> HUMAN

<400> 118
Glu Val Lys Pro Lys Ser Glu Glu Glu Gly Ser Leu Lys Leu Glu
1               5                   10                  15

<210> 119
<211> 12
<212> PRT
<213> HUMAN

<400> 119
Gly Glu Glu Asp Leu Pro Gly Glu Glu Asp Leu Pro
1               5                   10

<210> 120
<211> 12
<212> PRT
<213> HUMAN

<400> 120
Glu Glu Asp Leu Pro Gly Glu Glu Asp Leu Pro Gly
1               5                   10

<210> 121
<211> 10
<212> PRT
<213> HUMAN

<400> 121
Glu Asp Leu Pro Gly Glu Glu Asp Leu Pro
1               5                   10

<210> 122
<211> 12
<212> PRT

<213> HUMAN

<400> 122
Asp Leu Pro Gly Glu Glu Asp Leu Pro Gly Glu Glu
1               5                   10

<210> 123
<211> 12
<212> PRT
<213> HUMAN

<400> 123
Leu Pro Gly Glu Glu Asp Leu Pro Gly Glu Glu Asp
1               5                   10

<210> 124
<211> 12
<212> PRT
<213> HUMAN

<400> 124
Pro Gly Glu Glu Asp Leu Pro Gly Glu Glu Asp Leu
1               5                   10

<210> 125
<211> 9
<212> PRT
<213> HUMAN

<400> 125
Ala Pro Gly Glu Glu Asp Leu Pro Ala
1               5

<210> 126
<211> 9
<212> PRT
<213> HUMAN

<400> 126
Ala Gly Glu Glu Asp Leu Pro Gly Ala
1               5

<210> 127

<211> 9
<212> PRT
<213> HUMAN

<400> 127
Ala Glu Glu Asp Leu Pro Gly Glu Ala
1               5

<210> 128
<211> 9
<212> PRT
<213> HUMAN

<400> 128
Ala Glu Asp Leu Pro Gly Glu Glu Ala
1               5

<210> 129
<211> 9
<212> PRT
<213> HUMAN

<400> 129
Ala Asp Leu Pro Gly Glu Glu Asp Ala
1               5

<210> 130
<211> 9
<212> PRT
<213> HUMAN

<400> 130
Ala Leu Pro Gly Glu Glu Asp Leu Ala
1               5

<210> 131
<211> 8
<212> PRT
<213> HUMAN

<400> 131
Ala Gly Glu Glu Asp Leu Pro Ala
1               5

<210> 132
<211> 8
<212> PRT
<213> HUMAN

<400> 132
Ala Glu Glu Asp Leu Pro Gly Ala
1               5


<210> 133
<211> 8
<212> PRT
<213> HUMAN

<400> 133
Ala Glu Asp Leu Pro Gly Glu Ala
1               5


<210> 134
<211> 8
<212> PRT
<213> HUMAN

<400> 134
Ala Asp Leu Pro Gly Glu Glu Ala
1               5


<210> 135
<211> 8
<212> PRT
<213> HUMAN

<400> 135
Ala Leu Pro Gly Glu Glu Asp Ala
1               5


<210> 136
<211> 8
<212> PRT
<213> HUMAN

<400> 136
Ala Pro Gly Glu Glu Asp Leu Ala

1                   5


<210> 137
<211> 9
<212> PRT
<213> HUMAN

<400> 137
Ala Lys Lys Met Lys Arg Arg Lys Ala
1                   5


<210> 138
<211> 9
<212> PRT
<213> HUMAN

<400> 138
Ala Ile Thr Phe Asn Ala Gln Tyr Ala
1                   5


<210> 139
<211> 9
<212> PRT
<213> HUMAN

<400> 139
Ala Ser Ala Ser Ala Pro Val Ser Ala
1                   5


<210> 140
<211> 9
<212> PRT
<213> HUMAN

<400> 140
Ala Gly Gln Thr Arg Ser Pro Leu Ala
1                   5


<210> 141
<211> 6
<212> PRT
<213> HUMAN

<400> 141
Ser Glu Glu Asp Ser Pro
1               5

<210> 142
<211> 6
<212> PRT
<213> HUMAN

<400> 142
Arg Glu Glu Asp Pro Pro
1               5

<210> 143
<211> 12
<212> DNA
<213> HUMAN

<400> 143
agggcacagg gc                                                          12